# EUROPÄISCHE PATENTSCHRIFT

(11) **EP 2 260 023 B1**
(45) Veröffentlichungstag und Bekanntmachung des Hinweises auf die Patenterteilung: **25.04.2012**
(21) Anmeldenummer: 09730490.1
(22) Anmeldetag: 11.03.2009
(51) Int. Cl.: C07D 209/86, C07D 235/08, C07D 251/24, C07D 471/14, C07C 13/567, C07C 25/22, C07C 49/786, C07C 211/54, C07F 9/53, C09K 11/06, C07C 217/78, C07C 217/92, C07C 225/22, C07C 255/51, C07C 255/56, C07C 255/58, C07C 317/14, C07C 321/28, C07C 321/30, H01L 51/00

(54) **FLUORENDERIVATE FÜR ORGANISCHE ELEKTROLUMINESZENZVORRICHTUNGEN**
FLUORENE DERIVATIVES FOR ORGANIC ELECTROLUMINESCENCE DEVICES
DÉRIVÉS DU FLUORÈNE POUR DISPOSITIFS ÉLECTROLUMINESCENTS ORGANIQUES

(30) Priorität: 07.04.2008 DE 102008017591
(43) Veröffentlichungstag der Anmeldung: 15.12.2010
(73) Patentinhaber: Merck Patent GmbH, 64293 Darmstadt (DE)
(72) Erfinder: STOESSEL, Philipp, 60487 Frankfurt am Main (DE); HEIL, Holger, 60389 Frankfurt (DE); JOOSTEN, Dominik, 60487 Frankfurt (DE); PFLUMM, Christof, 60316 Frankfurt (DE); GERHARD, Anja, 63329 Egelsbach (DE)
(86) Internationale Anmeldenummer: PCT/EP2009/001736
(87) Internationale Veröffentlichungsnummer: WO 2009/124627

(56) Entgegenhaltungen:
- EP-A- 1 666 970
- EP-A1- 0 539 778
- EP-A1- 1 651 013
- WO-A1-94/04250
- WO-A1-2005/053055
- WO-A1-2006/033554
- WO-A2-98/02379
- JP-A- 9 080 791
- JP-A- 10 095 972
- JP-A- 11 255 859
- JP-A- 2005 085 599
- JP-A- 2005 272 614
- JP-A- 2006 089 541
- US-A- 5 007 945
- US-A- 5 176 977
- US-A- 5 232 471
- US-A- 5 447 960
- US-A- 5 698 740
- US-A1- 2003 162 653
- US-A1- 2004 192 848
- US-A1- 2005 209 404
- DATABASE WPI Week 200735 Thomson Scientific, London, GB; AN 2007-366366 XP002527927 -& JP 2007 049055 A (TOYO INK MANUFACTURING CO., LTD.) 22. Februar 2007 (2007-02-22)
- DATABASE BEILSTEIN [Online] BEILSTEIN INSTITUTE FOR ORGANIC CHEMISTRY, FRANKFURT-MAIN, DE; XP002527926 Database accession no. 8563317 (BRN) & K.-T. LIU ET AL.: JOURNAL OF THE CHINESE CHEMICAL SOCIETY, Bd. 47, Nr. 1, 2000, Seiten 71-76, Taipei, TW
- D. S. TARBELL, Y. SATO: "The Synthesis of 9-Phenanthrylmethyldichlorophenol and Related Compounds" J. AM. CHEM. SOC., Bd. 68, 1946, Seiten 1091-1094, XP002527921
- S. SETAYESH ET AL.: "Polyfluorenes with Polyphenylene Dendron Side Chains: Toward Non-Aggregating, Light-Emitting Polymers" J. AM. CHEM. SOC., Bd. 123, Nr. 5, 16. Januar 2001 (2001-01-16), Seiten 946-953, XP002527922
- K.-T. LIU ET AL.: "Solvolysis of 9-aryl-9-chlorofluorenes revisited. Solvent and substituent effect" ORGANIC REACTIVITY, Bd. 31, Nr. 1, 1997, Seiten 59-66, XP009116780
- K.-T. LIU, Y.-S. LIN: "Solvent and Substituent Effects in the Solvolysis of 9-Aryl-9-bromofluorenes" JOURNAL OF THE CHINESE CHEMICAL SOCIETY, Bd. 47, Nr. 1, 2000, Seiten 71-76, XP009116779
- M. R. PIXTON, D. R. PAUL: "Gas Transport Properties of Polyarylates Part II: Tetrabromination of the Bisphenol" JOURNAL OF POLYMER SCIENCE: PART B: POLYMER PHYSICS, Bd. 33, 1995, Seiten 1353-1364, XP002527923
- M. BALLESTER ET AL.: "Inert Carbon Free Radicals. 5. Perchloro-9-phenylfluorenyl Radical Series" J. ORG. CHEM., Bd. 49, Nr. 5, 1984, Seiten 770-778, XP002527924
- R. BOLTON ET AL.: "Competition in Intramolecular Arylation of Triphenylmethanols" J. CHEM. SOC. PERKIN TRANS. II, 1986, Seiten 405-408, XP009116852
- N. CHEN ET AL.: "New gas separation membrane materials - halogenated dicarbonyl phenylene based polyarylates" POLYMERIC MATERIALS SCIENCE AND ENGINEERING, Bd. 66, 1992, Seiten 412-413, XP009116849
- K. ONO ET AL.: "Synthesis and Properties of 9,9'-Diaryl-4,5-diazafluorenes. A New Type of Electron-Transporting and Hole-Blocking Material in EL Device" CHEMISTRY LETTERS, Bd. 33, Nr. 3, 9. Februar 2004 (2004-02-09), Seiten 276-277, XP009116781

## Beschreibung

Die vorliegende Erfindung betrifft organische Halbleiter und deren Verwendung in organischen elektronischen Vorrichtungen.

Organische Halbleiter werden für eine Reihe verschiedenartiger elektronischer Anwendungen entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings sind noch weitere Verbesserungen für die Verwendung dieser Vorrichtungen für hochwertige und langlebige Displays wünschenswert. So gibt es insbesondere bei der Lebensdauer und der Effizienz blau emittierender organischer Elektrolumineszenzvorrichtungen derzeit noch Verbesserungsbedarf. Weiterhin ist es erforderlich, dass die Verbindungen eine hohe thermische Stabilität und eine hohe Glasübergangstemperatur aufweisen und sich unzersetzt sublimieren lassen. Insbesondere für Anwendung bei erhöhter Temperatur ist eine hohe Glasübergangstemperatur für die Erreichung hohler Lebensdauern essentiell.

Es besteht weiterhin Bedarf an verbesserten Materialien, beispielsweise Hostmaterialien für fluoreszierende und phosphoreszierende Emitter, aber insbesondere auch bei Ladungstransportmaterialien, also Loch- und Elektronentransportmaterialien, und Ladungsblockiermaterialien sind weitere Verbesserungen wünschenswert. Gerade die Eigenschaften dieser Materialien sind häufig limitierende für die Lebensdauer und Effizienz der organischen Elektrolumineszenzvorrichtung.

Überraschend wurde gefunden, dass 9,9-Diphenylfluorenderivate, welche an beiden Phenylgruppen jeweils in 3'- und 5'-Position substituiert sind, sich sehr gut für die Verwendung in organischen Elektrolumineszenzvorrichtungen eignen und dort zu deutlichen Verbesserungen gegenüber dem Stand der Technik führen. Dies gilt ebenso, wenn statt des Fluorens 9,10-Dihydroanthracenderivate oder entsprechende heterocyclische Derivate verwendet werden. Diese Verbindungen und deren Anwendung in organischen elektronischen Vorrichtungen sind daher der Gegenstand der vorliegenden Erfindung. Je nach Substitution an den Phenylgruppen eignen sich die erfindungsgemäßen Verbindungen insbesondere als Lochtransportmaterialien, Elektronen- bzw. Excitonenblockiermaterialien, Matrixmaterialien für fluoreszierende oder phosphoreszierende Verbindungen, Lochblockiermaterialien und Elektronentransportmaterialien. Mit den erfindungsgemäßen Materialien ist eine Steigerung der Effizienz bei gleicher oder verbesserter Lebensdauer der organischen elektronischen Vorrichtung im Vergleich zu Materialien gemäß dem Stand der Technik möglich. Weiterhin weisen diese Verbindungen eine hohe thermische Stabilität auf. Generell sind diese Materialien sehr gut für die Verwendung in organischen elektronischen Vorrichtungen geeignet, da sie eine hohe Glasübergangstemperatur aufweisen. Die entsprechenden erweiterten Strukturen, insbesondere Indenofluorenstrukturen und Indenocarbazolstrukturen, weisen ebenfalls sehr gute Eigenschaften auf.

Als nächstliegender Stand der Technik können die US 5,698,740, JP 2005/085599 und JP 2007/049055 genannt werden. In der US 5,698,740 und JP 2005/085588 werden 9,9-Diphenylfluorenderivate offenbart, welche an jeder der beiden Phenylgruppen mit mindestens einer Aminogruppe bzw. mono- oder disubstituierten Aminogruppe substituiert sind. Es sind nur Strukturen explizit offenbart, welche jeweils in der 4'-Position der Phenylgruppen, also para zur Verknüpfung zum Fluoren, durch Aminogruppen substituiert sind. Strukturen, welche an einer Phenylgruppe mit mehreren Aminogruppen substituiert sind, sind nicht offenbart. In der JP 2007/049055 werden 9,9-Diphenylfluorenderivate offenbart, welche eine mindestens einer der beiden Phenylgruppen mit mindestens einer substituierten oder unsubstituierten Pyrrol- oder Benzimidazolgruppe substituiert sind. Es sind nur Strukturen explizit offenbart, welche jeweils in der 4-Position der Phenylgruppen, also para zur Verknüpfung zum Fluoren, durch Aminogruppen substituiert sind. Strukturen, welche an einer Phenylgruppe mit mehreren Pyrrol- oder Benzimidazolgruppe substituiert sind, sind nicht offenbart. Das in diesen Anmeldungen offenbarte Substitutionsmuster führt jedoch nicht zu Verbindungen, welche ausreichend gute Eigenschaften bei Verwendung in organischen elektronischen Vorrichtungen aufweisen. Es wurde überraschend gefunden, dass gerade die gleichzeitige Substitution beider Phenylgruppen in jeweils der 3'- und der 5'-Position für die guten Eigenschaften der erfindungsgemäßen Verbindungen verantwortlich ist.

Weiterhin wird in der WO 05/053055 ein 9,9-Bis(triazinyl)fluoren, welches an jeder Triazingruppe jeweils in 3,5-Position eine Phenylgruppe trägt, als Lochblockiermaterial in phosphoreszierenden Elektrolumineszenzvorrichtungen offenbart. Dabei wird der Effekt dieser Verbindung jedoch auf die Anwesenheit der Triazingruppen im Molekül zurückgeführt. Der Anwesenheit von Substituenten in 3,5-Position des Triazins wird keine Bedeutung zugemessen.

Der Übersichtlichkeit halber wird im Folgenden die Struktur und die Nummerierung des 9,9-Diphenylfluorens dargestellt:

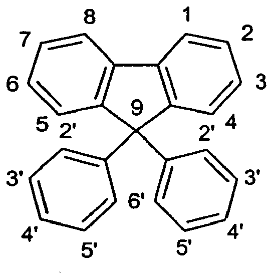

Gegenstand der Erfindung sind somit Verbindungen gemäß Formel (1), 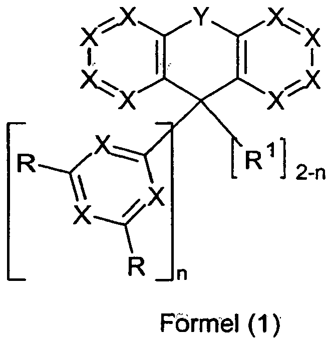 wobei für die verwendeten Symbole gilt:
- X: ist bei jedem Auftreten gleich oder verschieden CR¹ oder N, wobei in jedem Cyclus maximal 3 Gruppen X für N stehen; oder zwei direkt benachbarte Gruppen X stehen für eine Einheit der folgenden Formel (7), 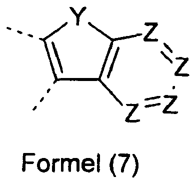 wobei die gestrichelten Bindungen die Verknüpfung der Einheit mit den benachbarten C- bzw. N-Atomen andeutet;
- Y: ist bei jedem Auftreten gleich oder verschieden eine Einfachbindung oder eine Gruppe ausgewählt aus BR¹, C(R¹)₂, C(=O), NR¹, P(=O)R¹ oder O;
- Z: ist bei bei jedem Auftreten gleich oder verschieden CR¹ oder N, wobei in jedem Cyclus maximal zwei Symbole Z für N stehen;
- R: ist bei jedem Auftreten gleich oder verschieden Br, I, Triflat, B(OR²)₂, B(R²)₂, B(N(R²)₂)₂, NAr₂, N(R²)₂, SiAr₃, Si(R²)₃, C(=O)Ar, C(=O)R², OAr, OR², SAr, SR², S(=O)Ar, S(=O)R², S(=O)₂Ar, S(=O)₂R², PAr₂, P(R²)₂, P(=O)Ar₂, P(=O)(R²)₂ oder ein aromatisches oder heteroaromatisches Ringsystem mit 5 bis 60 aromatischen Ringatomen, welches durch einen oder mehrere Reste R¹ substituiert sein kann;
- Ar: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
- R¹: ist bei jedem Auftreten gleich oder verschieden H, D, F, Cl, Br, I, CHO, N(R²)_{2,} N(Ar)₂, C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², eine geradkettige Alkyl-, Alkenyl-, Alkinyl-, Alkoxy- oder Thioalkoxygruppe mit 1 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C , Si(R²)₂, Ge(R²)₂, Sn(R²)₂, C=O, C=S, C=Se, C=NR², P(=O)(R²), SO, SO₂, NR²,O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches oder aromatisches Ringsystem bilden;
- R²: ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches, aliphatisches oder aromatisches Ringsystem bilden;
- n: ist 1 oder 2;
dabei sind die folgenden Verbindungen von der Erfindung ausgenommen:

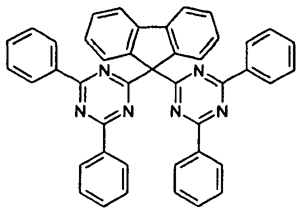

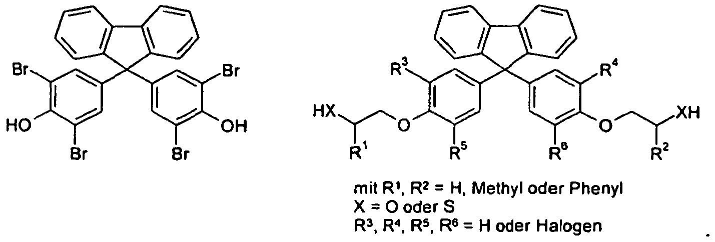

Bevorzugt weisen die Verbindungen gemäß Formel (1) eine Glasübergangstemperatur T_{G} von größer als 70°C auf, besonders bevorzugt größer als 100 °C, ganz besonders bevorzugt größer als 110°C.

Wie aus der Formel (1) hervorgeht, bedeutet n = 2, dass in der Verbindung zwei Arylreste, welche in 3,5-Position substituiert sind, in der 9,9-Position des Fluorens bzw. des entsprechenden Derivats gebunden sind, während n = 1 bedeutet, dass ein solcher Arylrest vorhanden ist und weiterhin eine Gruppe R¹.

Eine Arylgruppe im Sinne dieser Erfindung enthält 6 bis 60 C-Atome; eine Heteroarylgruppe im Sinne dieser Erfindung enthält 2 bis 60 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl und 2,2,2-Trifluorethyl verstanden. Unter einer Alkenyl-gruppe im Sinne dieser Erfindung werden insbesondere Ethenyl, Propenyl, Butenyl, Pentenyl, Cyclopentenyl, Hexenyl, Cyclohexenyl, Heptenyl, Cycloheptenyl, Octenyl und Cyclooctenyl verstanden. Unter einer Alkinylgruppe im Sinne dieser Erfindung werden insbesondere Ethinyl, Propinyl, Butinyl, Pentinyl, Hexinyl, Heptinyl oder Octinyl verstanden. Unter einer C₁-bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Benzanthracen, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der Erfindung steht das Symbol X gleich oder verschieden bei jedem Auftreten für CR¹ oder N, wobei maximal ein Symbol X pro Cyclus in der Fluoreneinheit für N steht und wobei entweder alle Symbole X in den Substituenten in 9-Position der Fluoreneinheit für CR¹ stehen oder alle Symbole X-für N stehen. In einer weiteren bevorzugten Ausführungsform der Erfindung stehen genau zwei benachbarte Gruppen X für eine Einheit der oben genannten Formel (7). Die Substituenten in der 9-Position des Fluorens stehen also bevorzugt für 3,5-substutiertes Phenyl oder Triazin. Entsprechendes gilt, wenn die zentrale Einheit kein Fluoren, sondern eines der anderen von Formel (1) umfassten Derivate darstellt. Besonders bevorzugt steht das Symbol X für CR¹.

Das Symbol Z in der Einheit gemäß Formel (7) steht bevorzugt für CR¹.

Eine bevorzugte Ausführungsform der Verbindungen gemäß Formel (1) sind die Verbindungen gemäß Formel (2), (3), (8), (9), (10) und (11), 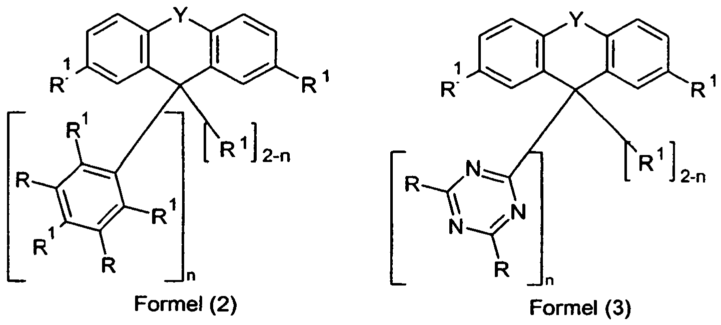 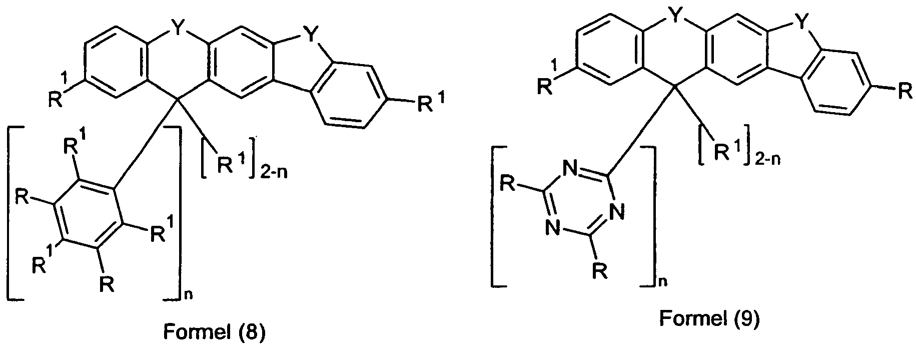 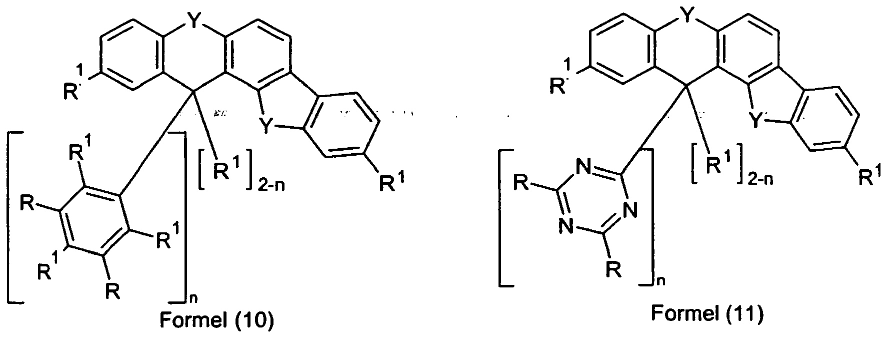 wobei die verwendeten Symbole und Indizes die oben aufgeführten Bedeutungen haben.

In einer bevorzugten Ausführungsform der Erfindung steht das Symbol Y in Verbindungen der Formel (1), (2), (3), (8), (9), (10) oder (11) in dem Sechsring für eine Einfachbindung oder für eine Gruppen ausgewählt aus C(R¹)₂, O oder NR¹, besonders bevorzugt für eine Einfachbindung, und in dem Fünfring bevorzugt für eine Gruppe, ausgewählt aus C(R¹)₂, O oder NR¹, besonders bevorzugt für C(R¹)₂ oder N, ganz besonders bevorzugt C(R¹)₂.

Bevorzugt sind daher die Verbindungen gemäß den Formeln (2a), (3a), (8a), (8b), (9a), (9b), (10a), (10b), (11 a) und (11 b), 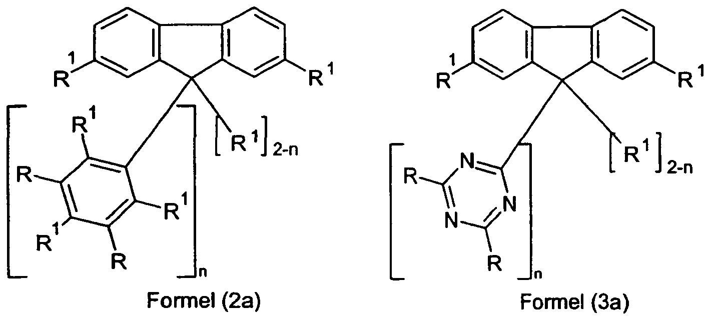 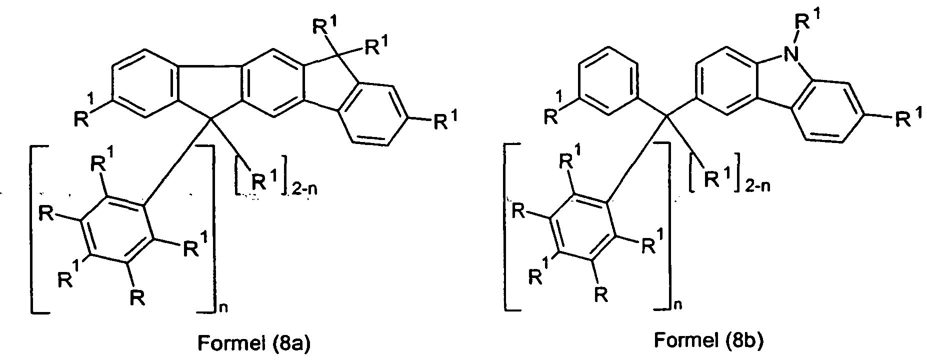 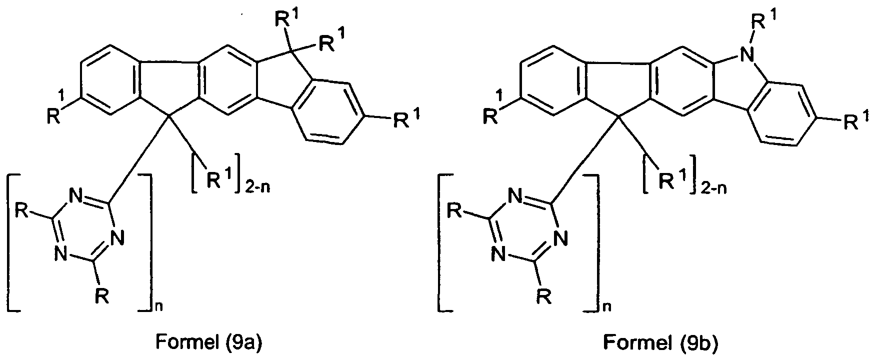 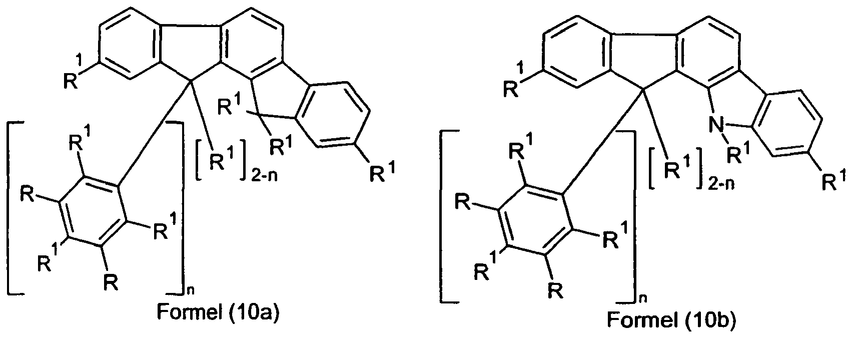 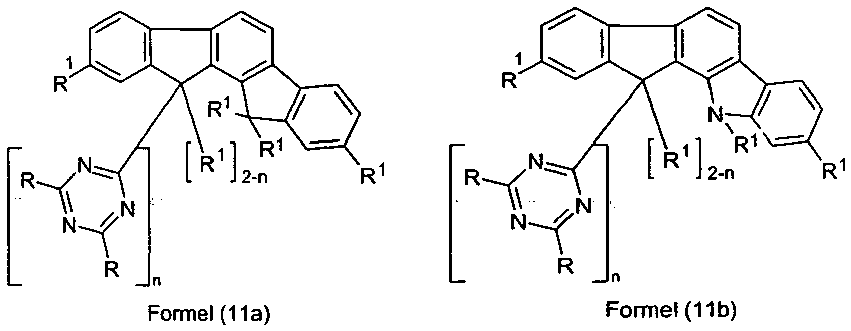 wobei die verwendeten Symbole und Indizes die oben genannten Bedeutungen haben.

In einer bevorzugten Ausführungsform der Erfindung ist n = 2.

Eine weitere bevorzugte Ausführungsform der Verbindungen gemäß Formel (1) sind die Verbindungen gemäß Formel (4a) und (4b), 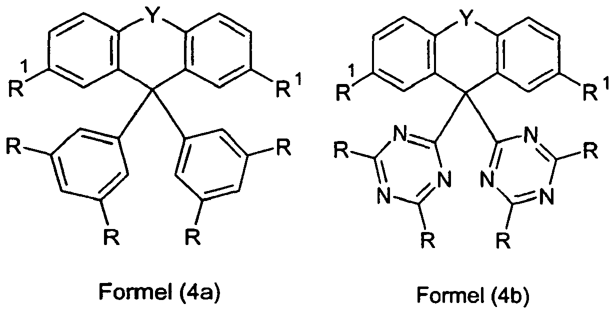 wobei die verwendeten Symbole die oben aufgeführten Bedeutungen haben.

Eine besonders bevorzugte Ausführungsform der Erfindung sind die Verbindungen der folgenden Formeln (4c), (4d), (8c), (8d), (9c), (9d), (10c), (10d), (11c) und (11d) 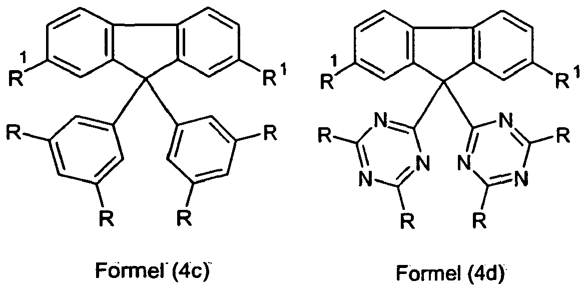 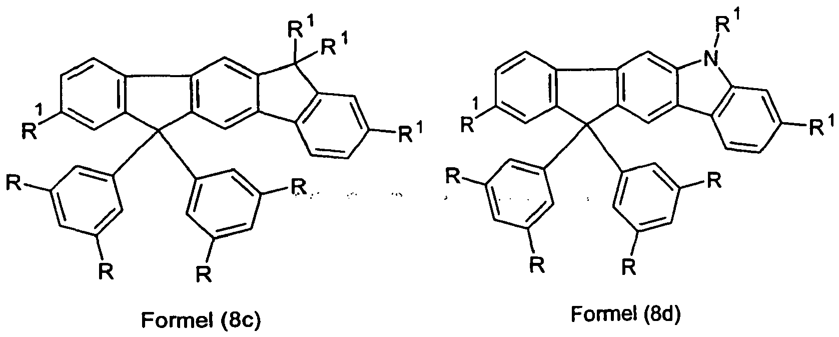 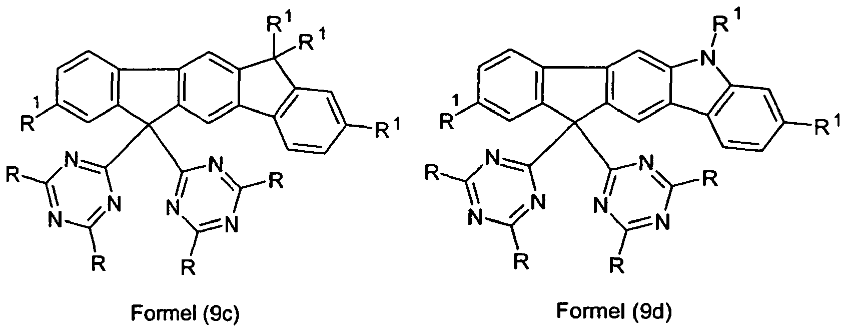 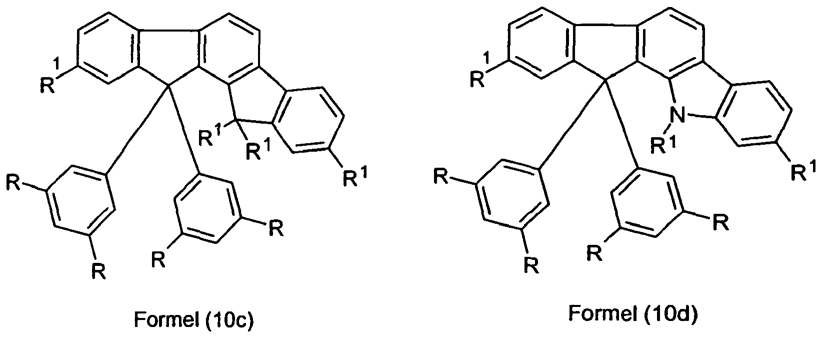 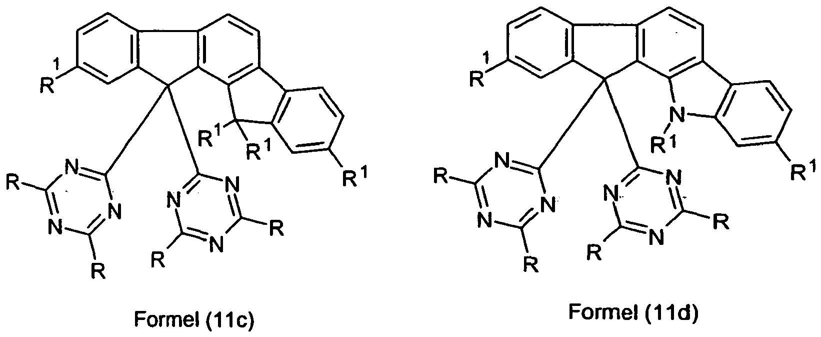 wobei die verwendeten Symbole und Indizes die oben genannten Bedeutungen haben.

In einer weiteren bevorzugten Ausführungsform der Erfindung steht das Symbol R in Verbindungen der oben genannten Formels gleich oder verschieden bei jedem Auftreten für NAr₂, C(=O)Ar, P(=O)Ar₂ oder für ein aromatisches oder heteroaromatisches Ringsystem mit 5 bis 30 aromatischen Ringatomen, welches durch einen oder mehrere nicht-aromatische Reste R¹ substituiert sein kann. Besonders bevorzugt steht R, wenn es an eine Phenylgruppe gebunden ist, gleich oder verschieden, bevorzugt gleich, bei jedem Auftreten für NAr₂ oder C(=O)Ar, ganz besonders bevorzugt für NAr₂. Besonders bevorzugt steht R, wenn es an eine Triazingruppe gebunden ist, für eine Aryl- oder Heteroarylgruppe mit 5 bis 10 aromatischen Ringatomen. Weiterhin bevorzugte Substituenten R sind Br, I und Triflat, insbesondere Br, da dies wertvolle Intermediate bei der Synthese weiterer erfindungsgemäßer Verbindungen sind.

In einer weiteren bevorzugten Ausführungsform der Erfindung sind alle Symbole R in Verbindungen der oben genannten Formeln gleich gewählt. Diese Bevorzugung lässt sich durch die leichtere synthetische Zugänglichkeit der Verbindungen erklären.

Wenn der Rest R bzw. R¹ für eine Gruppe N(Ar)₂ steht, so ist diese Gruppe bevorzugt ausgewählt aus den Gruppen der Formel (5) oder der Formel (6), 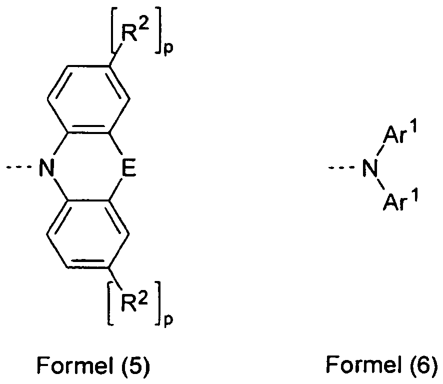 wobei R² die oben aufgeführte Bedeutung hat und weiterhin gilt:
- E: steht für eine Einfachbindung, O, S, N(R²) oder C(R²)₂;
- Ar¹: ist gleich oder verschieden bei jedem Auftreten ein aromatisches oder heteroaromatisches Ringsystem mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, bevorzugt mit 18 bis 22 aromatischen Ringatomen, welche jeweils mit einem oder mehreren Resten R² substituiert sein kann;
- p: ist bei jedem Auftreten gleich oder verschieden 0 oder 1.

Besonders bevorzugt steht Ar¹ gleich oder verschieden bei jedem Auftreten für Phenyl, Biphenyl, 1-Naphthyl, 2-Naphthyl, 2-, 3- oder 4-Triphenylamin, 1- oder 2-Naphthyldiphenylamin, welches jeweils über die Naphthyl- oder die Phenylgruppe gebunden sein kann, oder 1- oder 2-Dinaphthylphenylamin, welches jeweils über die Naphthyl- oder die Phenylgruppe gebunden sein kann, N-Carbazolyl oder N-Phenyl-2-carbazolyl oder N-Phenyl-3-carbazolyl. Diese Gruppen können jeweils durch eine oder mehrere Alkylgruppen mit 1 bis 4 C-Atomen oder durch Fluor substituiert sein.

Wenn der Rest R bzw. R¹ ein aromatisches bzw. hetoroaromatisches Ringsystem darstellt, so ist dieses bevorzugt ausgewählt aus aromatischen oder heteroaromatischen Ringsystemen mit 5 bis 30 aromatischen Ringatomen, insbesondere mit 6 bis 20 aromatischen Ringatomen, ganz besonders bevorzugt aus Phenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl.

In einer weiteren bevorzugten Ausführungsform der Erfindung steht das Symbol R¹ in Verbindungen der oben genannten Formeln gleich oder verschieden bei jedem Auftreten für H, F, N(Ar)₂, C(=O)Ar, P(=O)(Ar)₂, S(=O)Ar, S(=O)₂Ar, CR²=CR²Ar, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, wobei eine oder mehrere nicht benachbarte CH₂-Gruppen durch R²C=CR² oder O ersetzt sein können und wobei ein oder mehrere H-Atome durch F ersetzt sein können, oder ein aromatisches oder heteroaromatisches Ringsystem mit 5 bis 20 aromatischen Ringatomen, das jeweils durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches oder aromatisches Ringsystem bilden. In einer besonders bevorzugten Ausführungsform der Erfindung steht das Symbol R¹ in Verbindungen der oben genannten Formeln gleich oder verschieden bei jedem Auftreten für H, F, N(Ar)₂, eine geradkettige Alkylgruppe mit 1 bis 6 C-Atomen oder eine verzweigte oder cyclische Alkylgruppe mit 3 bis 6 C-Atomen, die jeweils mit einem oder mehreren Resten R² substituiert sein kann, wobei ein oder mehrere H-Atome durch F ersetzt sein können, oder ein aromatisches oder heteroaromatisches Ringsystem mit 5 bis 14 aromatischen Ringatomen, das jeweils durch einen oder mehrere Reste R² substituiert sein kann; dabei können zwei oder mehrere benachbarte Substituenten R¹ auch miteinander ein mono- oder polycyclisches, aliphatisches oder aromatisches Ringsystem bilden. Insbesondere steht R¹ gleich oder verschieden bei jedem Auftreten für H, F, Methyl, Ethyl, iso-Propyl oder tert-Butyl, insbesondere für H. Bei Verbindungen, die aus Lösung verarbeitet werden, sind auch lineare oder verzweigte Alkylketten mit bis zu 10 C-Atomen bevorzugt.

Bevorzugte Reste R¹, die bei entsprechender Wahl von Y in der Brücke Y enthalten sind, sind gleich oder verschieden bei jedem Auftreten und sind bevorzugt ausgewählt aus H, geradkettigen Alkylgruppen mit 1 bis 6 C-Atomen oder verzweigten Alkylgruppen mit 3 bis 6 C-Atomen, wobei eine oder mehrere nicht benachbarte CH₂-Gruppen durch -R²C=CR²-, -C=C- oder -O- ersetzt sein können und wobei ein oder mehrere H-Atome durch F ersetzt sein können, oder Arylgruppen mit 6 bis 20 C-Atomen oder Heteroarylgruppen mit 2 bis 20 C-Atomen, die jeweils mit einem oder mehreren Resten R² substituiert sein können, oder eine Kombination aus zwei oder drei dieser Systeme; dabei können zwei der Reste R¹, die beide an Y gebunden sind, auch miteinander ein Ringsystem bilden und so ein Spirosystem bilden. Besonders bevorzugte Reste R¹, die an die Brücken Y gebunden sind, sind gleich oder verschieden bei jedem Auftreten und sind ausgewählt aus Methyl, Ethyl, iso-Propyl, tert-Butyl, wobei jeweils ein oder mehrere H-Atome durch F ersetzt sein können, oder Arylgruppen mit 6 bis 14 C-Atomen, die mit einem oder mehreren Resten R² substituiert sein können; dabei können zwei Reste R¹ auch miteinander ein Ringsystem bilden. Bei Verbindungen, die aus Lösung verarbeitet werden, sind auch lineare oder verzweigte Alkylketten mit bis zu 10 C-Atomen bevorzugt. Wenn die Brücke Y eine Gruppe NR¹ ist, ist die Gruppe R¹ auch besonders bevorzugt gewählt aus einem aromatischen oder heteroaromatischen Ringsystem mit 5 bis 20 aromatischen Ringatomen.

Beispiele für bevorzugte Verbindungen gemäß den Formeln (1) bis (4) sind die im Folgenden abgebildeten Strukturen (1) bis (276).

| | |
|---|---|
| 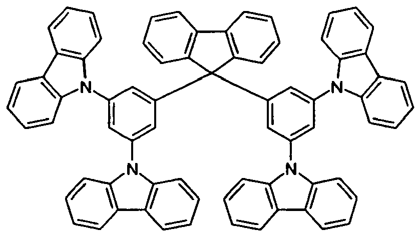 | 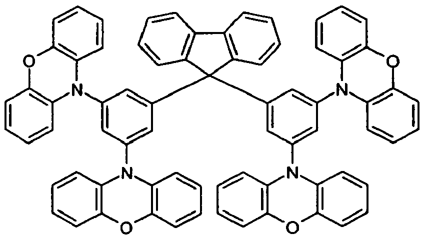 |
| (1) | (2) |
| 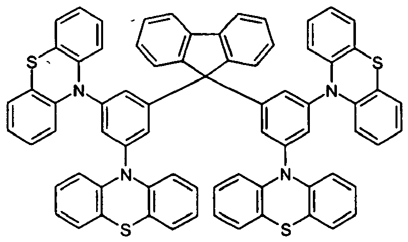 | 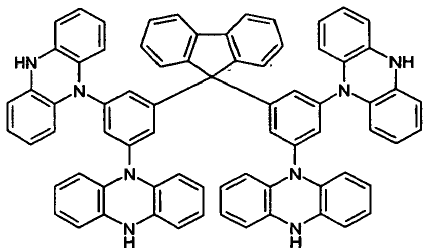 |
| (3) | (4) |
| 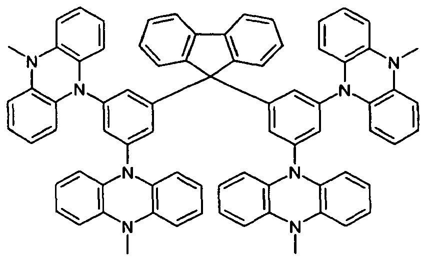 | 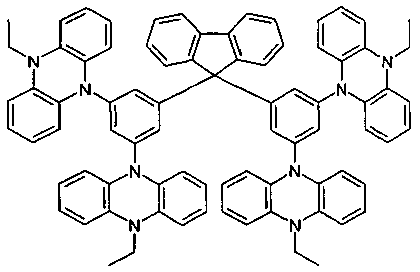 |
| (5) | (6) |
| 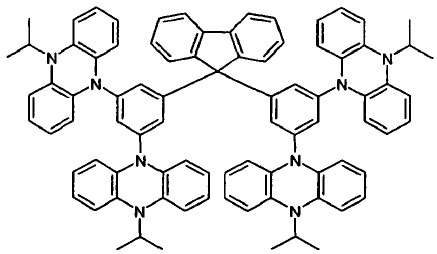 | 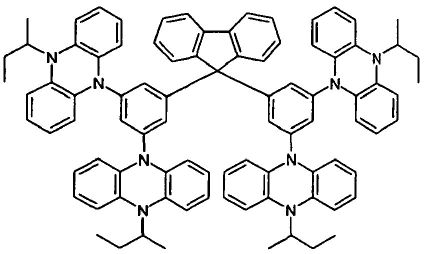 |
| (7) | (8) |
| 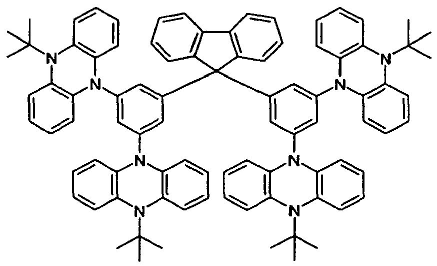 | 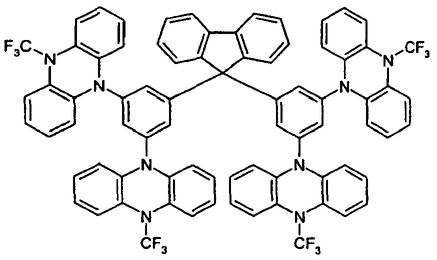 |
| (9) | (10) |
| 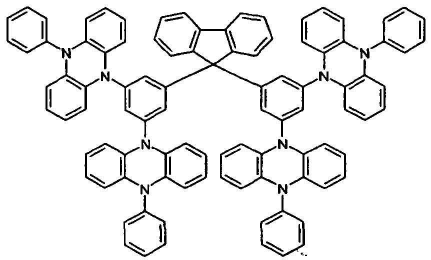 | 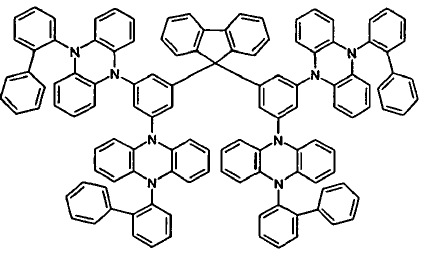 |
| (11) | (12) |
| 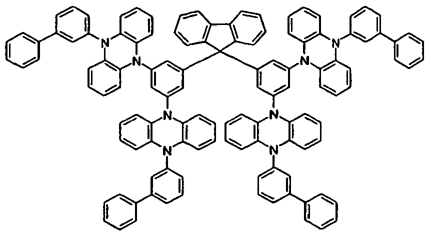 | 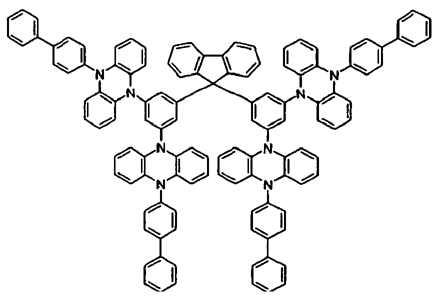 |
| (13) | (14) |
| 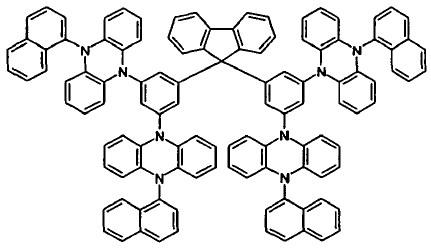 | 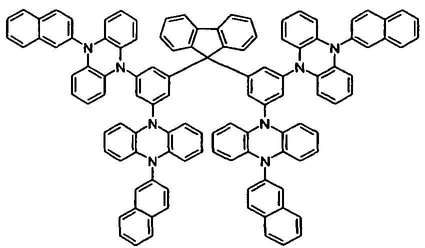 |
| (15) | (16) |
| 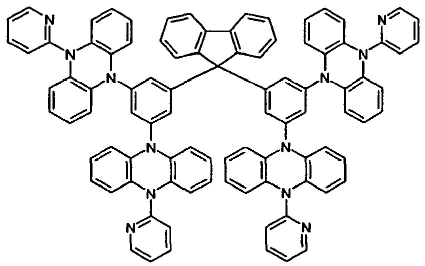 | 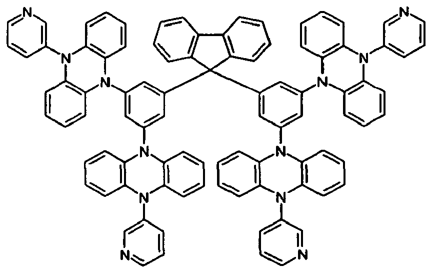 |
| (17) | (18) |
| 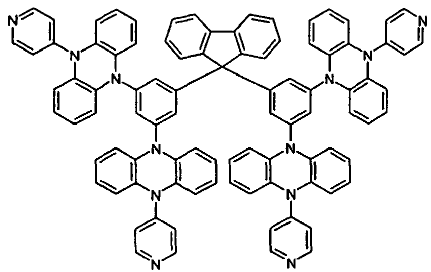 | 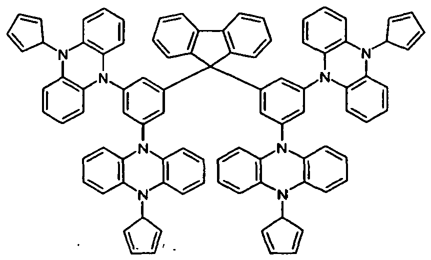 |
| (19) | (20) |
| 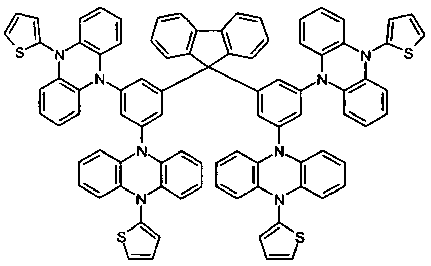 | 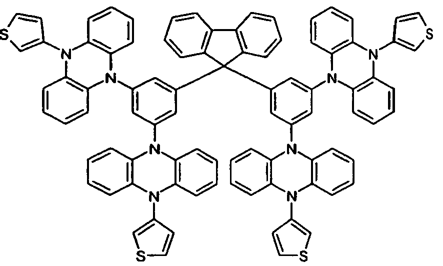 |
| (21) | (22) |
| 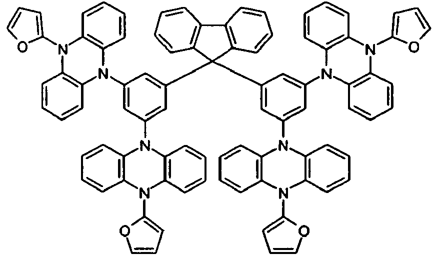 | 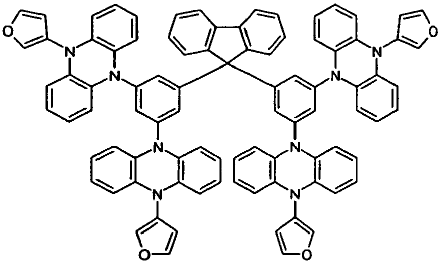 |
| (23) | (24) |
| 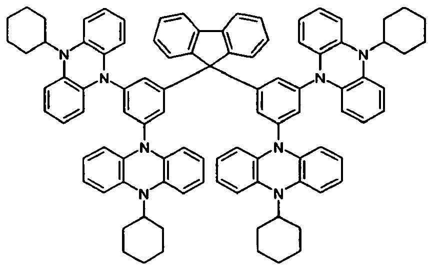 | 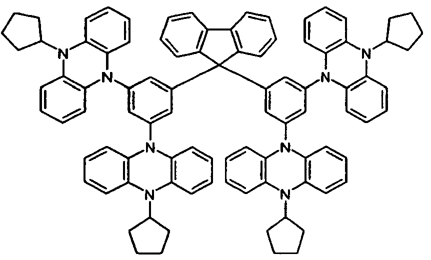 |
| (25) | (26) |
| 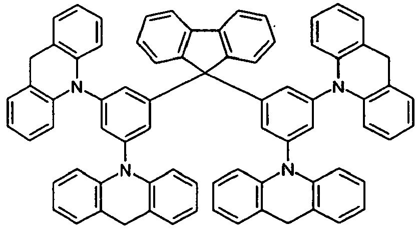 | 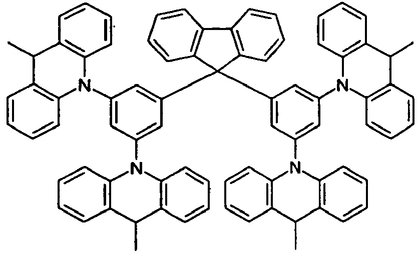 |
| (27) | (28) |
| 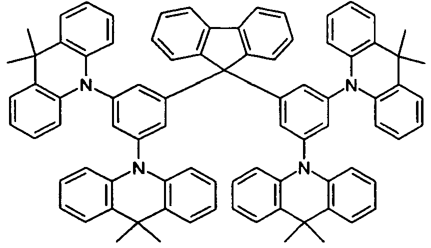 | 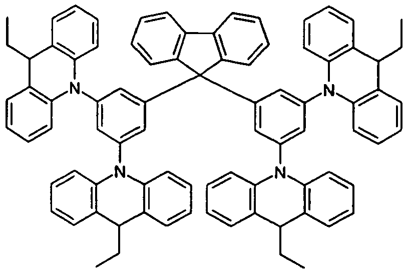 |
| (29) | (30) |
| 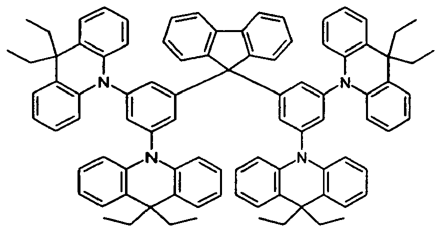 | 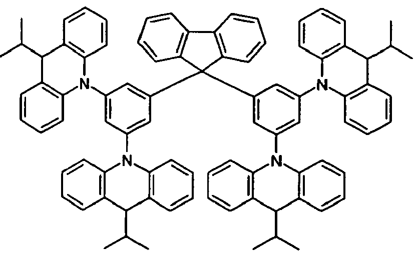 |
| (31) | (32) |
| 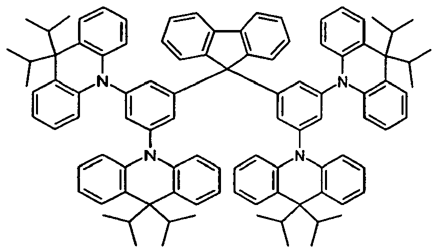 | 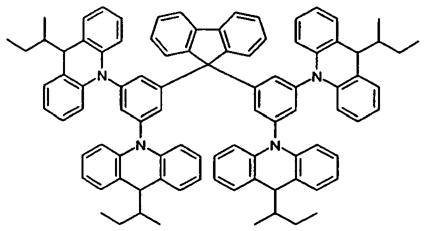 |
| (33) | (34) |
| 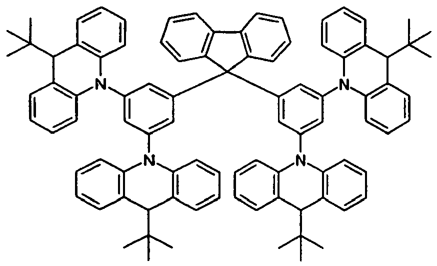 | 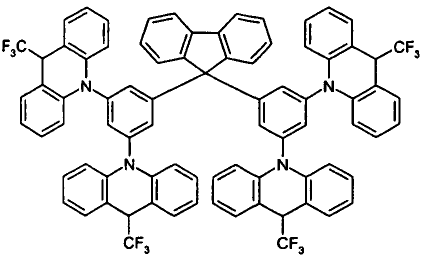 |
| (35) | (36) |
| 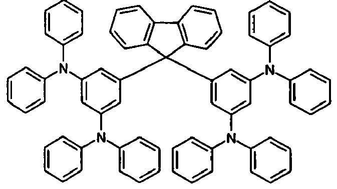 | 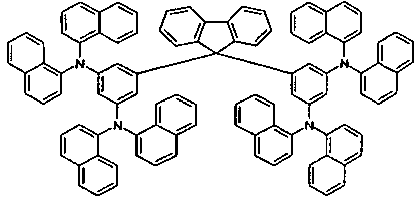 |
| (37) | (38) |
| 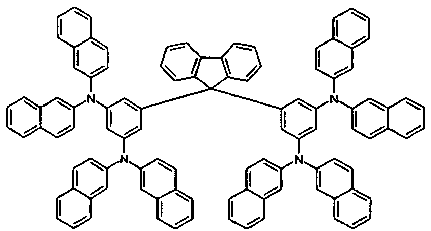 | 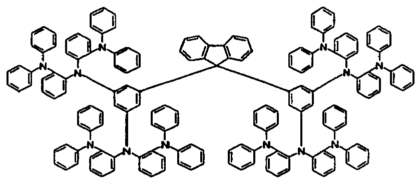 |
| (39) | (40) |
| 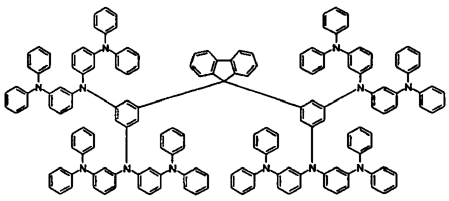 | 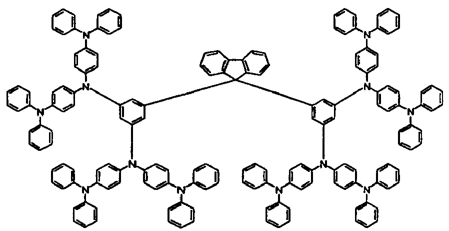 |
| (41) | (42) |
| 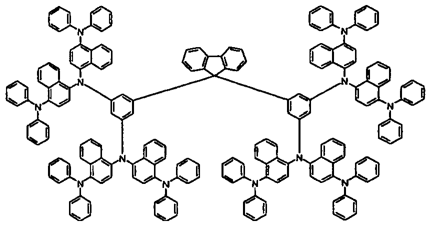 | 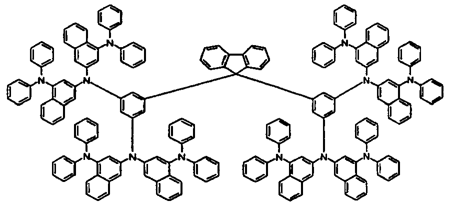 |
| (43) | (44) |
| 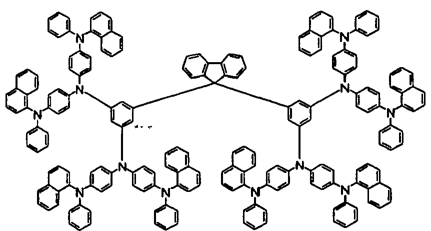 | 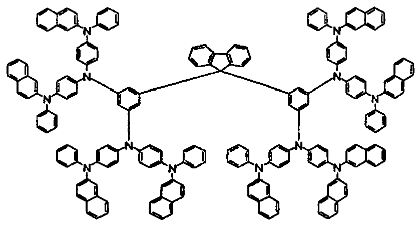 |
| (45) | (46) |
| 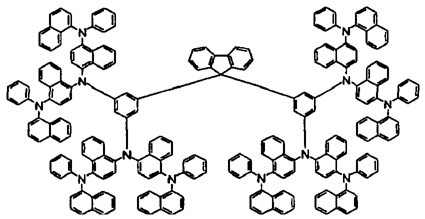 | 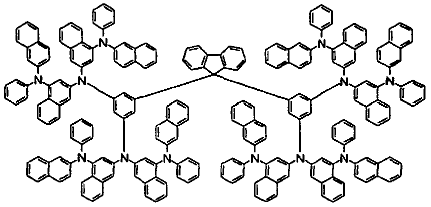 |
| (47) | (48) |
| 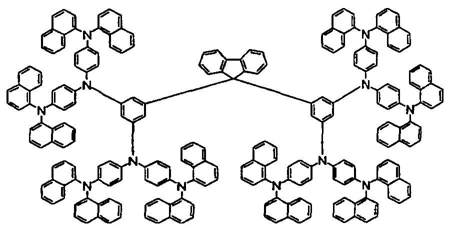 | 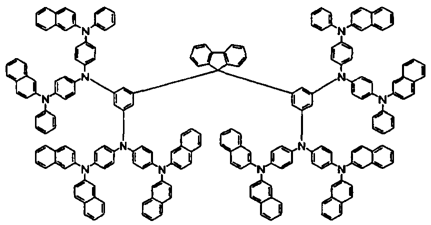 |
| (49) | (50) |
| 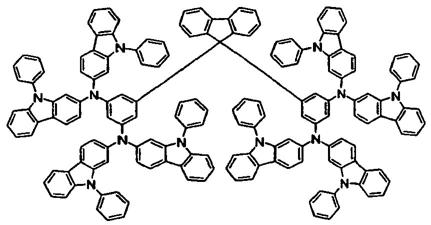 | 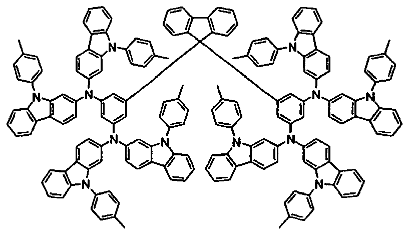 |
| (51) | (52) |
| 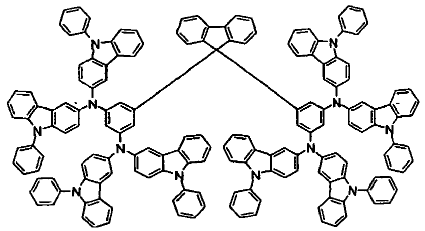 | 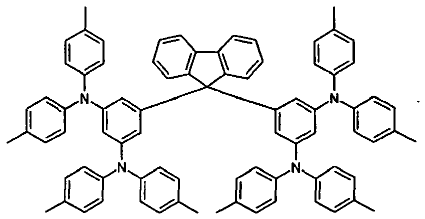 |
| (53) | (54) |
| 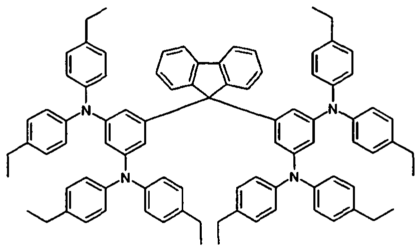 | 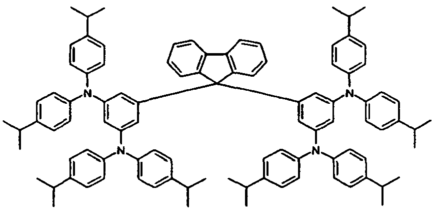 |
| (55) | (56) |
| 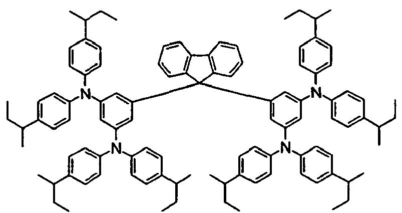 | 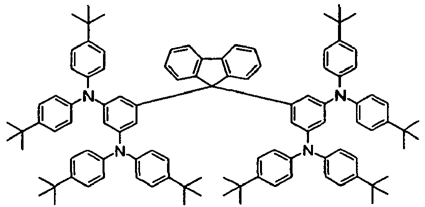 |
| (57) | (58) |
| 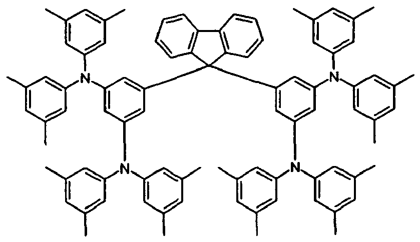 | 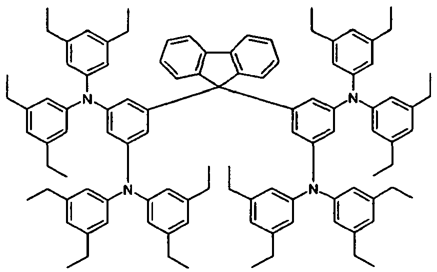 |
| (59) | (60) |
| 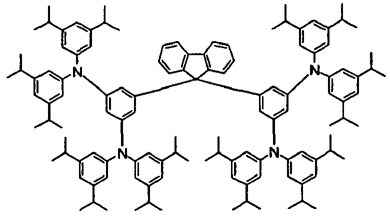 | 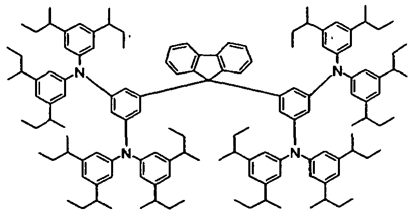 |
| (61) | (62) |
| 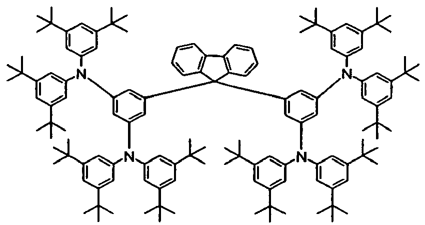 | 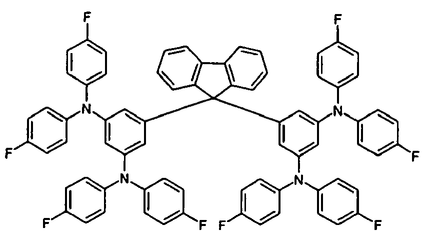 |
| (63) | (64) |
| 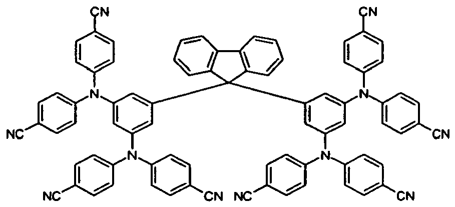 | 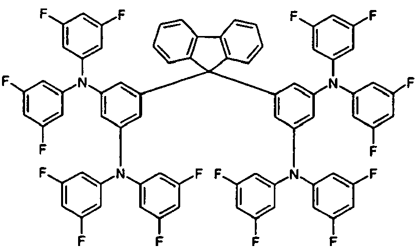 |
| (65) | (66) |
| 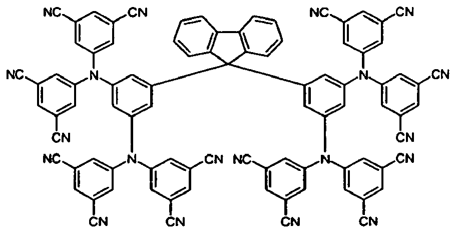 | 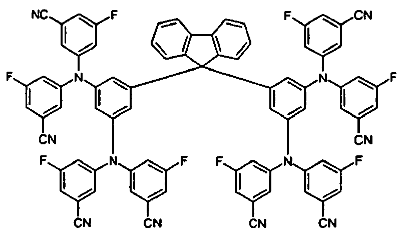 |
| (67) | (68) |
| 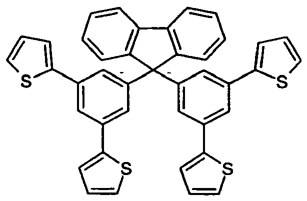 | 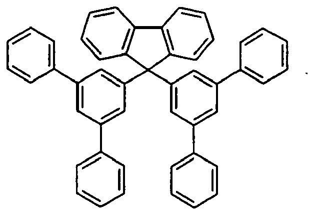 |
| (69) | (70) |
| 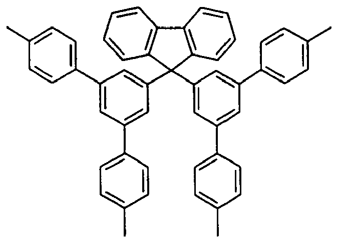 | 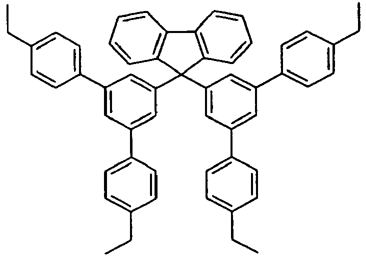 |
| (71) | (72) |
| 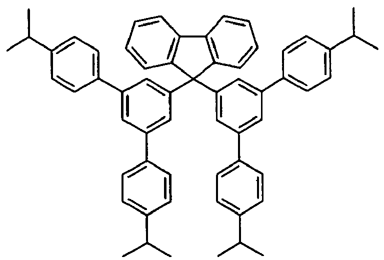 | 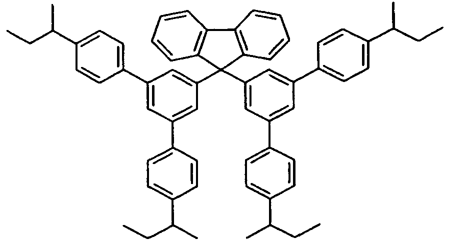 |
| (73) | (74) |
| 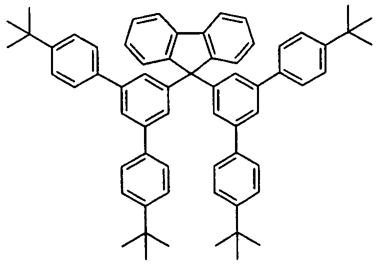 | 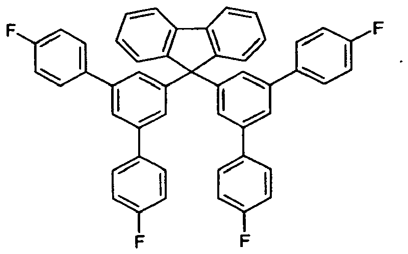 |
| (75) | (76) |
| 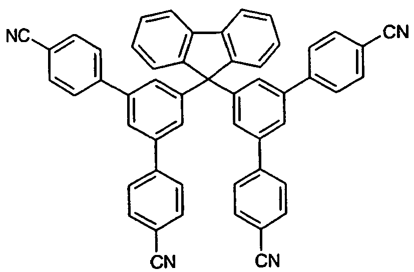 | 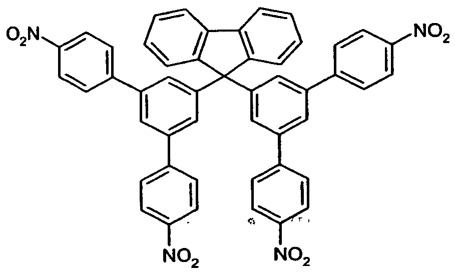 |
| (77) | (78) |
| 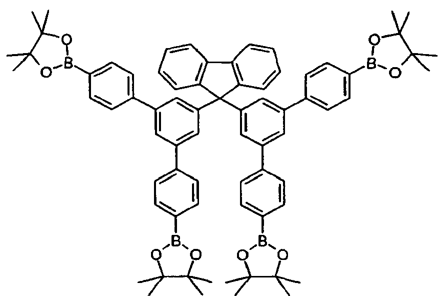 | 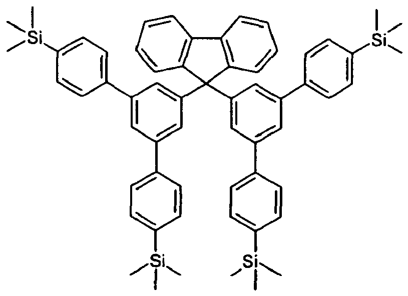 |
| (79) | (80) |
| 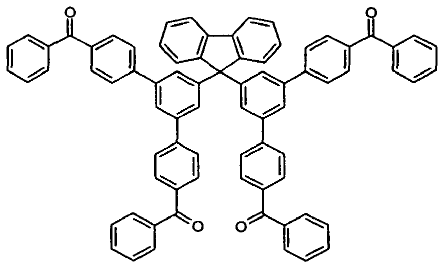 | 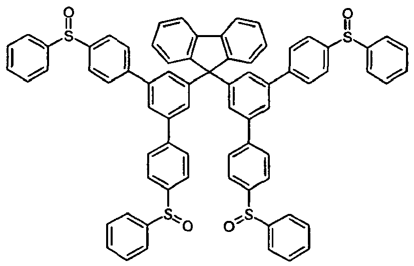 |
| (81) | (82) |
| 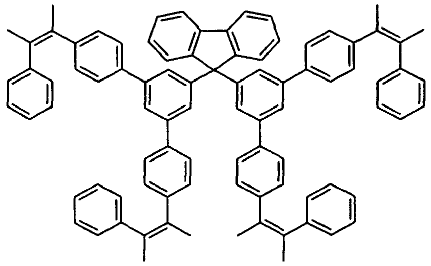 | 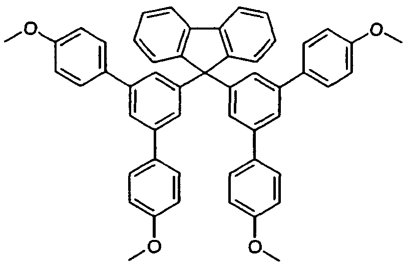 |
| (83) | (84) |
| 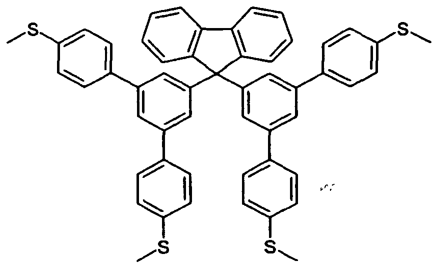 | 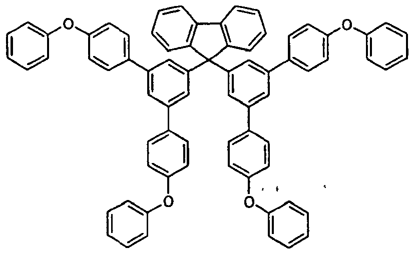 |
| (85) | (86) |
| 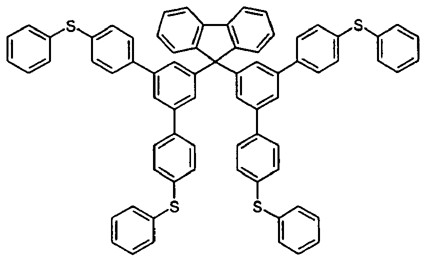 | 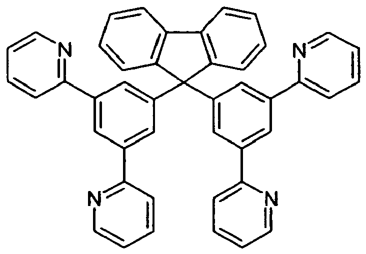 |
| (87) | (88) |
| 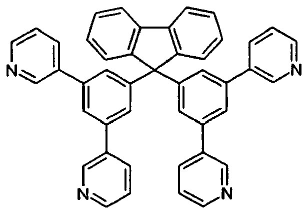 | 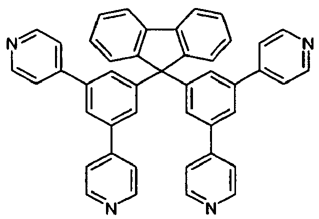 |
| (89) | (90) |
| 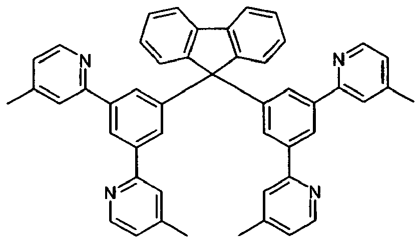 | 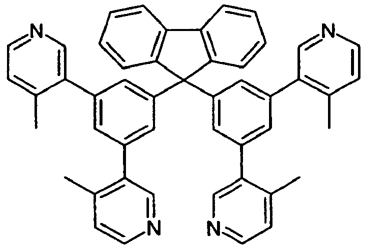 |
| (91) | (92) |
| 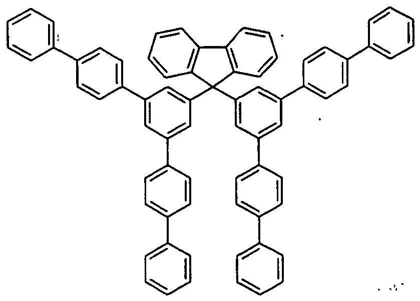 | 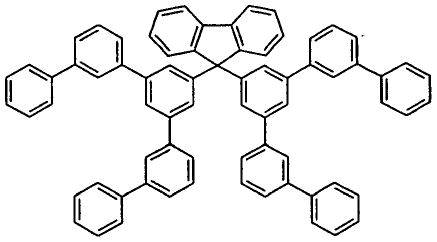 |
| (93) | (94) |
| 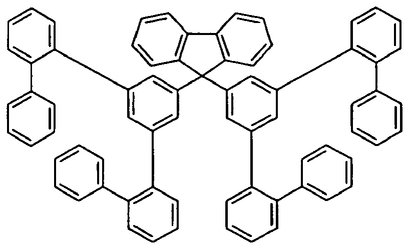 | 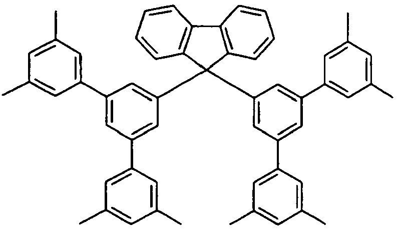 |
| (95) | (96) |
| 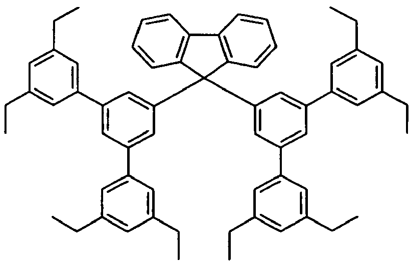 | 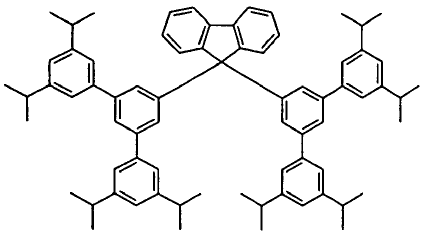 |
| (97) | (98) |
| 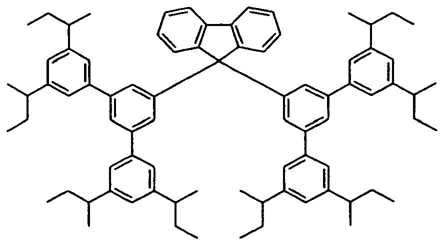 | 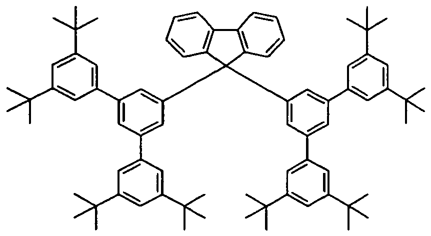 |
| (99) | (100) |
| 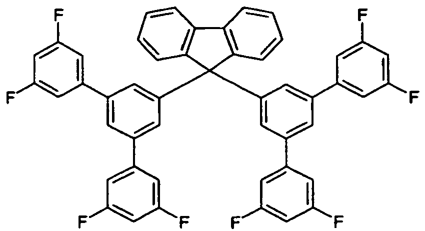 | 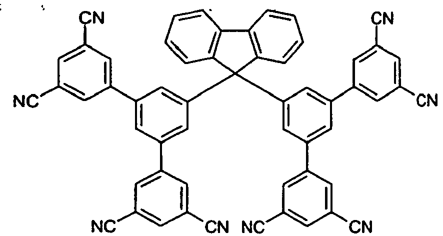 |
| (101) | (102) |
| 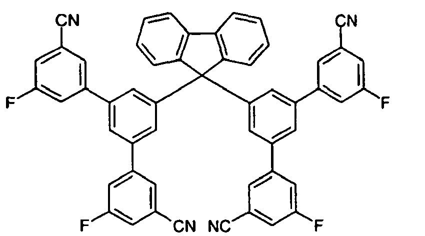 | 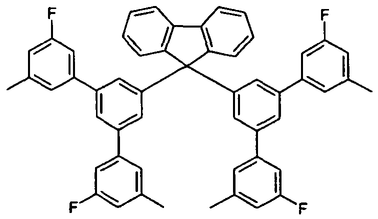 |
| (103) | (104) |
| 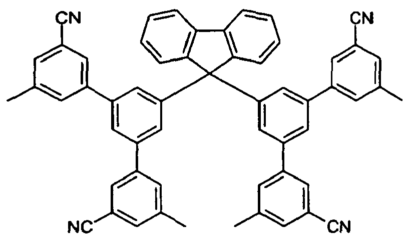 | 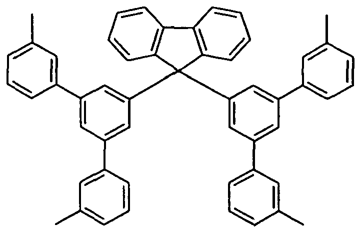 |
| (105) | (106) |
| 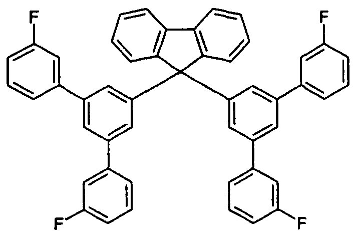 | 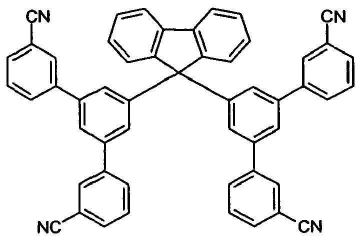 |
| (107) | (108) |
| 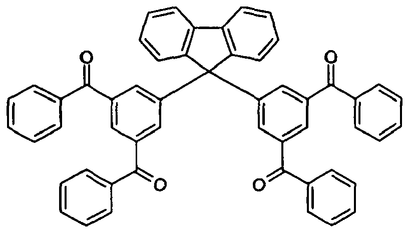 | 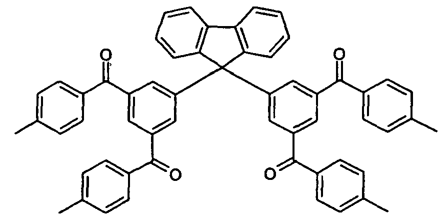 |
| (109) | (110) |
| 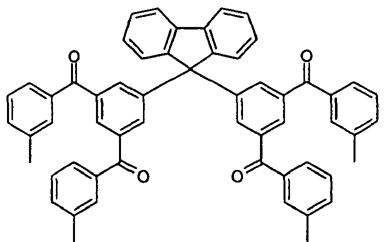 | 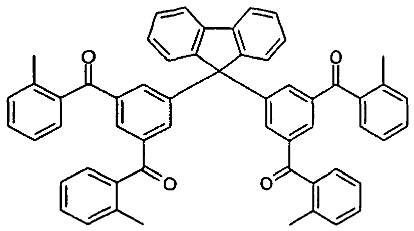 |
| (111) | (112) |
| 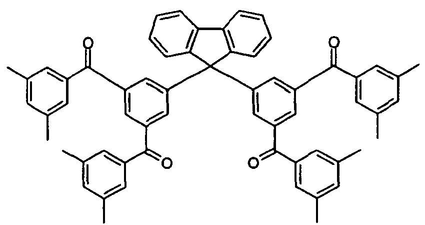 | 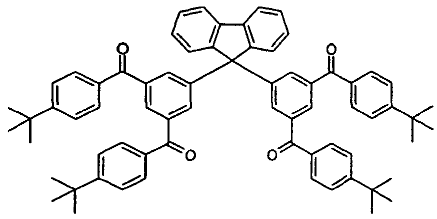 |
| (113) | (114) |
| 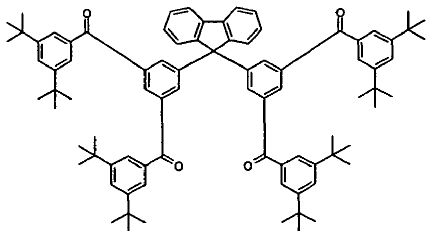 | 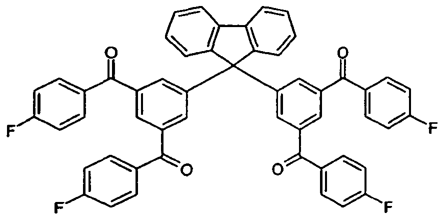 |
| (115) | (116) |
| 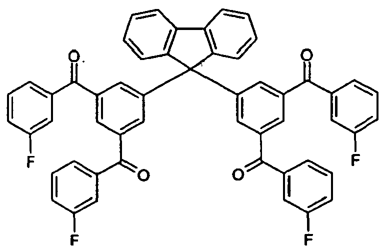 | 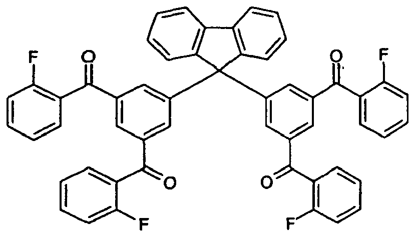 |
| (117) | (118) |
| 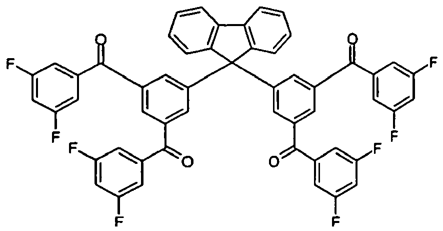 | 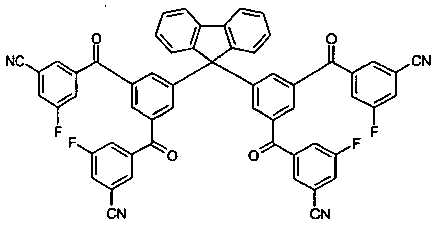 |
| (119) | (120) |
| 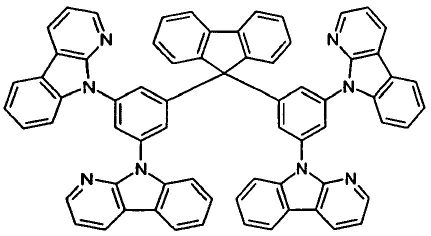 | 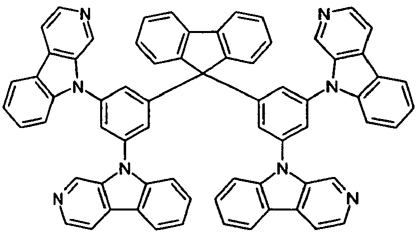 |
| (121) | (122) |
| 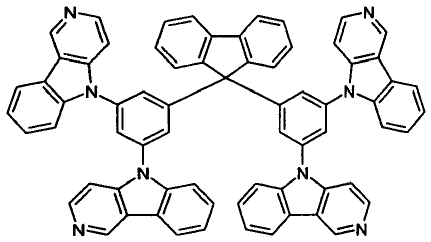 | 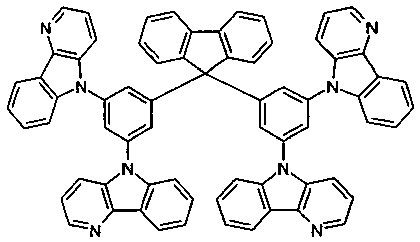 |
| (123) | (124) |
| 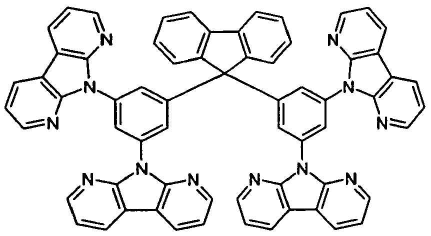 | 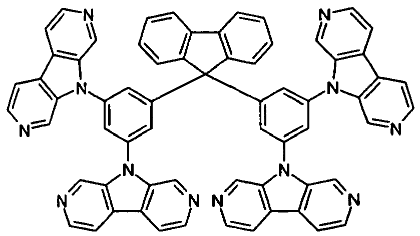 |
| (125) | (126) |
| 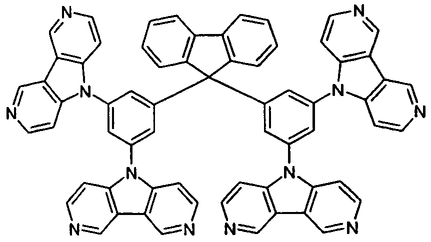 | 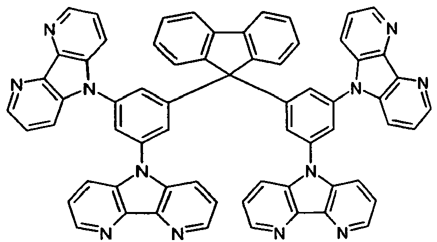 |
| (127) | (128) |
| 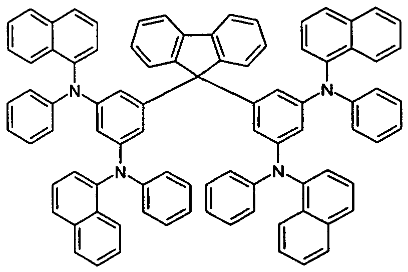 | 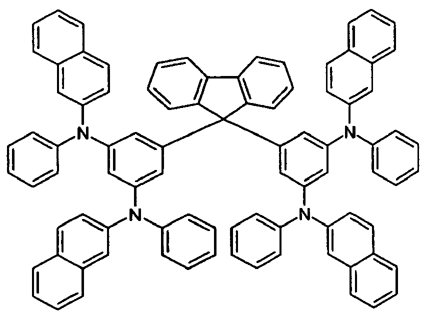 |
| (129) | (130) |
| 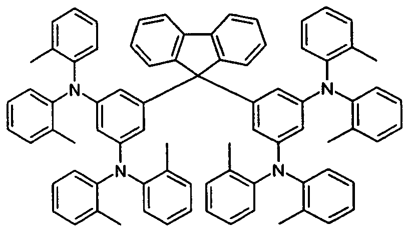 | 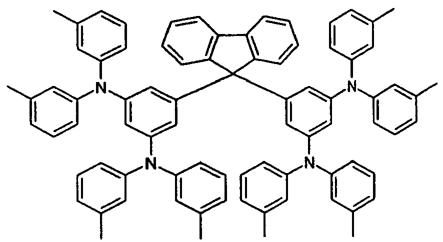 |
| (131) | (132) |
| 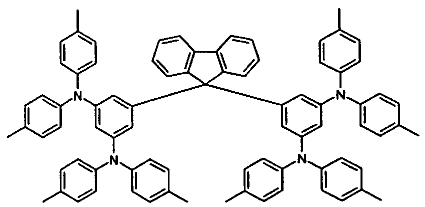 | 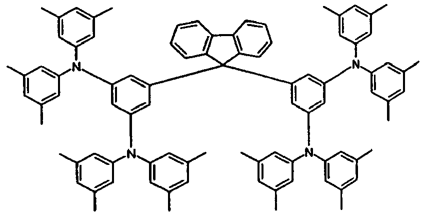 |
| (133) | (134) |
| 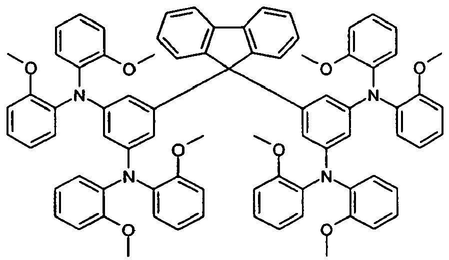 | 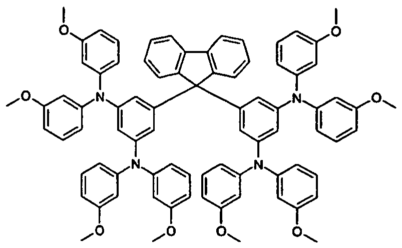 |
| (135) | (136) |
| 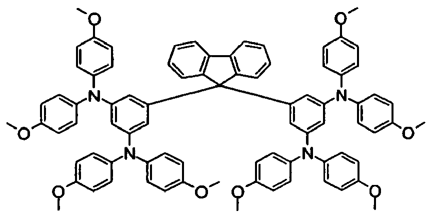 | 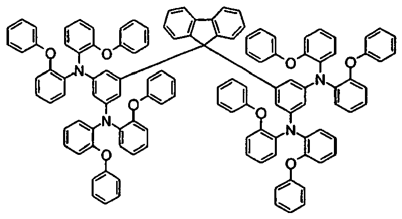 |
| (137) | (138) |
| 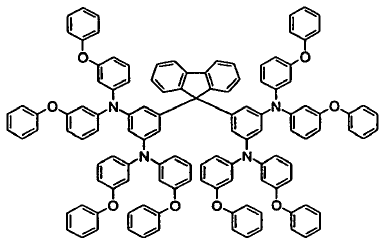 | 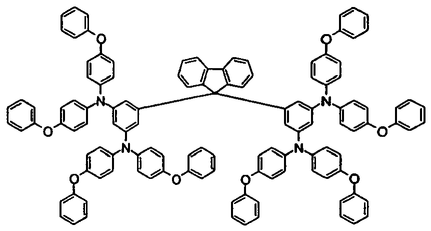 |
| (139) | (140) |
| 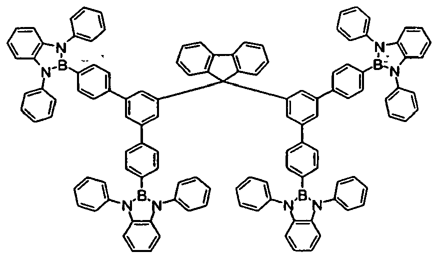 | 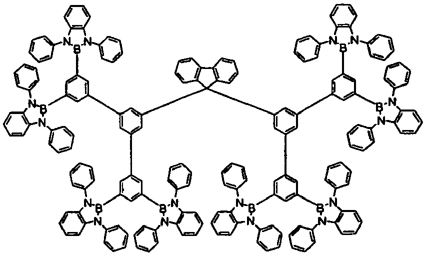 |
| (141) | (142) |
| 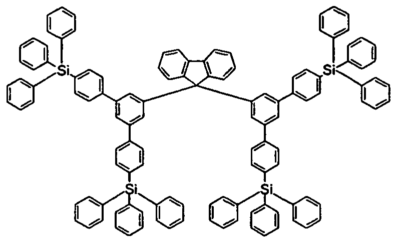 | 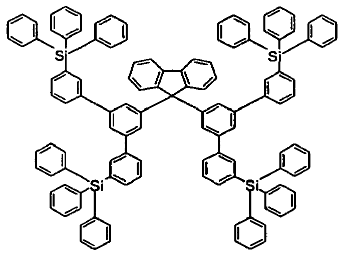 |
| (143) | (144) |
| 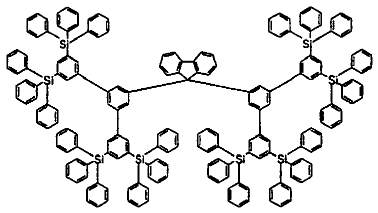 | 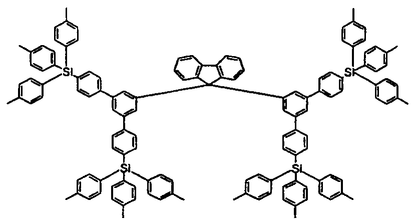 |
| (145) | (146) |
| 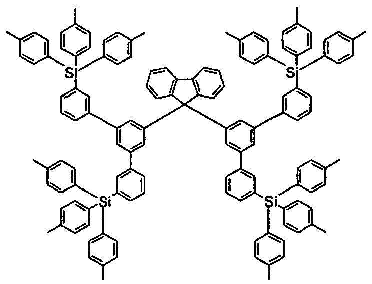 | 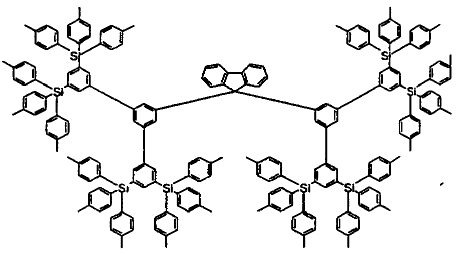 |
| (147) | (148) |
| 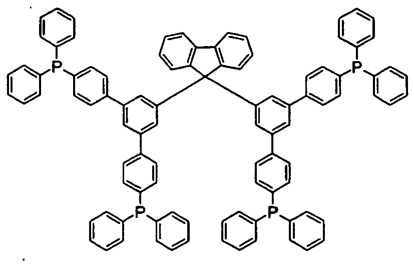 | 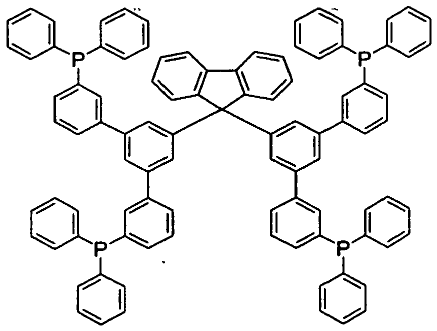 |
| (149) | (150) |
| 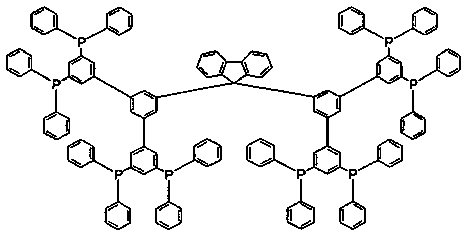 | 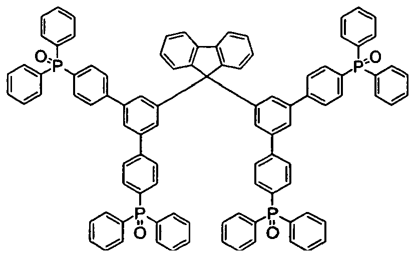 |
| (151) | (152) |
| 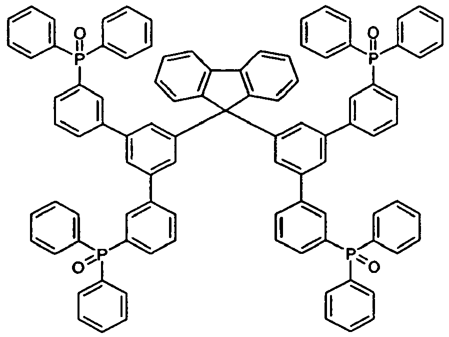 | 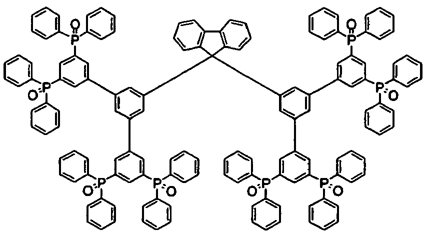 |
| (153) | (154) |
| 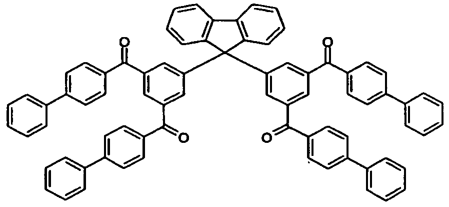 | 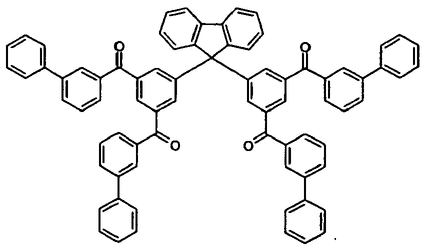 |
| (155) | (156) |
| 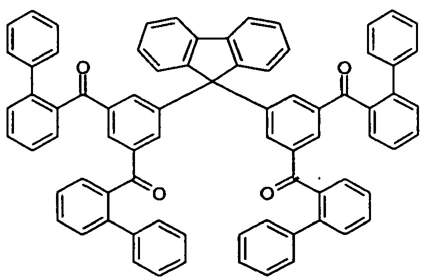 | 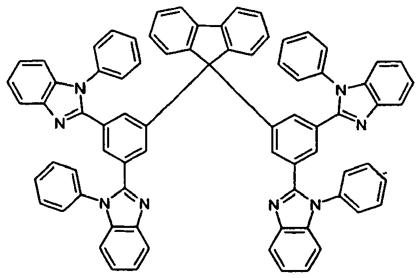 |
| (157) | (158) |
| 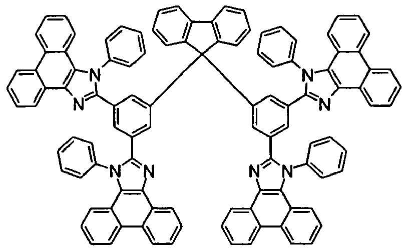 | 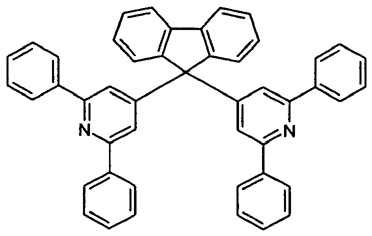 |
| (159) | (160) |
| 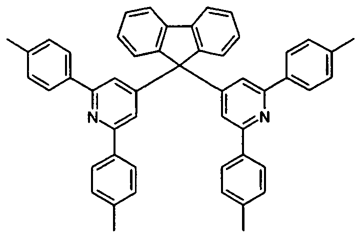 | 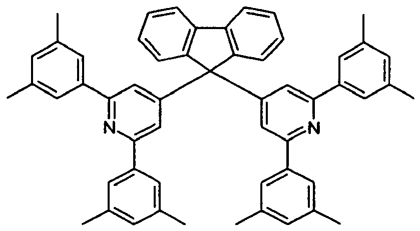 |
| (161) | (162) |
| 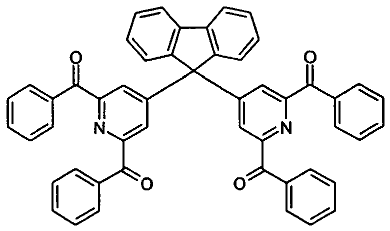 | 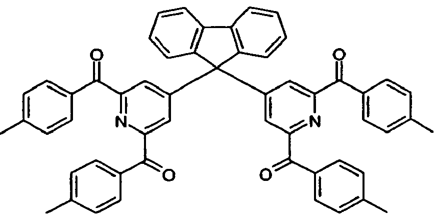 |
| (163) | (164) |
| 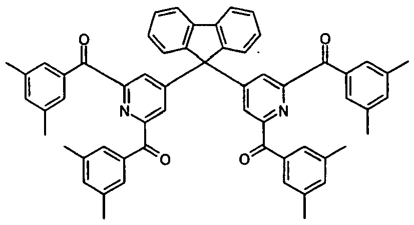 | 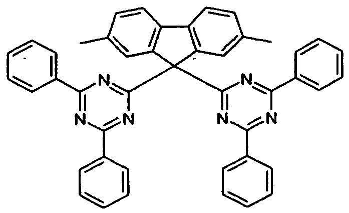 |
| (165) | (166) |
| 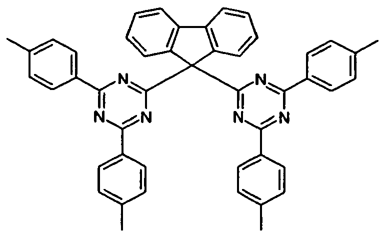 | 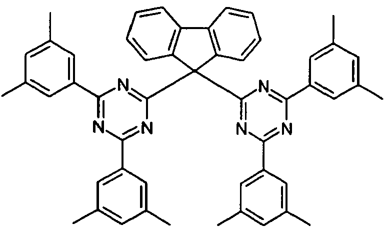 |
| (167) | (168) |
| 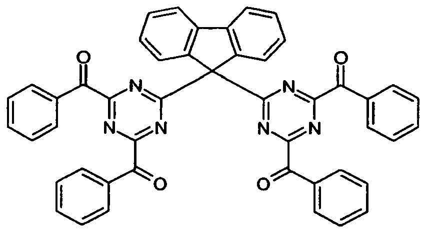 | 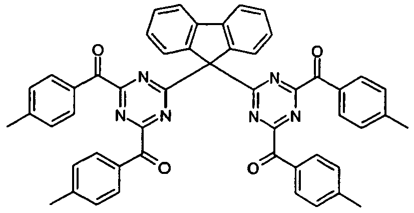 |
| (169) | (170) |
| 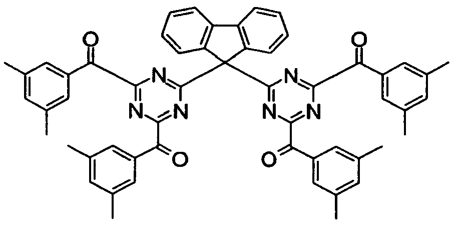 | 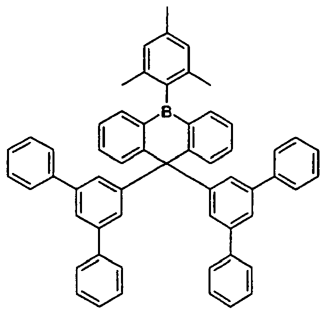 |
| (171) | (172) |
| 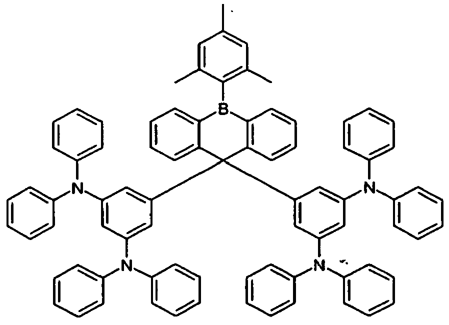 | 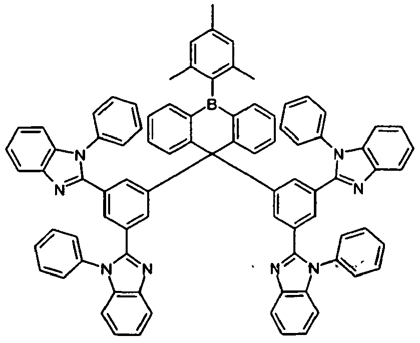 |
| (173) | (174) |
| 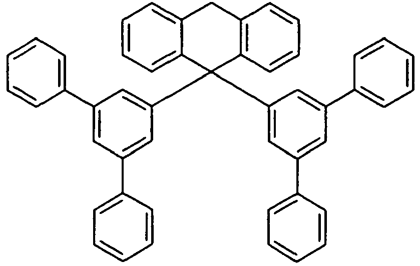 | 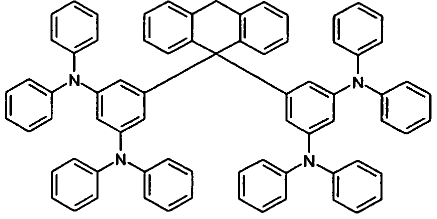 |
| (175) | (176) |
| 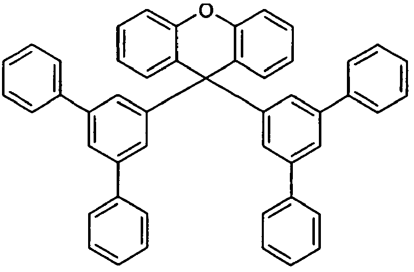 | 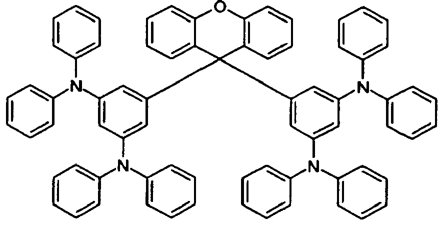 |
| (177) | (178) |
| 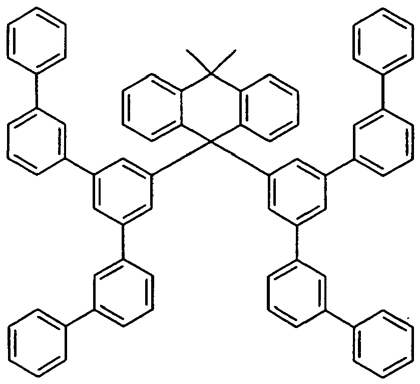 | 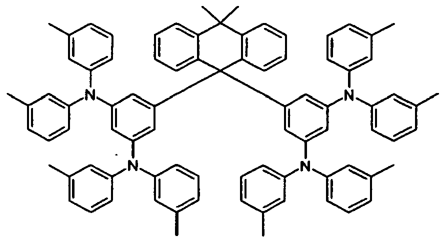 |
| (179) | (180) |
| 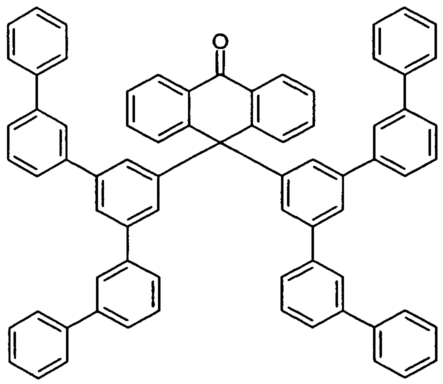 | 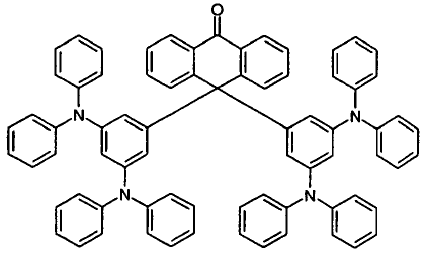 |
| (181) | (182) |
| 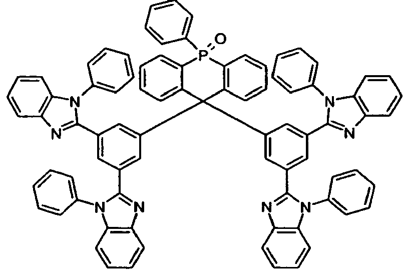 | 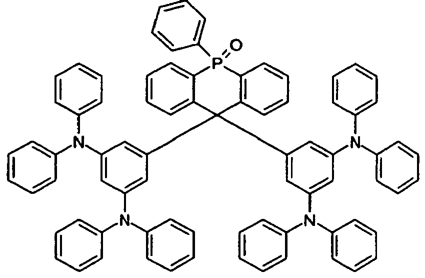 |
| (183) | (184) |
| 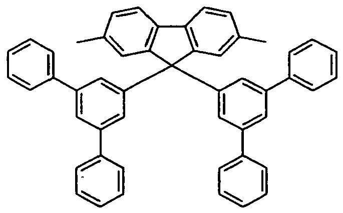 | 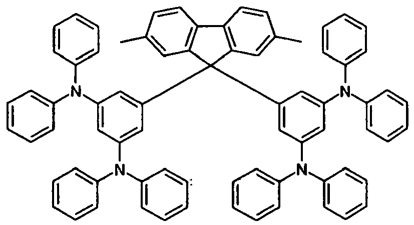 |
| (185) | (186) |
| 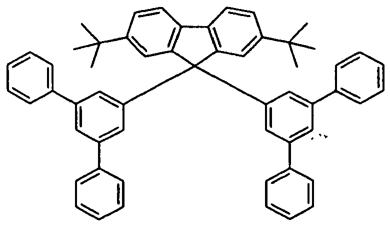 | 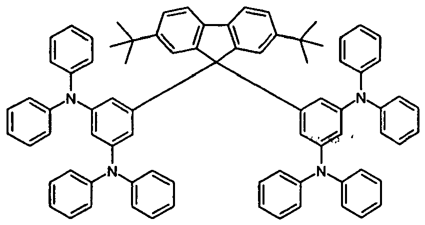 |
| (187) | (188) |
| 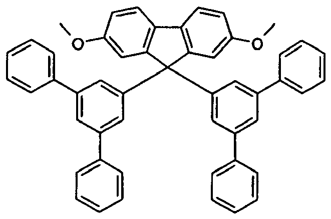 | 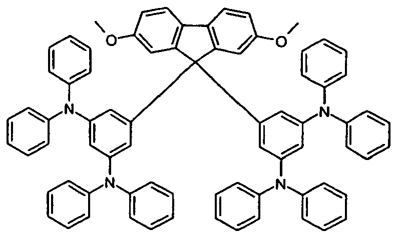 |
| (189) | (190) |
| 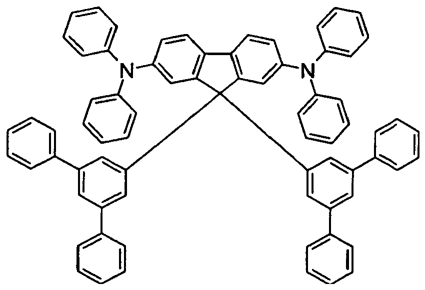 | 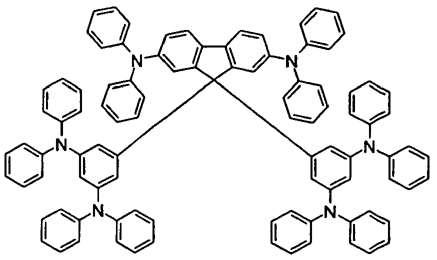 |
| (191) | (192) |
| 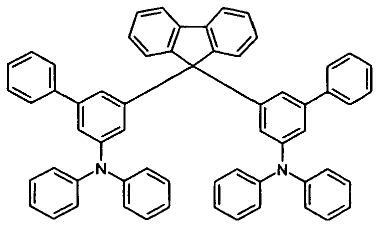 | 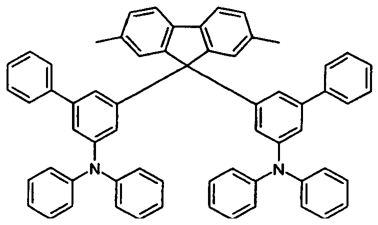 |
| (193) | (194) |
| 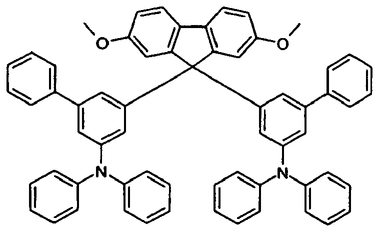 | 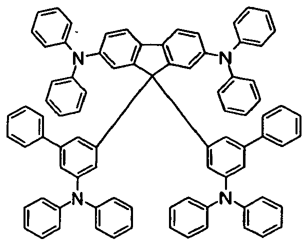 |
| (195) | (196) |
| 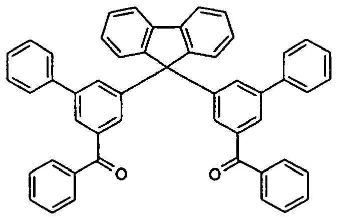 | 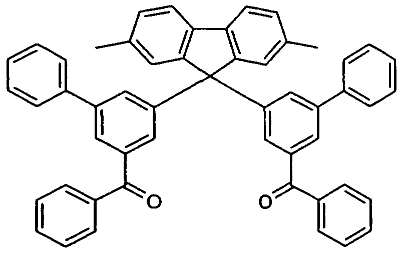 |
| (197) | (198) |
| 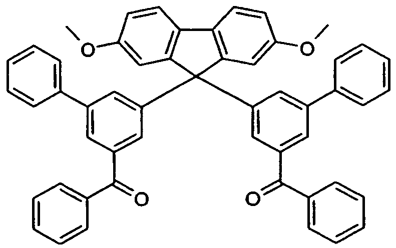 | 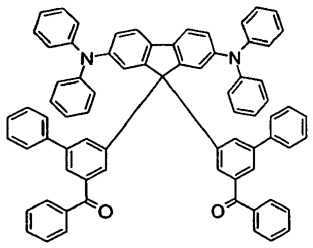 |
| (199) | (200) |
| 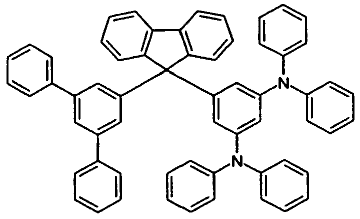 | 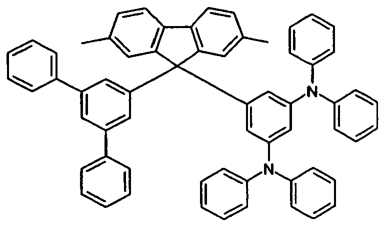 |
| (201) | (202) |
| 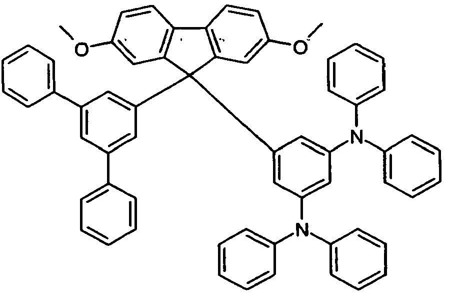 | 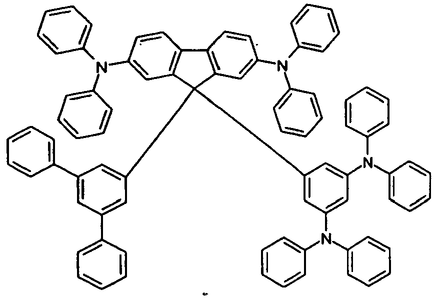 |
| (203) | (204) |
| 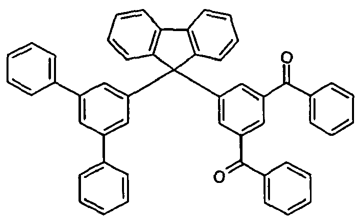 | 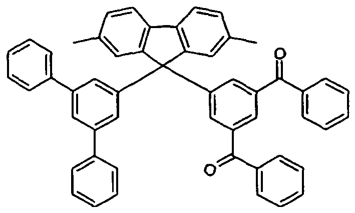 |
| (205) | (206) |
| 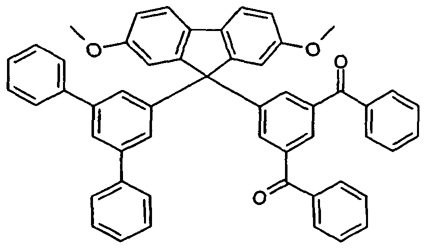 | 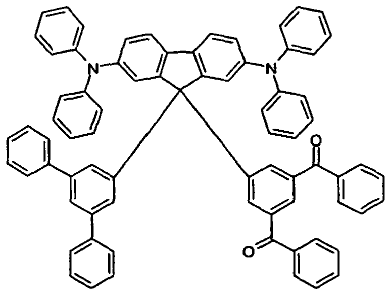 |
| (207) | (208) |
| 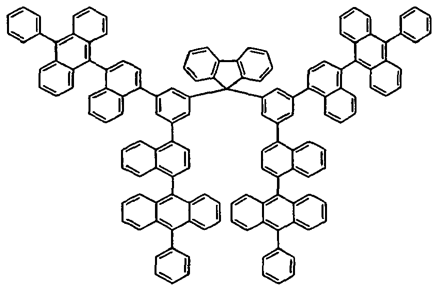 | 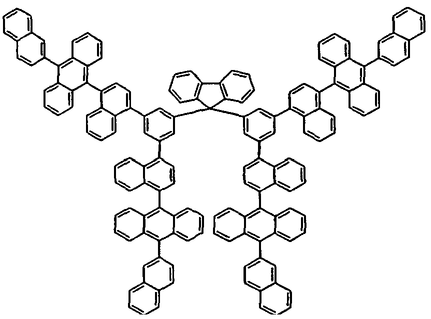 |
| (209) | (210) |
| 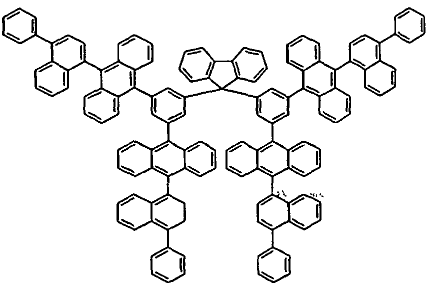 | 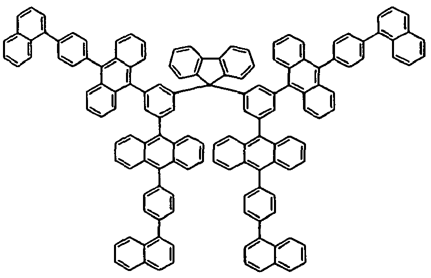 |
| (211) | (212) |
| 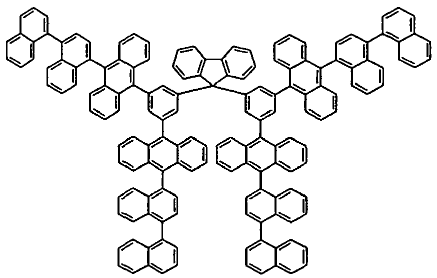 | 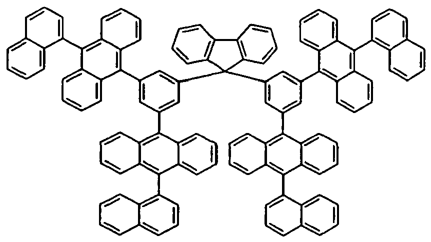 |
| (213) | (214) |
| 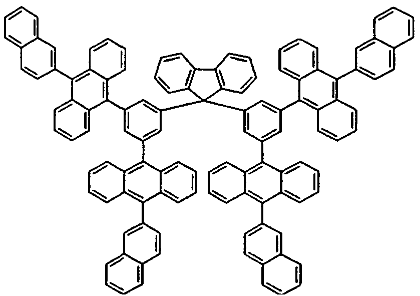 | 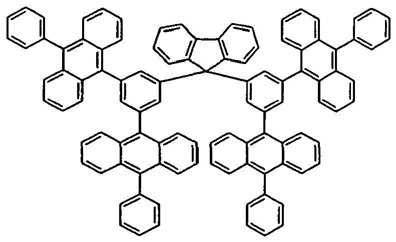 |
| (215) | (216) |
| 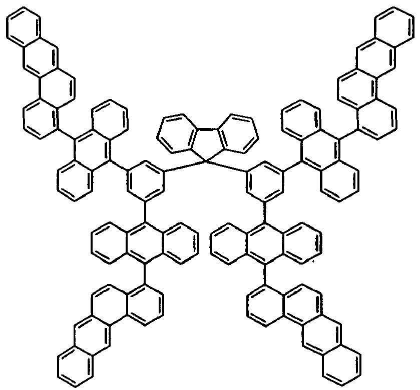 | 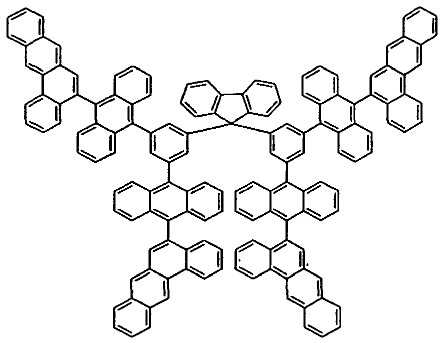 |
| (217) | (218) |
| 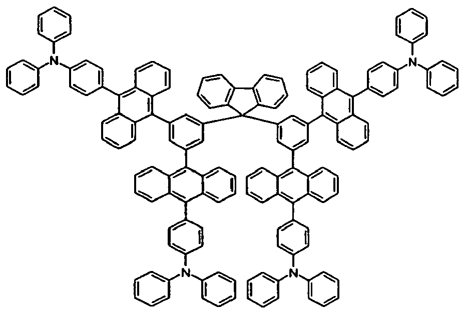 | 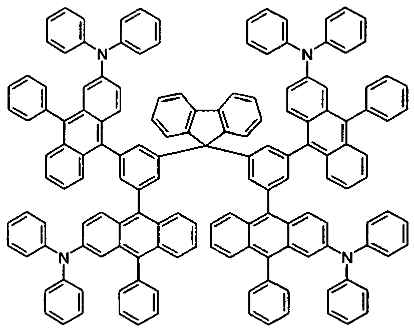 |
| (219) | (220) |
| 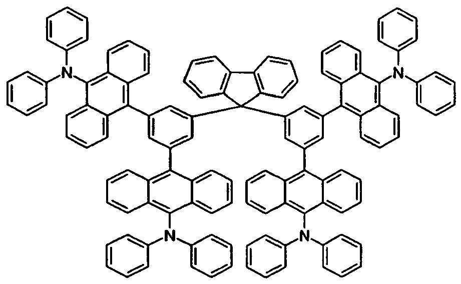 | 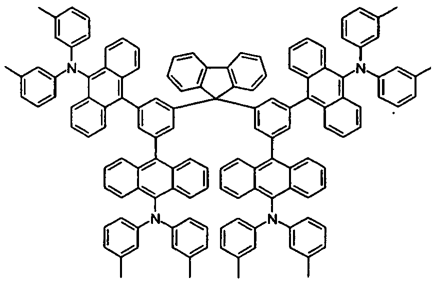 |
| (221) | (222) |
| 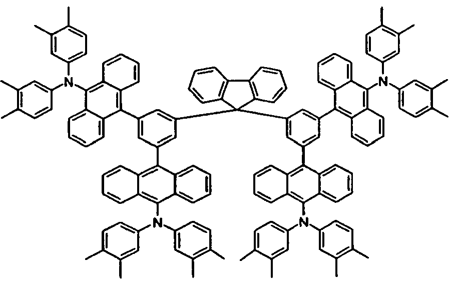 | 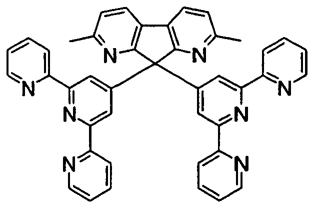 |
| (223) | (224) |
| 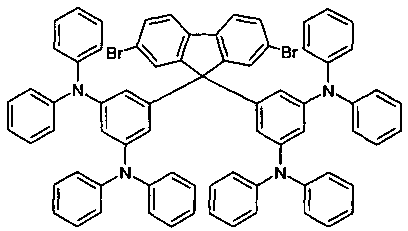 | 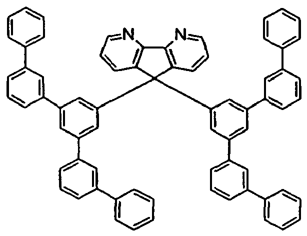 |
| (225) | (226) |
| 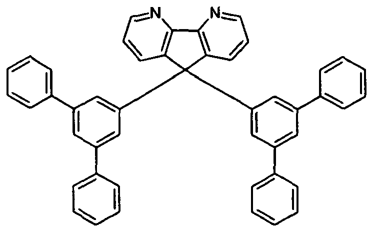 | 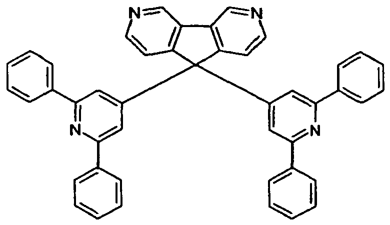 |
| (227) | (228) |
| 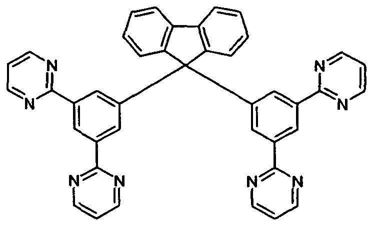 | 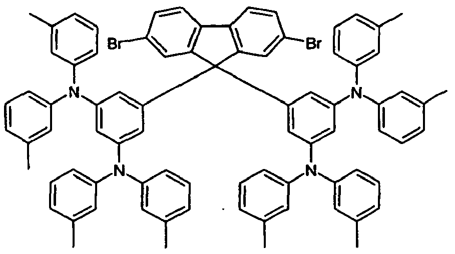 |
| (229) | (230) |
| 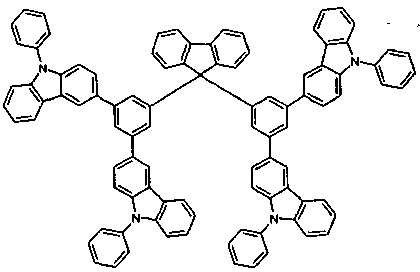 | 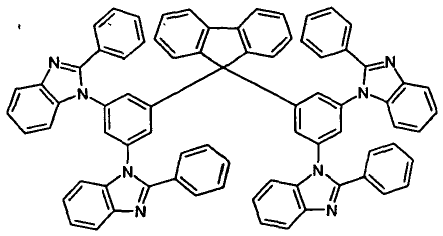 |
| (231) | (232) |
| 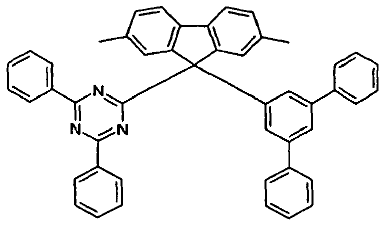 | 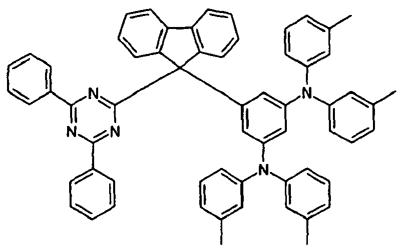 |
| (233) | (234) |
| 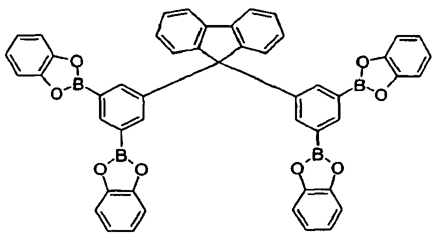 | 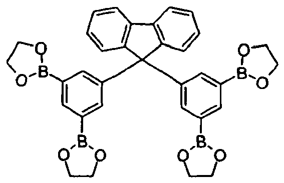 |
| (235) | (236) |
| 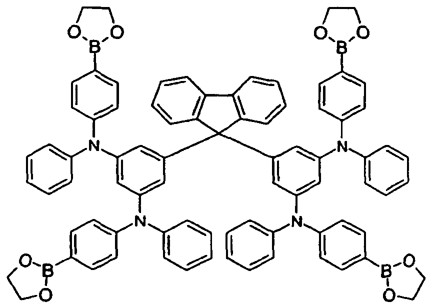 | 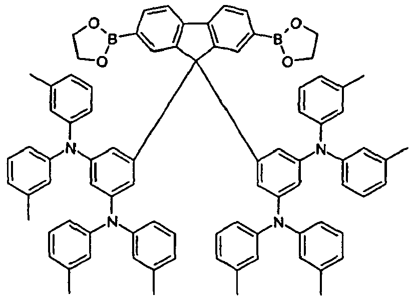 |
| (237) | (238) |
| 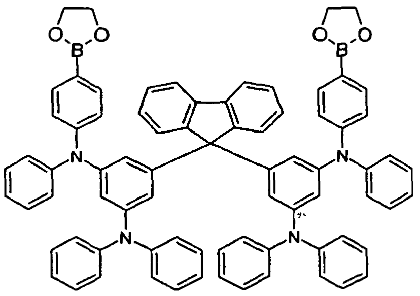 | 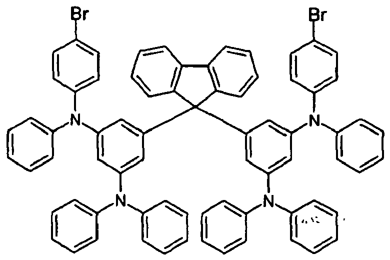 |
| (239) | (240) |
| 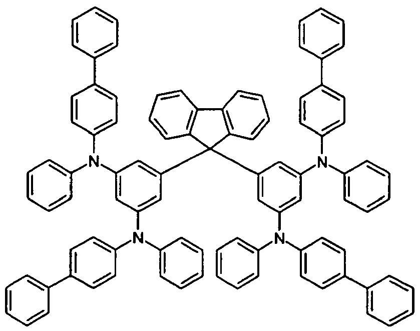 | 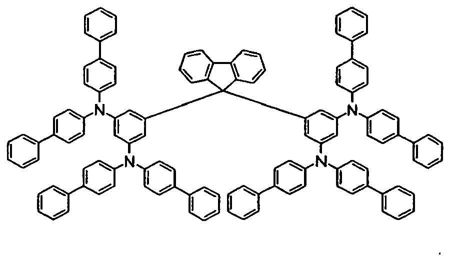 |
| (241) | (242) |
| 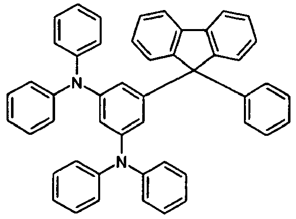 | 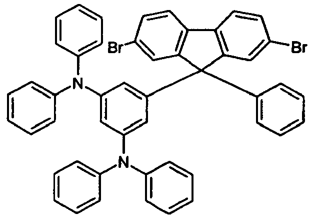 |
| (243) | (244) |
| 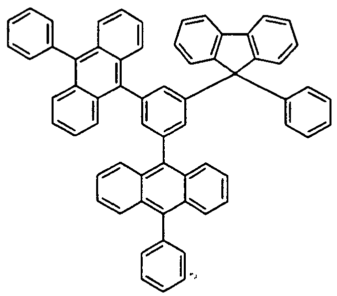 | 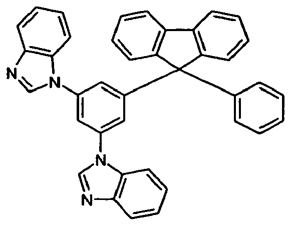 |
| (245) | (246) |
| 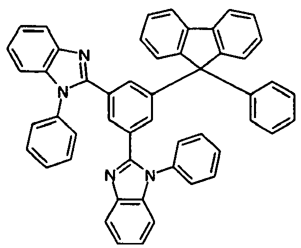 | 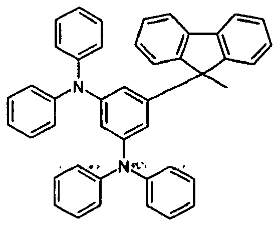 |
| (247) | (248) |
| 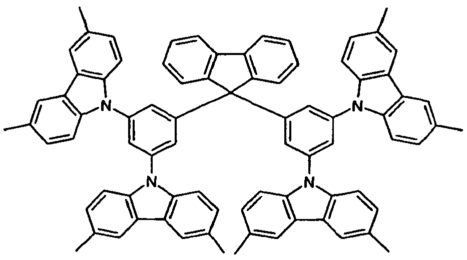 | 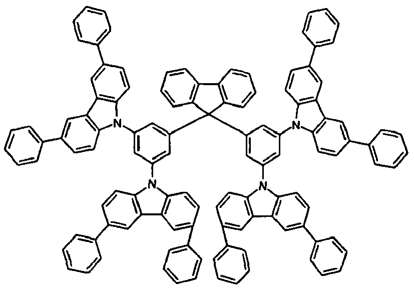 |
| (249) | (250) |
| 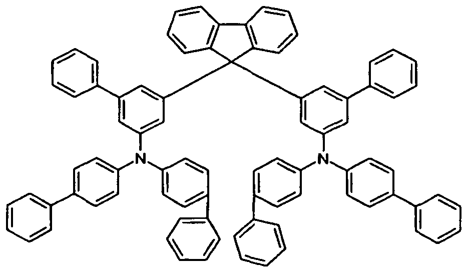 | 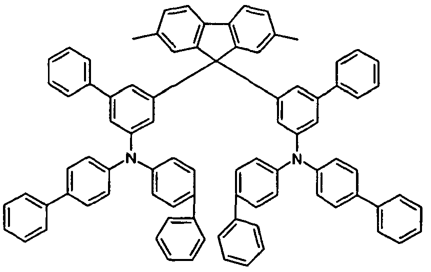 |
| (251) | (252) |
| 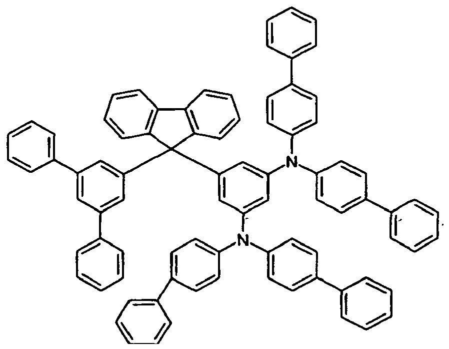 | 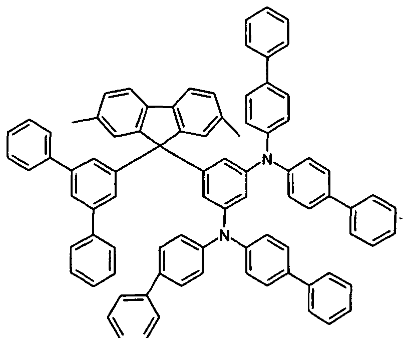 |
| (253) | (254) |
| 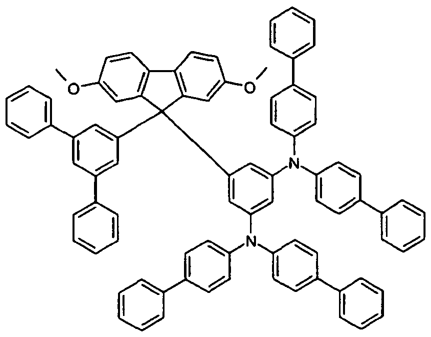 | 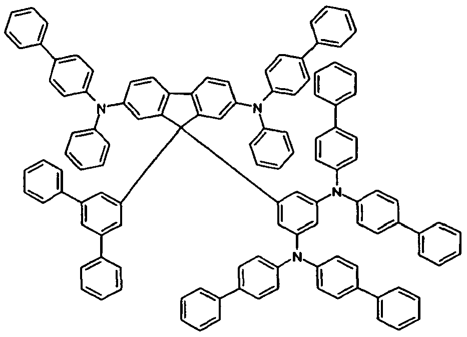 |
| (255) | (256) |
| 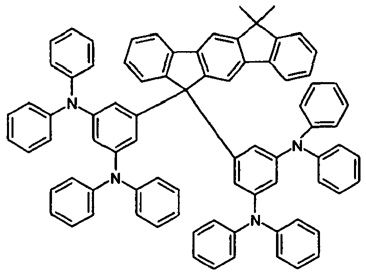 | 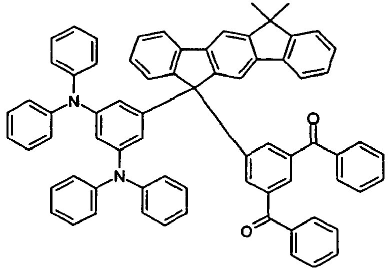 |
| (257) | (258) |
| 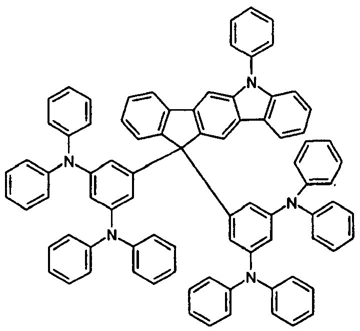 | 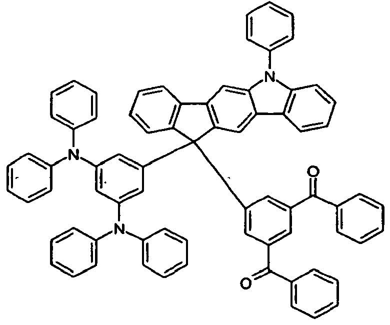 |
| (259) | (260) |
| 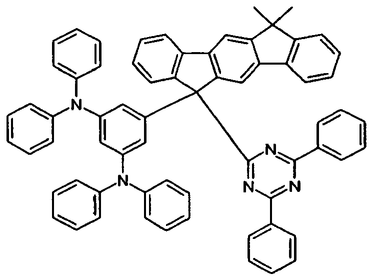 | 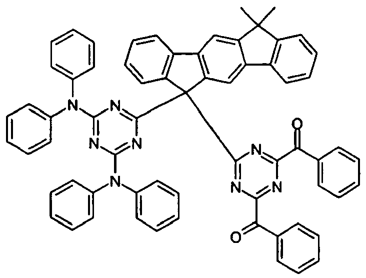 |
| (261) | (262) |
| 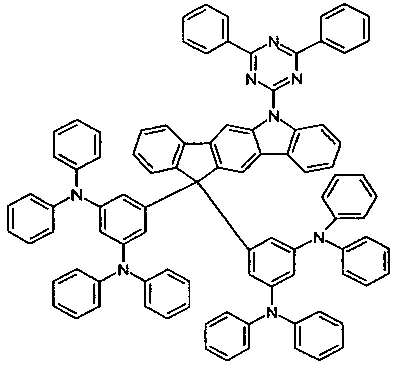 | 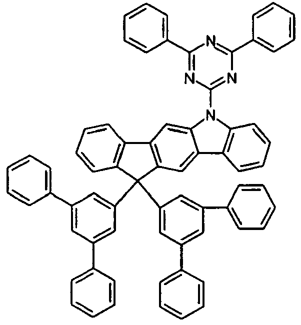 |
| (263) | (264) |
| 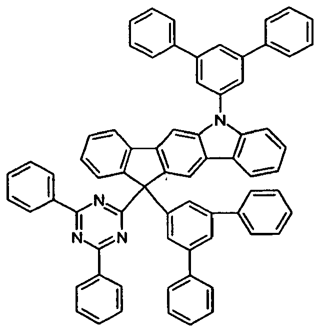 | 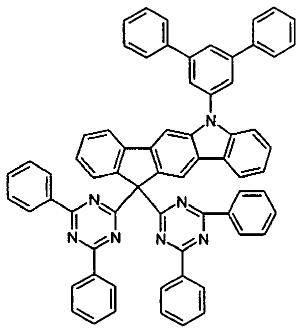 |
| (265) | (266) |
| 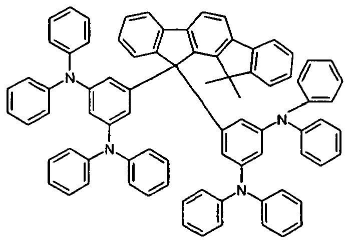 | 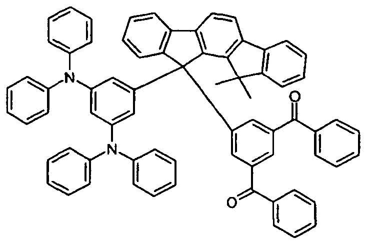 |
| (267) | (268) |
| 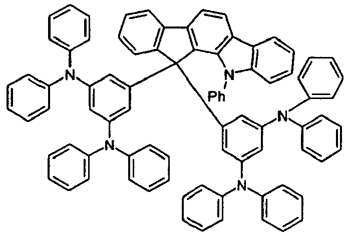 | 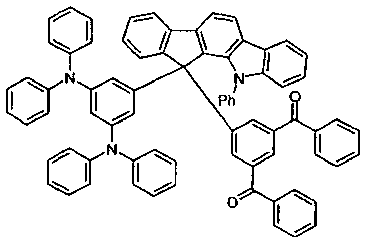 |
| (269) | (270) |
| 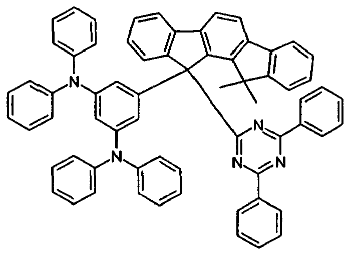 | 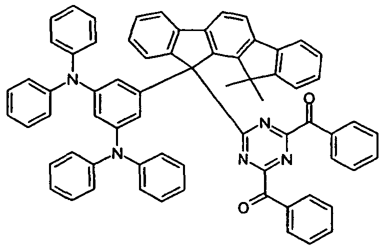 |
| (271) | (272) |
| 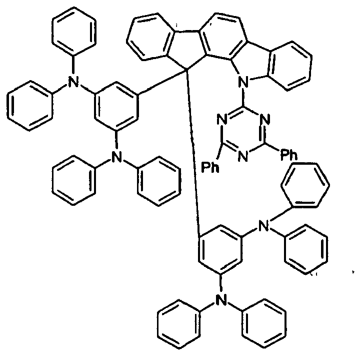 | 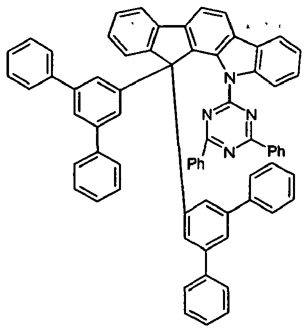 |
| (273) | (274) |
| 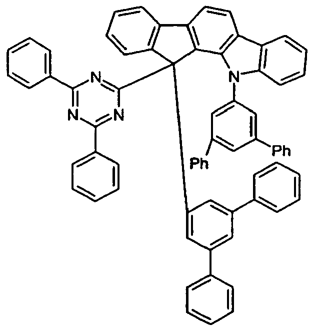 | 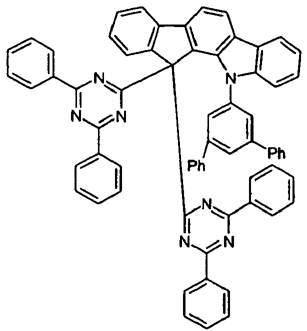 |
| (275) | (276) |

Die erfindungsgemäßen Verbindungen gemäß Formel (1) können nach dem Fachmann allgemein bekannten Syntheseschritten dargestellt werden. Als Ausgangsverbindung zu symmetrisch substituierten erfindungsgemäßen Verbindungen kann z. B. 3,3',5,5'-Tetrabrombenzophenon (Eur. J. Org. Chem. 2006, 2523-2529) dienen. Dieses kann z. B. gemäß Schema 1 durch Umsetzung mit einem substituierten oder unsubstituierten 2-Lithiobiphenyl, 2-Lithiodiphenylether, 2-Lithiodiphenylthioether, 2-(2-Lithiophenyl)-2-phenyl-1,3-dioxolan oder 2-Lithiophenyldiphenylamin zu den entsprechenden Triarylmethanolen umgesetzt werden, die dann sauer, z. B. in Gegenwart von Essigsäure und einer Mineralsäure wie Bromwasserstoff, cyclisiert werden. Die für diese Umsetzung benötigten Organolithiumverbindungen können durch Transmetallierung der entsprechenden Arylbromide (2-Brombiphenyl, 2-Bromdiphenylether, 2-Bromdiphenylthioether, 2-(2-Bromphenyl)-2-phenyl-1,3-dioxolan, 2-Bromphenyl-diphenylamin, etc.) mit Alkyllithiumverbindungen, wie *n*-Butyllithium, dargestellt werden. Analog dazu ist es selbstverständlich möglich, die entsprechenden Grignard-Verbindungen einzusetzen.

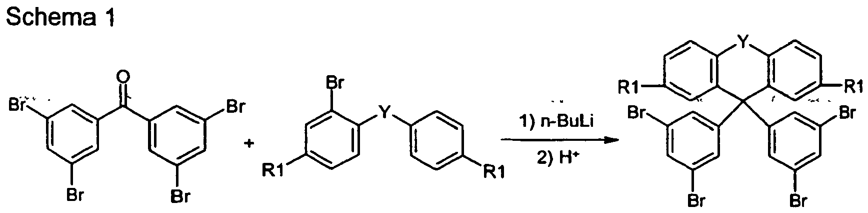

Die so erzeugten Tetrabromide können nach dem Fachmann bekannten Methoden weiter umgewandelt werden. Die palladiumkatalysierte Umsetzung mit Boronsäuren (Suzuki-Kuppung) oder palladiumkatalysierte Umsetzung mit Organozinkverbindungen (Negishi-Kupplung) führt zu erfindungsgemäßen aromatischen oder heteroaromatischen Verbindungen (Schema 2).

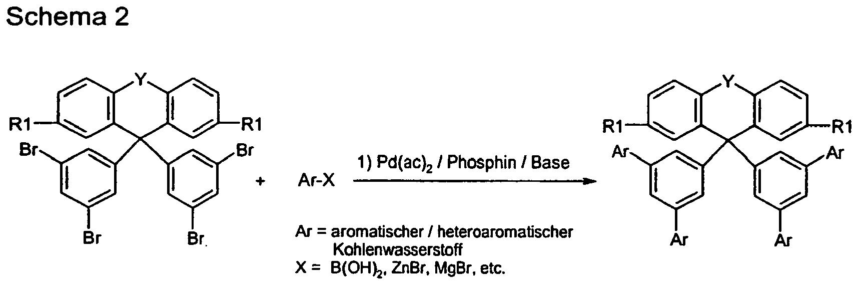

Die palladiumkatalysierte Umsetzung mit Aminen (Hartwig-Buchwald-Kupplung) führt zu erfindungsgemäßen aromatischen oder heteroaromatischen Aminen (Schema 3).

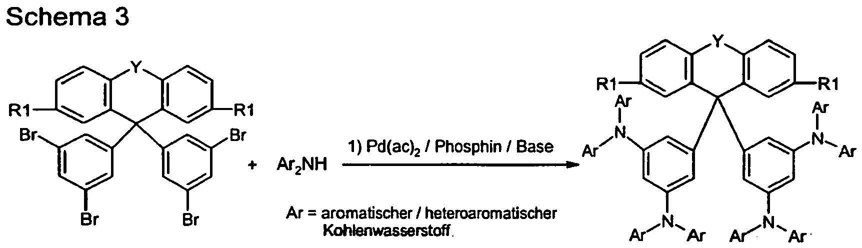

Die Bromfunktion kann durch Transmetallierung mit Organolithiumverbindungen bzw. Grignardverbindungen in eine elektrophile Gruppe überführt werden, die dann mit einer Vielzahl von Elektrophilen, wie z. B. Aryl-Bor-Halogeniden, Aldehyden, Ketonen, Nitrilen, Estern, Halogenestern, Kohlendioxid, Arylphosphinhalogeniden, Halogensulfinsäuren, Halogenarylsulfonsäuren, etc., gekoppelt werden, wobei die so erhaltenen Verbindungen erfindungsgemäße Endprodukte oder aber Intermediate sein können, die weiter umgesetzt werden können. Dies ist exemplarisch am Beispiel der Herstellung eines erfindungsgemäßen Ketons, eines Phosphinoxids und eines Benzimidazols verdeutlicht (Schema 4). 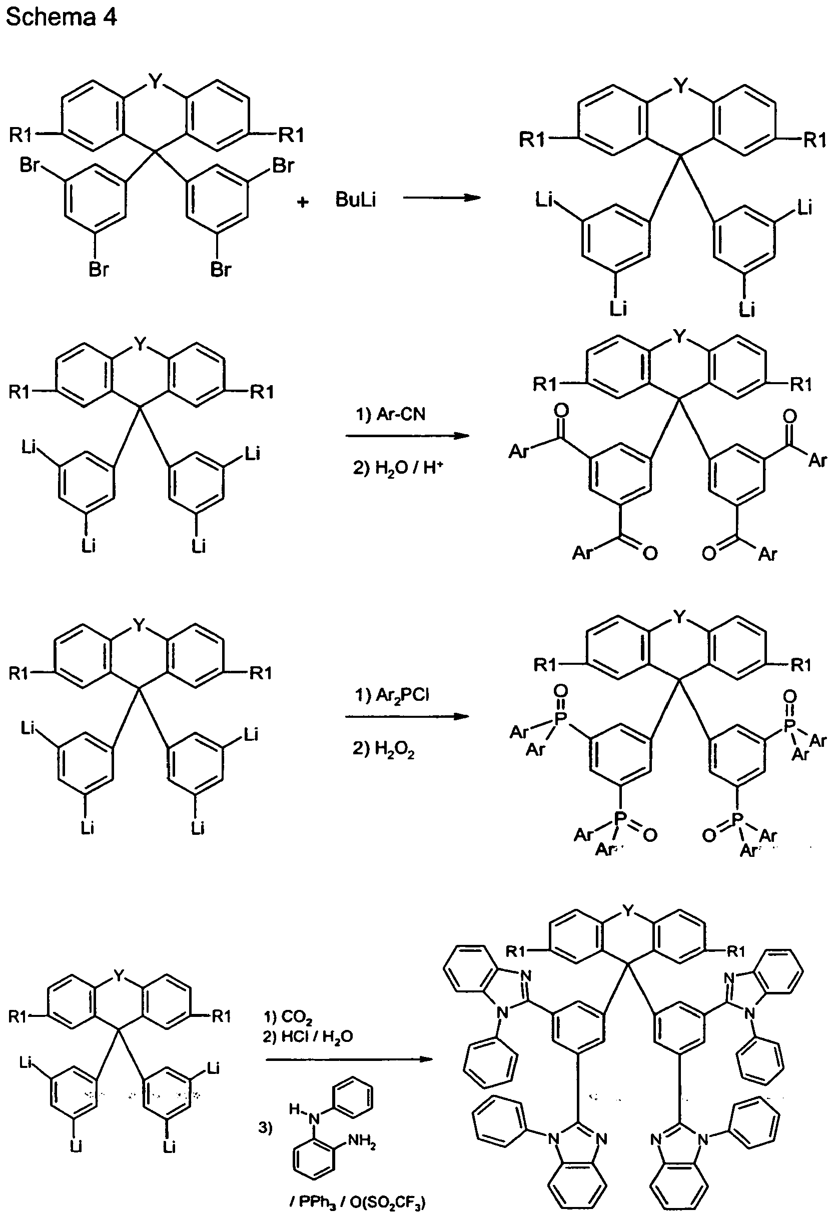 Unsymmetrisch substituierte erfindungsgemäße Verbindungen können durch die Sequenz gemäß Schema 5 ausgehend von Fluorenon und analogen Arylketonen durch Addition einer Aryl-Metallverbindung, z. B. 1-Lithio-3,5-dibrombenzol, an die Carbonylfunktion, Umwandlung des Bromaromaten nach einer der oben genannten Methoden unter Aufbau der einen Funktionalität und anschließender Einführung der anderen Funktionalität via säurekatalysierter Friedel-Crafts-Arylierung an 1,3-Dibrombenzol und Umwandlung des Bromaromaten nach einer der oben genannten Methoden erhalten werden (s. z. B. Org. Lett. 2001, 3(15), 2285.).

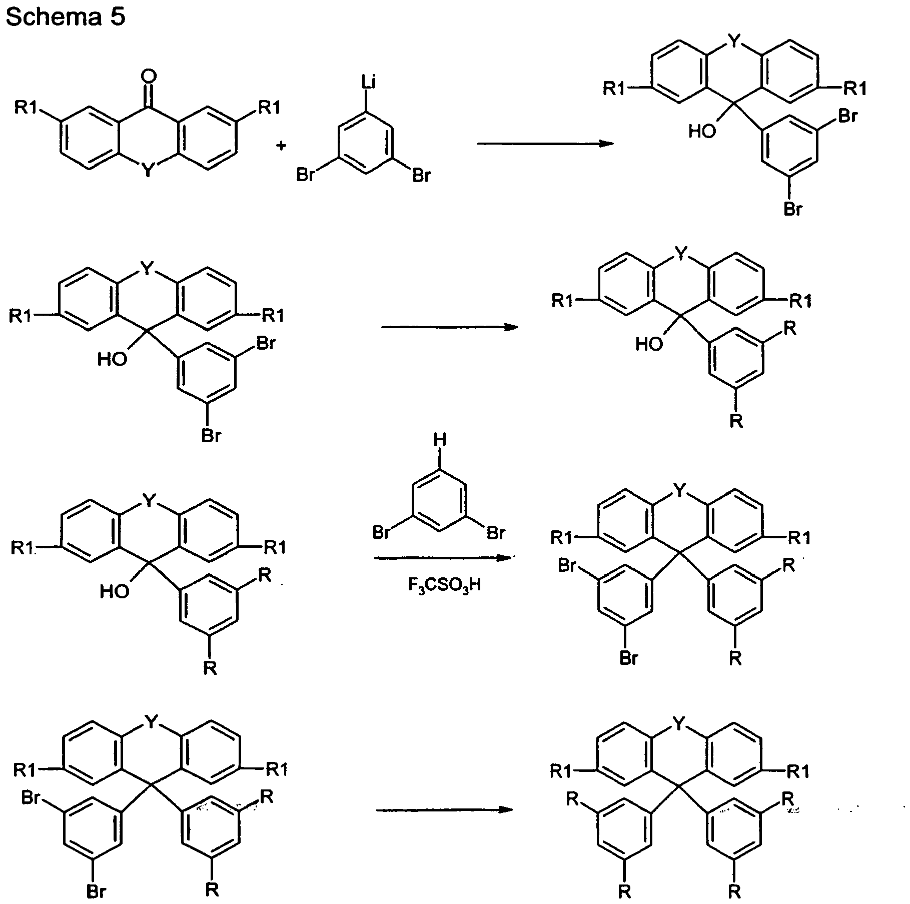

Die entsprechenden Indenofluorenderivate, Indenocarbazolderivate und die weiteren Derivate der Formel (1) lassen sich entsprechend synthetisieren.

Ein weiterer Gegenstand der Erfindung ist ein Verfahren zur Herstellung der Verbindungen gemäß Formel (1) umfassend die Reaktion von Bis(3,5-dibrom)benzophenon mit einem substituierten oder unsubstituierten 2-Lithiobiphenyl, 2-Lithiodiphenylether, 2-Lithiodiphenylthioether, 2-(2-Lithiophenyl)-2-phenyl-1,3-dioxolan, 2-Lithiophenyldiphenylamin oder der entsprechenden Grignardverbindung zu den Triarylmethanolen, gefolgt von Cyclisierung unter sauren Bedingungen und gegebenenfalls gefolgt von weiterer Umsetzung der Bromgruppen.

Die oben beschriebenen erfindungsgemäßen Verbindungen, insbesondere Verbindungen, welche mit reaktiven Abgangsgruppen, wie Brom, Iod, Triflat, Tosylat, Boronsäure oder Boronsäureester, substituiert sind, können als Monomere zur Erzeugung entsprechender Dimere, Trimere, Tetramere, Pentamere, Oligomere, Polymere oder als Kern von Dendrimeren Verwendung finden. Die Oligomerisation bzw. Polymerisation erfolgt dabei bevorzugt über die Halogenfunktionalität bzw. die Boronsäurefunktionalität. Dies gilt insbesondere für Verbindungen gemäß Formel (4), in denen die Reste R¹ jeweils für eine reaktive Abgangsgruppe stehen, insbesondere ausgewählt aus den oben genannten Gruppen.

Weiterer Gegenstand der Erfindung sind daher Dimere, Trimere, Tetramere, Pentamere, Oligomere, Polymere oder Dendrimere enthaltend eine oder mehrere Verbindungen gemäß Formel (1), wobei ein oder mehrere Reste R¹ oder R² Bindungen zwischen den Verbindungen gemäß Formel (1) im Dimer, Trimer, Tetramer bzw. Pentamer bzw. Bindungen der Verbindung gemäß Formel (1) zum Polymer, Oligomer oder Dendrimer darstellen oder wobei diese Bindung über Substituenten an den Gruppen R erfolgt. Unter einem Oligomer im Sinne dieser Erfindung wird eine Verbindung verstanden, welche mindestens sechs Einheiten gemäß Formel (1) aufweist. Die Polymere, Oligomere oder Dendrimere können konjugiert, teilkonjugiert oder nicht-konjugiert sein. Die Trimere, Tetramere, Pentamere, Oligomere oder Polymere können linear oder verzweigt sein. In den linear verknüpften Strukturen können die Einheiten gemäß Formel (1) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe, miteinander verknüpft sein. In verzweigten Strukturen können beispielsweise drei oder mehrere Einheiten gemäß Formel (1) über eine trivalente oder höhervalente Gruppe, beispielsweise über eine trivalente oder höhervalente aromatische oder heteroaromatische Gruppe, zu einem verzweigten Trimer, Tetramer, Pentamer, Oligomer oder Polymer verknüpft sein.

Für die Wiederholeinheiten gemäß Formel (1) in Dimeren, Trimeren, Tetrameren, Pentameren, Oligomeren und Polymeren gelten dieselben Bevorzugungen wie oben beschrieben. Bevorzugte Wiederholeinheiten sind daher auch hier die Einheiten gemäß den oben genannten Formeln.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis-und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 07/068325) oder phosphoreszierende Metallkomplexe (z. B. gemäß WO 06/003000), und/oder Ladungstransporteinheiten. Dabei eignen sich die erfindungsgemäßen Wiederholeineiten insbesondere als Ladungstransporteinheiten für Löcher, wenn eine oder mehrere Gruppen R für NAr₂ stehen.

Ein weiterer Gegenstand der vorliegenden Erfindung sind Mischungen enthaltend mindestens eine Verbindung gemäß Formel (1) oder ein entsprechendes Dimer, Trimer, Tetramer, Pentamer, Oligomer oder Polymer und mindestens eine weitere Verbindung. Die weitere Verbindung kann beispielsweise ein fluoreszierender oder phosphoreszierender Dotand sein, wenn die Verbindung der Formel (1) als Matrixmaterial verwendet wird. Geeignete fluoreszierende und phosphoreszierende Dotanden sind unten im Zusammenhang mit den organischen Elektrolumineszenzvorrichtungen aufgeführt und sind auch für die erfindungsgemäßen Mischungen bevorzugt. Die weitere Verbindung kann auch ein Dotierstoff sein, wenn die Verbindung gemäß Formel (1) eine Lochtransport- oder Elektronentransportverbindung ist. Geeignete Dotierstoffe sind unten im Zusammenhang mit den organischen Elektrolumineszenzvorrichtungen aufgeführt.

Nochmals ein weiterer Gegenstand der vorliegenden Erfindung sind Lösungen enthaltend mindestens eine Verbindung gemäß Formel (1) oder ein entsprechendes Dimer, Trimer, Tetramere, Pentamer, Oligomer oder Polymer und mindestens ein organisches Lösemittel. Derartige Lösungen sind erforderlich für der Herstellung der organischen elektronischen Vorrichtung aus Lösung, beispielsweise durch Spin-Coating oder durch Druckverfahren.

Die erfindungsgemäßen Verbindungen gemäß Formel (1) und entsprechende Dimere, Trimere, Tetramere, Pentamere, Oligomere, Polymere oder Dendrimere eignen sich für den Einsatz in elektronischen Vorrichtungen, insbesondere in organischen Elektrolumineszenzvorrichtungen (OLEDs, PLEDs). Abhängig von der Substitution werden die Verbindungen in unterschiedlichen Funktionen und Schichten eingesetzt. Dabei entsprechen die bevorzugten Ausführungsformen den oben genannten Formeln.

Ein weiterer Gegenstand der Erfindung ist daher die Verwendung von Verbindungen gemäß Formel (1) bzw. entsprechender Dimere, Trimere, Tetramere, Pentamere, Oligomere, Polymere oder Dendrimere in elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine Verbindung gemäß Formel (1) bzw. ein entsprechendes Dimer, Trimer, Tetramer, Pentamer, Oligomer, Polymer oder Dendrimer, insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) oder ein entsprechendes Dimer, Trimer, Tetramer, Pentamer, Oligomer, Polymer oder Dendrimer enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Charge-Generation Layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

In einer weiteren bevorzugten Ausführungsform der Erfindung enthält die organische Elektrolumineszenzvorrichtung mehrere emittierende Schichten, wobei mindestens eine organische Schicht mindestens eine Verbindung gemäß Formel (1) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

In einer bevorzugten Ausführungsform der Erfindung werden die Verbindungen gemäß Formel (1) als Matrixmaterial für fluoreszierende oder phosphoreszierende Verbindungen in einer emittierenden Schicht eingesetzt. Bei einem Matrixmaterial für phosphoreszierende Verbindungen stehen bevorzugt eine oder mehrere Gruppen R und/oder R¹ für C(=O)Ar, N(Ar)₂, S(=O)Ar, S(=O)₂Ar oder P(=O)Ar₂. Dieselben Bevorzugungen gelten für die Gruppen R bzw. R¹ in Strukturen gemäß Formel (2), (3) und (4). Bei einem Matrixmaterial für fluoreszierende Verbindungen stehen bevorzugt eine oder mehrere Gruppen R und/oder R¹ für ein aromatisches oder heteroaromatisches Ringsystem, insbesondere für ein aromatisches Ringsystem enthaltend Anthracen. Dieselben Bevorzugungen gelten für die Gruppen R bzw. R¹ in Strukturen gemäß den oben genannten Formeln.

Unter einem Matrixmaterial wird in einem System aus Matrix und Dotand diejenige Komponente verstanden, die in dem System im höheren Anteil vorliegt. Bei einem System aus einer Matrix und mehreren Dotanden wird als Matrix diejenige Komponente verstanden, deren Anteil der höchste in der Mischung ist.

In einer bevorzugten Ausführungsform der Erfindung wird als Matrix eine Mischung eingesetzt, wobei mindestens eine Komponente dieser Mischung eine Verbindung der Formel (1) ist. Bevorzugt ist eine Komponente dieser Mischung eine Lochtransportverbindung und die andere eine Elektronentransportverbindung. Bevorzugte Lochtransportverbindungen sind aromatische Amine und Carbazolderivate. Bevorzugte Elektronentransportverbindungen sind aromatische Ketone.

Wenn die Verbindung gemäß Formel (1) als Matrixmaterial für eine emittierende Verbindung in einer emittierenden Schicht eingesetzt wird, kann sie in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Erfindung werden alle insbesondere lumineszierenden Iridium-, Platin-, Osmium-, Gold- und Kupferverbindungen als phosphoreszierende Materialien bezeichnet. Die Mischung aus der Verbindung gemäß Formel (1) und der emittierenden Verbindung enthält dann zwischen 99 und 1 Gew.-%, vorzugsweise zwischen 98 und 10 Gew.-%, besonders bevorzugt zwischen 97 und 60 Gew.-%, insbesondere zwischen 95 und 85 Gew.-% der Verbindung gemäß Formel (1) bezogen auf die Gesamtmischung aus Emitter und Matrixmaterial. Entsprechend enthält die Mischung zwischen 1 und 99 Gew.-%, vorzugsweise zwischen 2 und 90 Gew.-%, besonders bevorzugt zwischen 3 und 40 Gew.-%, insbesondere zwischen 5 und 15 Gew.-% des Emitters bezogen auf die Gesamtmischung aus Emitter und Matrixmaterial.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 01/41512, WO 02/02714, WO 02/15645, EP 1191613, EP 1191612, EP 1191614 und WO 05/033244 entnommen werden. Weiterhin eignen sich als Emitter die oben aufgeführten erfindungsgemäßen Verbindungen. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Wenn die Verbindung gemäß Formel (1) als Matrixmaterial für fluoreszierende Verbindungen eingesetzt wird, beträgt der Anteil des Matrixmaterials in der emittierenden Schicht zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden zwischen 0.1 und 50.0 Gew.-%, bevorzugt zwischen 0.1 und 20.0 Gew.-%, besonders bevorzugt zwischen 0.5 und 15 Gew.-%, ganz besonders bevorzugt zwischen 1.0 und 10.0 Gew.-%.

Bevorzugte Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind gewählt aus Indenofluorenaminen bzw. -diaminen, beispielsweise gemäß WO 06/122630, Benzoindenofluorenaminen bzw. -diaminen, beispielsweise gemäß WO 08/006449, und Dibenzoindenofluorenaminen bzw. -diaminen, beispielsweise gemäß WO 07/140847. Beispiele für Dotanden aus der Klasse der Styrylamine sind substituierte oder unsubstituierte Tristilbenamine oder die Dotanden, die in WO 06/000388, WO 06/058737, WO 06/000389, WO 07/065549 und WO 07/115610 beschrieben sind.

In einer weiteren Ausführungsform der Erfindung werden die Verbindungen gemäß Formel (1) als Lochtransportmaterial bzw. als Lochinjektionsmaterial bzw. als Elektronenblockiermaterial bzw. als Excitonenblockiermaterial eingesetzt. Die Verbindungen sind dann bevorzugt mit mindestens einer Gruppe N(Ar)₂ substituiert, bevorzugt mit mindestens zwei Gruppen N(Ar)₂ und/oder sie enthalten weitere Gruppen, die den Lochtransport verbessern. Besonders bevorzugt stehen hier alle Gruppen R für N(Ar)₂. Die Gruppen N(Ar)₂ sind bevorzugt ausgewählt aus den oben beschriebenen Formeln (5) oder (6). Dies gilt insbesondere für die Reste R an den Strukturen gemäß den oben genannten Formeln. Weitere bevorzugte Gruppen, die den Lochtransport verbessern, sind beispielsweise die Gruppen N(R¹), S oder O, insbesondere N(R¹) als Brücke Y oder elektronenreiche Heteroaromaten, insbesondere Thiophen, Pyrrol oder Furan als Gruppe R oder R¹. Die Verbindung wird bevorzugt in einer Lochtransport- bzw. in einer Lochinjektions- bzw. in einer Elektronenblockier- bzw. in einer Excitonenblockierschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Eine Elektronenblockier- bzw. Excitonenblockierschicht im Sinne dieser Erfindung ist eine Schicht, die auf Anodenseite direkt an eine emittierende Schicht angrenzt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

In nochmals einer weiteren Ausführungsform der Erfindung werden die Verbindungen gemäß Formel (1) als Elektronentransportmaterial bzw. als Lochblockiermaterial in einer Elektronentransportschicht bzw. einer Lochblockierschicht eingesetzt. Hier ist es bevorzugt, wenn die Gruppe Y für C=O, P(=O), SO oder SO₂ steht und/oder mindestens einer der Substituenten R und/oder R¹ für eine Heteroarylgruppe, welche einen elektronenarmen Heterocyclus darstellt, wie beispielsweise Imidazol, Pyrazol, Thiazol, Benzimidazol, Benzothiazol, Triazol, Oxadiazol, Benzothiadiazol, Phenanthrolin, etc., oder für C(=O)Ar, P(=O)Ar₂, S(=O)Ar oder S(O)₂Ar steht. Weiterhin kann es bevorzugt sein, wenn die Verbindung mit Elektronendonorverbindungen dotiert ist. Eine Lochblockierschicht im Sinne dieser Erfindung ist eine Schicht, die auf zwischen einer emittierenden Schicht und einer Elektronentransportschicht liegt und direkt an die emittierende Schicht angrenzt. Wenn die Verbindung gemäß Formel (1) als Elektronentransportmaterial eingesetzt wird, kann es bevorzugt sein, diese als Mischung mit einer weiteren Verbindung einzusetzen. Bevorzugte Mischungskomponenten sind Alkalimetallverbindungen, bevorzugt Lithiumverbindungen, besonders bevorzugt Liq (Lithiumchinolinat) bzw. Liq-Derivate.

Auch in Polymeren können Wiederholeinheiten gemäß Formel (1) entweder als Polymergrundgerüst (Backbone), als lochtransportierende Einheit und/oder als elektronentransportierende Einheit eingesetzt werden. Dabei entsprechen die bevorzugten Substitutionsmuster den oben beschriebenen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar aufgedampft. Es sei jedoch angemerkt, dass der Druck auch noch geringer sein kann, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen. Dabei können nicht nur Lösungen aus einzelnen Materialien aufgebracht werden, sondern auch Lösungen, die mehrere Verbindungen enthalten, beispielsweise Matrixmaterial und Dotand.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen weisen eine hohe thermische Stabilität auf und lassen sich unzersetzt sublimieren.
2. Die erfindungsgemäßen Verbindungen, insbesondere solche, die Diarylaminosubstituenten als Gruppen R enthalten, führen bei Verwendung in einer Elektronen-/Excitonenblockierschicht in einer phosphoreszierenden Elektrolumineszenzvorrichtung zu einer erheblichen Verbesserung der Effizienz gegenüber Materialien gemäß dem Stand der Technik.
3. Die erfindungsgemäßen Verbindungen, insbesondere solche, welche mit Diarylaminogruppen substituitert sind und/oder welche eine Einfachbindung oder S, O oder N(R¹) als Gruppe Y enthalten und/oder welche mit elektronenreichen Heteroaromaten substituiert sind, eignen sich sehr gut für die Verwendung als Lochinjektions- und Lochtransportmaterial und führen zu einer Verringerung der Betriebsspannung.
4. Die mit den erfindungsgemäßen Verbindungen hergestellten OLEDs weisen generell eine sehr hohe Lebensdauer auf.
5. Die mit den erfindungsgemäßen Verbindungen hergestellten OLEDs weisen generell eine sehr hohe Quanteneffizienz auf.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch in anderen elektronischen Vorrichtungen einzusetzen, z. B. in organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele genauer beschrieben, ohne sie dadurch einschränken zu wollen. Der Fachmann kann, ohne erfinderisch tätig zu werden, weitere erfindungsgemäße Verbindungen herstellen und diese in organischen elektronischen verwenden.

### Beispiele:

Die nachfolgenden Synthesen werden - sofern nicht anders angegeben - unter einer Schutzgasatmosphäre in getrockneten Lösungsmitteln durchgeführt. Die Lösungsmittel und Reagenzien können von den Firmen ALDRICH bzw. ABCR bezogen werden. Die Vorstufe 3,3',5,5'-Tetrabrombenzophenon wird gemäß Eur. J. Org. Chem. 2006, 2523-2529 hergestellt.

### Beispiel 1: Synthese von 9,9-Bis-(3,5-dibromphenyl)fluoren

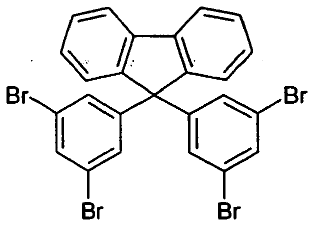

Aus 144.5 g (620 mmol) 2-Brombiphenyl und 15.3 g (580 mmol) Magnesium wird in einem Gemisch aus 500 ml Tetrahydrofuran und 250 ml Dimethoxyethan die entsprechende Grignard-Verbindung hergestellt. Dann wird bei Raumtemperatur mit einer Suspension von 224.0 g (450 mmol) Bis-(3,5-dibromphenyl)keton in 1000 ml Tetrahydrofuran versetzt und zwölf Stunden nachgerührt. Das Lösungsmittel wird im Vakuum entfernt, der Rückstand mit 1000 ml Eisessig und 5 ml Bromwasserstoff versetzt und eine Stunde gerührt. Die Suspension wird eine halbe Stunde unter Rückfluss erhitzt und zwölf Stunden bei Raumtemperatur gerührt. Der Feststoff wird abgesaugt, dreimal mit 300 ml Ethanol gewaschen und zweimal aus Toluol umkristallisiert. Ausbeute: 183.2 g (289 mmol), 64.3 %, Reinheit ca. 99.8 % (HPLC).

Analog zu Beispiel 1 werden aus entsprechenden Bromiden folgende erfindungsgemäße Verbindungen erhalten (Beispiele 2 und 3):

| Bsp. | Bromid | Produkt | Ausbeute |
|---|---|---|---|
| 2 | 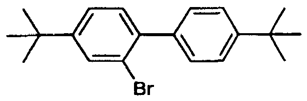 | 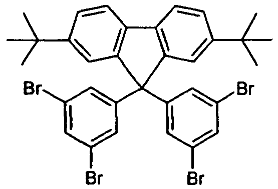 | 74.8 % |
| 3 | 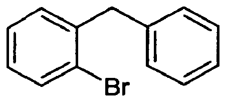 | 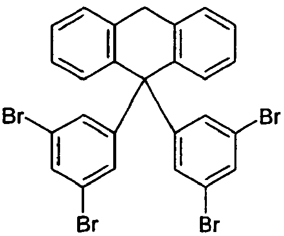 | 58.7 % |

### Beispiel 4: Synthese von 9,9-Bis-(3,5-diphenylphenyl)fluoren

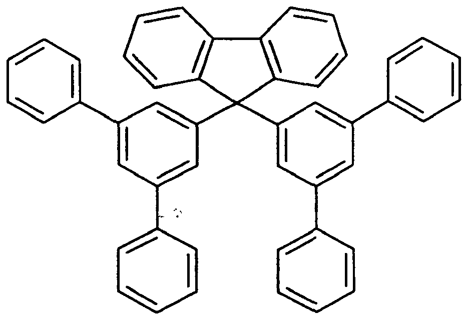

Eine gut gerührte Suspension von 30.4 g (48 mmol) 9,9-Bis-(3,5-dibromphenyl)fluoren, 35.4 g (290 mmol) Phenylboronsäure und 121.0 g (570 mmol) Trikaliumphosphat in einem Gemisch von 300 ml Toluol, 300 ml 1,4-Dioxan und 300 ml Wasser wird mit 1.0 g (3.3 mmol) Tri-o-tolylphosphin und 0.5 g (2.2 mmol) Palladium(II)acetat versetzt und anschließend drei Stunden unter Rückfluss erhitzt. Nach Erkalten wird die organische Phase abgetrennt, dreimal mit je 150 ml Wasser gewaschen und über Silicagel filtriert. Das Lösungsmittel wird im Vakuum entfernt, der Rückstand mit 200 ml Ethanol aufgenommen, abgesaugt und dreimal mit 100 ml Ethanol gewaschen. Der Feststoff wird dreimal aus Chlorbenzol umkristallisiert und nach Trocknen im Vakuum zweimal sublimiert (p = 1 x 10⁻⁵ mbar, T = 320 °C). Ausbeute: 11.1 g (18 mmol), 37.1 %, Reinheit ca. 99.9 % (HPLC).

Analog zu Beispiel 4 werden aus den entsprechenden Boronsäuren folgende erfindungsgemäße Verbindungen erhalten (Beispiele 5-7):

| Bsp | Boronsäure | Fluoren | Produkt | Ausbeute |
|---|---|---|---|---|
| 5 | 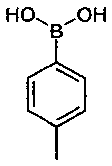 | 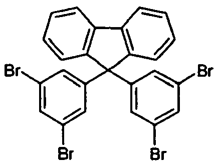 | 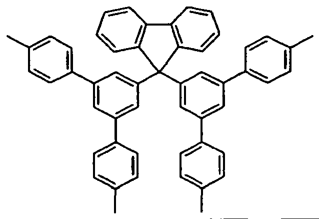 | 48.2% |
| 6 | 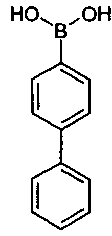 | 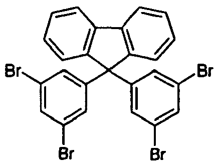 | 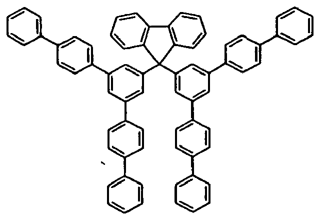 | 34.6% |
| 7 | 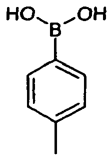 | 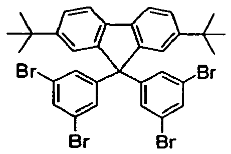 | 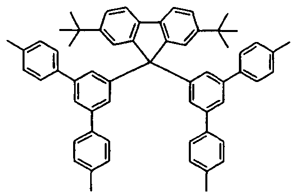 | 42.9 % |

### Beispiel 8: Synthese von 9,9-Bis-(3,5-diphenylaminophenyl)fluoren

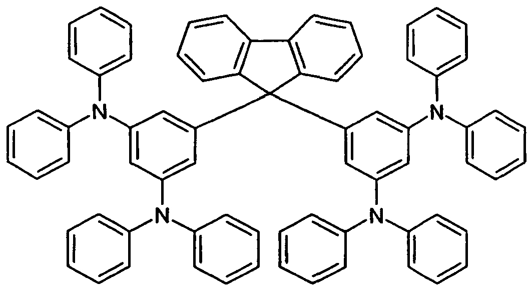

Eine gut gerührte Suspension von 25.4 g (40 mmol) 9,9-Bis-(3,5-dibromphenyl)fluoren, 33.8 g (200 mmol) Diphenylamin und 21.1 g (220 mmol) in 500 ml Toluol wird mit 101 mg (0.50 mmol) Tri-tert-butylphosphin und 56 mg (0.25 mmol) Palladium(II)acetat versetzt und fünf Stunden unter Rückfluss erhitzt. Nach Erkalten wird die Lösung über Silicagel filtriert und anschließend im Vakuum bis zur Trockne eingeengt. Der Rückstand wird eine Stunde bei 60 °C in 600 ml eines 1:1 Gemisches von Ethanol und Wasser gerührt, abgesaugt , fünfmal mit 250 ml Ethanol gewaschen und im Vakuum getrocknet. Der beigefarbene Feststoff wird fünfmal aus Dimethylformamid und dreimal aus Chlorbenzol umkristallisiert, im Vakuum getrocknet und anschließend zweimal sublimiert (p = 1 x 10⁻⁵ mbar, T = 350 °C). Ausbeute: 10.2 g (10 mmol), 25.0 %, Reinheit 99.9 % (HPLC), T_{g} = 99.8 °C.

Analog zu Beispiel 8 werden aus den entsprechenden Aminen und den entsprechenden Fluorenen folgende erfindungsgemäße Verbindungen erhalten (Beispiele 9-13):

| Bsp. | Amin | Fluoren | Produkt | Ausbeute |
|---|---|---|---|---|
| 9 | 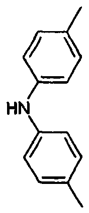 | 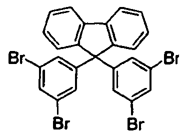 | 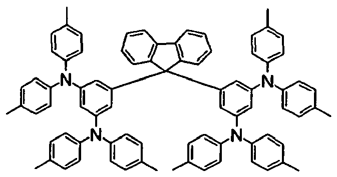 | 59.8 % |
| 10 | 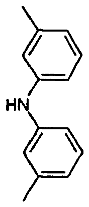 | 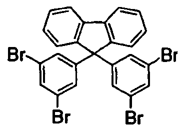 | 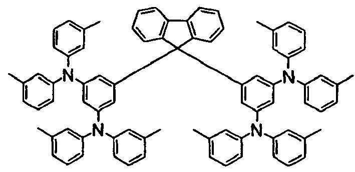 | 48.3 % |
| 11 | 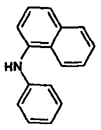 | 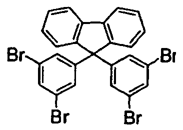 | 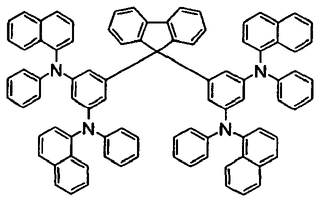 | 53.0% |
| 12 | 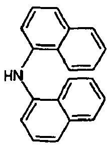 | 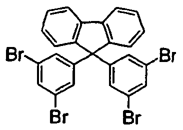 | 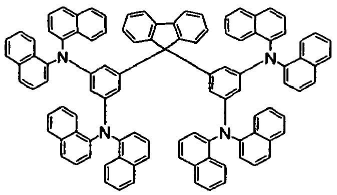 | 38.1 % |
| 13 | 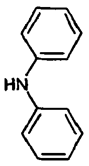 | 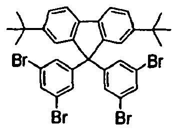 | 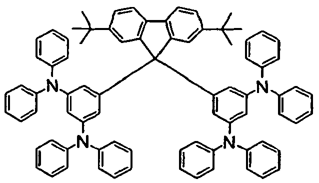 | 62.7 % |

### Beispiel 14: Synthese von 9,9-Bis-(3,5-dicarbazol-N-yl)fluoren

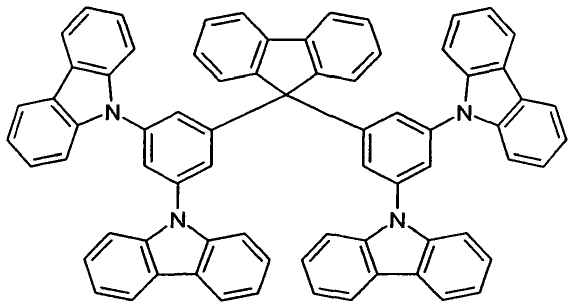

Eine Suspension von 50.7 g (80 mmol) 9,9-Bis-(3,5-dibromphenyl)fluoren, 78.6 g (470 mmol) Carbazol und 201.7 g (950 mmol) Trikaliumphosphat wird mit 500 g Glasperlen in 1000 ml *p*-Xylol gut gerührt. Die Suspension wird mit 1.62 g (8.0 mmol) Tri-tert-butylphospin und 894 mg (4.0 mmol) Palladium(II)acetat versetzt und fünf Tage unter Rückfluss erhitzt. Nach Erkalten wird mit 1000 ml Wasser versetzt, zwölf Stunden gerührt und dann filtriert. Die organische Phase wird abgetrennt, dreimal mit 200 ml Wasser gewaschen und anschließend im Vakuum zu einem zähen Öl eingeengt. Unter Rühren mit 300 ml Ethanol bildet sich ein kristalliner Feststoff, der abgesaugt und dreimal mit 250 ml Ethanol gewaschen wird. Eine Lösung des Feststoffs in 350 ml Dimethylformamid wird in 1500 ml siedendes Ethanol getropft. Nach Erkalten wird der Feststoff abgesaugt, dreimal aus Chlorbenzol umkristallisiert, im Vakuum getrocknet und dann zweimal im Vakuum sublimiert (p = 1 x 10⁻⁵ mbar, T = 370 °C). Ausbeute: 33_{.}2 g (34 mmol), 42.4 %, Reinheit-99.8 % (HPLC).

Analog zu Beispiel 14 werden aus den entsprechenden Carbazolderivaten folgende erfindungsgemäße Verbindungen erhalten (Beispiele 15-16):

| Bsp | Edukt | Produkt | Ausbeute |
|---|---|---|---|
| 15 | 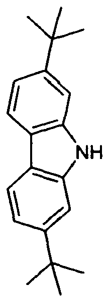 | 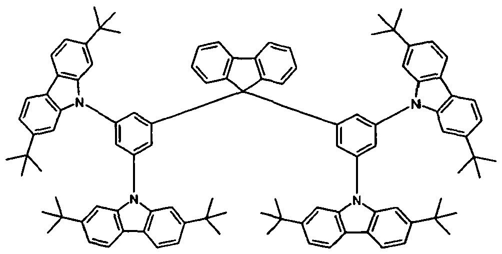 | 51.3 % |
| 16 | 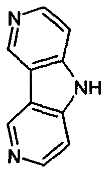 | 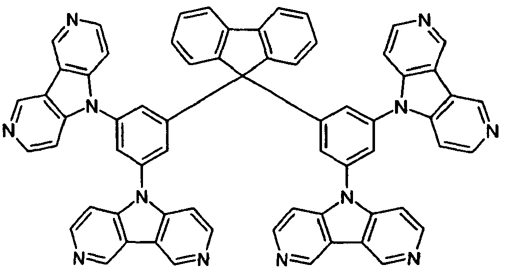 | 21.4 % |

### Beispiel 17: Synthese von 9,9-Bis-((3,5-bis-benzoyl)phenyl)fluoren

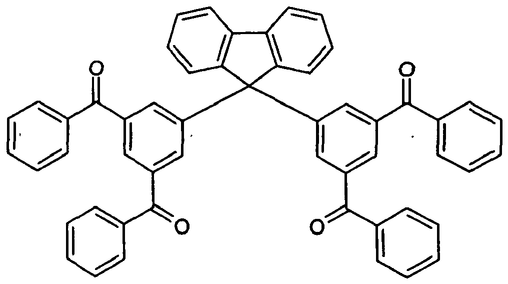

Eine auf -78 °C gekühlte Lösung von 25.4 g (40 mmol) 9,9-Bis-(3,5-dibromphenyl)fluoren in 1000 ml THF wird tropfenweise mit 67.5 ml n-Butyl-litium (2.5 M in Hexan) versetzt und anschließend 30 min. bei -78 °C gerührt. Dann gibt man ein Gemisch aus 18.6 g (180 mmol) Benzonitril und 50 ml THF zügig zu, rührt 1 h bei -78 °C nach, lässt dann auf Raumtemperatur erwärmen, gibt 100 ml 5 N Salzsäure zu und kocht 5 h unter Rückfluss. Nach Erkalten rotiert man das THF im Vakuum ab, nimmt den Rückstand in 500 ml Dichlormethan auf, wäscht mit Wasser und ges. Natriumhydrogencarbonat-Lösung neutral, trocknet über Magnesiumsulfat und filtriert dann über eine kurze Säule mit Kieselgel ab. Man rotiert das Lösemittel im Vakuum bis auf ca. 50 ml ab, versetzt mit 300 ml Methanol, saugt vom ausgefallen Feststoff ab, wäscht diesen einmal mit 100 ml Methanol. Nach Trocknen wird der beigefarbene Feststoff fünfmal aus Dimethylformamid umkristallisiert, im Vakuum getrocknet und anschließend zweimal sublimiert (p = 1 x 10⁻⁵ mbar, T = 360 °C). Ausbeute: 14.3 g (19 mmol), 48,6 %, Reinheit 99.9 % (HPLC).

Analog zu Beispiel 17 werden aus den entsprechenden Nitrilen folgende erfindungsgemäße Verbindungen erhalten (Beispiele 18 und 19):

| Bsp. | Amin | Produkt | Ausbeute |
|---|---|---|---|
| 18 | 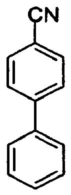 | 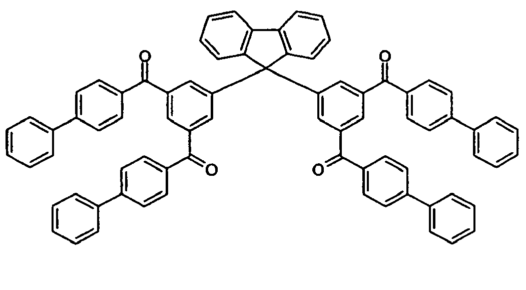 | 51.8 % |
| 19 | 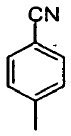 | 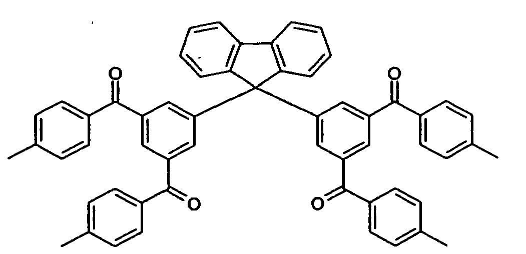 | 37.0% |

### Beispiel 20: Synthese von 9,9-Bis-((3,5-bis-diphenylphospinyl)-phenyl)fluoren

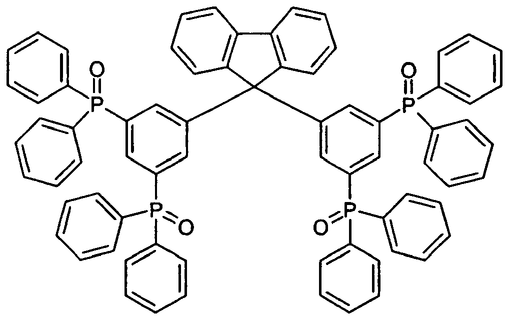

Eine auf -78 °C gekühlte Lösung von 25.4 g (40 mmol) 9,9-Bis-(3,5-dibromphenyl)fluoren in 1000 ml THF wird tropfenweise mit 67.5 ml n-Butyllitium (2.5 M in Hexan) versetzt und anschließend 30 min. bei -78 °C gerührt. Dann gibt man ein Gemisch aus 39.7 g (180 mmol) Chlordiphenylphosphin und 50 ml THF zügig zu, rührt 1 h bei -78 °C nach, lässt dann auf Raumtemperatur erwärmen, rotiert das THF im Vakuum vollständig ab, nimmt den Rückstand in 500 ml Essigsäureethylester auf, versetzt unter intensivem Rühren tropfenweise mit 150 ml 10 %igem Wasserstoffperoxid, rührt 16 h nach, trennt die wässrige Phase ab, rotiert das Lösemittel im Vakuum bis auf ca. 50 ml ab, versetzt mit 300 ml Methanol, saugt vom ausgefallen Feststoff ab, wäscht diesen einmal mit 100 ml Methanol. Nach Trocknen wird der beigefarbene Feststoff fünfmal aus Chlorbenzol umkristallisiert, im Vakuum getrocknet und anschließend zweimal sublimiert (p = 1 x 10⁻⁵ mbar, T = 390 °C). Ausbeute: 12.0 g (11 mmol), 26.8 %, Reinheit 99.9 % (HPLC).

Analog zu Beispiel 20 wird aus dem entsprechenden Chlorophosphin folgende erfindungsgemäße Verbindung erhalten (Beispiel 21):

| Bsp. | Amin | Produkt | Ausbeute |
|---|---|---|---|
| 21 | 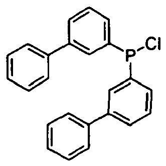 | 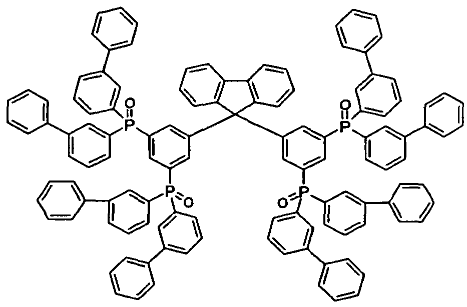 | 27.6 % |

### Beispiel 22: Synthese von 9,9-Bis-((3,5-bis-N-phenylbenzimidazol-2-yl)phenyl)fluoren

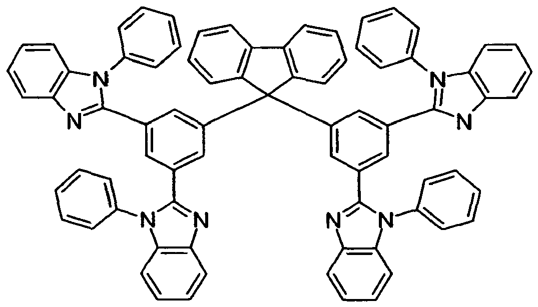

**a) 9,9-Bis-(3,5-dicyano-phenyl)fluoren**
   Ein Suspension von 63.4 g (100 mmol) 9,9-Bis-(3,5-dibrom-phenyl)fluoren, 58.7 g (500 mmol) Zinkcyanid, 3.3 g (50 mmol) Zink und 11.6 g (10 mmol) Tetrakis(triphenylphosphino)palladium(0) in 1000 ml Dimethylacetamid wird 60 h bei 140 °C gerührt. Nach Erkalten gibt man 1000 ml konz. Ammoniaklösung zu, rührt 1 h nach, saugt ab, und wäscht den Feststoff mit 500 ml Wasser und dreimal mit 100 ml Ethanol und trocknet im Vakuum. Ausbeute: 39.8 g (95 mmol), 95.1 %, Reinheit 98 % n. ¹H-NMR.
**b) 9,9-Bis-(3,5-dicarboxy-phenyl)fluoren**
   Eine Suspension von 39.8 g (95 mmol) 9,9-Bis-(3,5-dicyano-phenyl)fluoren wird in einer Lösung von 40 g Natriumhydroxid in einem Gemisch aus 300 ml Ethanol und 100 ml Wasser so lange (ca. 10 h) unter Rückfluss erhitzt, bis eine klare Lösung entsteht. Nach Erkalten wird durch Zugabe von 5 N Salzsäure auf pH 1 eingestellt. Der ausgefallene Feststoff wird abgesaugt, mit Wasser gewaschen, bis die Mutterlauge mit pH = 4-5 abläuft, trocken gesaugt und dann mit Toluol azeoptop getrocknet. Ausbeute: 44.5 g (90 mmol), 94.8 %, Reinheit 98 % n. ¹H-NMR.
**c) 9,9-Bis-((3,5-bis-N-phenylbenzimidazol-2-yl)phenyl)fluoren**
   44.5 g (90 mmol) 9,9-Bis-(3,5-dicarboxy-phenyl)fluoren wird in 150 ml Thionylchlorid suspendiert, die Suspension wird mit einem Tropfen DMF versetzt und anschließend so lange auf 60 °C erwärmt, bis die Gasentwicklung beendet ist. Dann wird das überschüssige Thionylchlorid im Vakuum entfernt, der Rückstand in 500 ml Dichlomethan gelöst und anschließend tropfenweise mit einer Lösung von 66.3 g (360 mmol) N-Phenyl-o-phenylendiamin in einem Gemisch aus 200 ml Dichlormethan und 150 ml Triethylamin versetzt. Nach Abklingen der exothermen Reaktion wird weitere 16 h bei Raumtemperatur nachgerührt. Die Reaktionsmischung wird mit 500 ml 1 N NaOH und dann zweimal mit 500 ml Wasser gewaschen, über Magnesiumsulfat getrocknet und dann an Aluminiumoxid (basisch, Aktivitätsstufe 1) mit Dichlormethan chromatographiert. Abschließend wird dreimal aus Chlorbenzol umkristallisiert, im Vakuum getrocknet und dann zweimal sublimiert (p = 1 x 10⁻⁵ mbar, T = 410 °C). Ausbeute: 33.8 g (31 mmol), 31.0 %, Reinheit 99.9 % (HPLC).

### Beispiel 23: Synthese von 9,9-Bis-(4,6-diphenyl-triazin-2-yl)fluoren

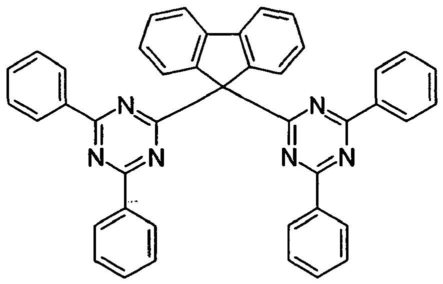

Eine Lösung von 16.6 g (100 mmol) Fluoren in 500 ml THF wird mit 7.2 g (300 mmol) Natriumhydrid versetzt. Nach Zugabe von 0.5 ml Diisopropylamin wird die Mischung 1 h bei Raumtemperatur gerührt, anschließend tropfenweise mit einer Lösung von 58.9 g (220 mmol) 2-Chlor-4,6-dipheny-1,3,5-triazin versetzt und dann 16 h bei 50 °C gerührt. Nach Erkalten wird durch Zugabe von 5 ml Wasser gequencht, das THF wird im Vakuum abgezogen, der Rückstand wird in Dichlormethan gelöst, die organische Phase wird mit Wasser gewaschen und dann über Magnesiumsulfat getrocknet. Nach Entfernen des Dichlormethans wird der Rückstand viermal aus DMF umkristallisiert, im Vakuum getrocknet und dann zweimal sublimiert (p = 1 x 10⁻⁵ mbar, T = 340 °C). Ausbeute: 17.0 g (27 mmol), 27.0 %, Reinheit 99.9 % (HPLC).

### Beispiel 24: Synthese von 10-Phenyl-12,12-bis-[1,1';3,1 "]terphenyl-5'-yl-10,12- dihydro-10-aza-indeno[2,1-b]fluoren

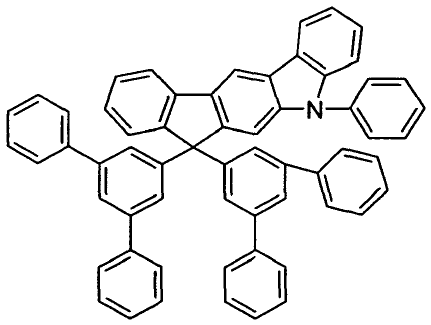

**a) 3-(2-Bromphenyl)-N-phenylcarbazol**
   Ein Gemisch aus 28.7 g (100 mmol) N-Phenylcarbazol-3-boronsäure, 36.1 ml (300 mmol) 1,2-Dibrombenzol in 150 ml Dioxan, 100 ml 2-Ethoxyethanol und 250 ml 2 N Natriumcarbonat-Lösung wird mit 1.1 g (1 mmol) Tetrakis(triphenylhposphino)palladium(0) versetzt und 16 h unter Rückfluss erhitzt. Nach Erkalten wird die organische Phase abgetrennt, mit 500 ml Toluol versetzt, dreimal mit 500 ml Wasser gewaschen, über Magnesiumsulfat getrocknet, über Kieselgel filtriert und dann vom Toluol und dem überschüssigen 1,2-Dibrombenzol im Vakuum befreit. Der Rückstand wird dreimal mit Ethanol heiß ausgerührt. Ausbeute 26.7 g (67 mmol), 67.1 %, Reinheit 97 % n.¹H-NMR.
**b) 12,12-Bis-(3,5-dibrom-phenyl)-10-phenyl-10,12-dihydro-10-aza-indeno[2,1-b]fluoren**
   Eine auf -78 °C gekühlte Lösung von 23.9 g (60 mmol) 3-(2-Bromphenyl)-N-phenylcarbazol in 500 ml THF wird tropfenweise mit 24.0 ml (60 mmol) n-Butyllithium (2.5 N in Hexan) versetzt, anschließend 30 min. bei -78 °C nachgerührt und dann tropfenweise mit einer Lösung von 38.0 g (60 mmol) 9,9-Bis-(3,5-dibromphenyl)fluoren in 100 ml THF versetzt. Nach vollendeter Zugabe lässt man auf Raumtemperatur erwärmen, entfernt das THF im Vakuum, nimmt den Rückstand in 500 ml Eisessig auf, versetzt mit 5 ml Bromwasserstoff und erhitzt die Suspension eine halbe Stunde unter Rückfluss. Nach Erkalten wird der Feststoff abgesaugt, dreimal mit 300 ml Ethanol gewaschen und einmal aus Toluol/Ethanol umkristallisiert. Ausbeute: 36.2 g (45 mmol), 75.3 %, Reinheit ca. 97 % (HPLC).
**c) 10-Phenyl-12,12-bis-[1,1';3,1"]terphenyl-5'-yl-10,12- dihydro-10-aza-indeno[2,1-b]fluoren**
   Darstellung analog zu Beispiel 4. Anstelle von 30.4 g (48 mmol) 9,9-Bis-(3,5-dibromphenyl)fluoren werden 36.0 g (45 mmol) 12,12-Bis-(3,5-dibromphenyl)-10-phenyl-10,12-dihydro-10-aza-indeno[2,1-b]fluoren verwendet. Der Feststoff wird dreimal aus NMP umkristallisiert und nach Trocknen im Vakuum zweimal sublimiert (p = 1 x 10⁻⁵ mbar, T = 400 °C). Ausbeute: 15.0 g (19 mmol), 42.3 %, Reinheit ca. 99.9 % (HPLC).

### Beispiel 25: Herstellung und Charakterisierung von organischen Elektrolumineszenzvorrichtungen, enthaltend die erfindungsgemäßen Verbindungen

Erfindungsgemäße Elektrolumineszenzvorrichtungen können, wie beispielsweise in WO 05/003253 beschrieben, dargestellt werden. Hier werden die Ergebnisse verschiedener OLEDs gegenübergestellt. Der grundlegende Aufbau, die verwendeten Materialien, der Dotierungsgrad und ihre Schichtdicken sind zur besseren Vergleichbarkeit identisch. Das erste Devicebeispiel beschreibt einen Vergleichsstandard nach dem Stand der Technik, bei dem die Emissionsschicht aus dem Wirtsmaterial Bis(9,9'-spirobifluoren-2-yl)keton und dem Gastmaterial (Dotand) Ir(ppy)₃ besteht. Des Weiteren werden OLEDs verschiedener Aufbauten beschrieben, jeweils ist das Gastmaterial (Dotand) Ir(ppy)₃. Analog dem o. g. allgemeinen Verfahren werden OLEDs mit folgendem Aufbau erzeugt:
- Lochinjektionsschicht (HIL): 20 nm 2,2',7,7'-Tetrakis(di-para-tolyl-amino)spiro-9,9'-bifluoren
- Lochtransportschicht (HTL): 20 nm NPB (N-Naphthyl-N-phenyl-4,4'-diaminobiphenyl) oder Amin-1 als Vergleich oder Verbindung 1.
- Emissionsschicht (EML): 40 nm Host: Spiro-Keton (SK) (Bis(9,9'-spirobifluoren-2-yl)keton) als Vergleich oder Verbindung 2. Dotand: Ir(ppy)₃ (10 % Dotierung, aufgedampft; synthetisiert nach WO 04/085449).
- Elektronenleiter (ETL): 20 nm AlQ₃ (Tris(chinolinato)aluminium(III)) als Vergleich oder Verbindung 3.
- Kathode: 1 nm LiF, darauf 150 nm Al.

Die Strukturen von Ir(ppy)₃, Spiro-Keton (SK) und Amin-1 sind der Übersichtlichkeit halber im Folgenden abgebildet. Dabei ist Amin-1 eine Vergleichsverbindung gemäß dem nächstliegenden Stand der Technik (JP 2005/085599):

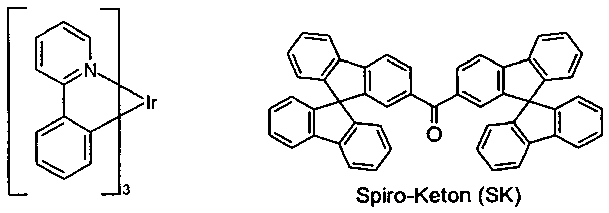

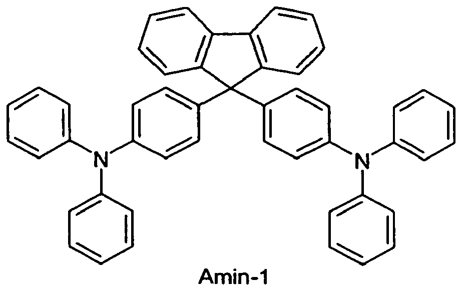

Die erfindungsgemäßen Verbindungen 1 bis 7 sind im Folgenden abgebildet:

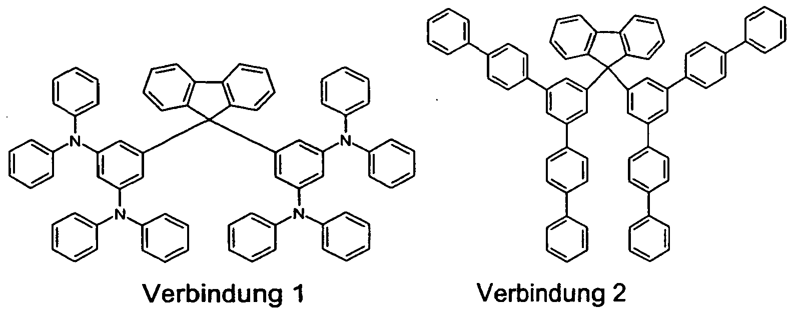

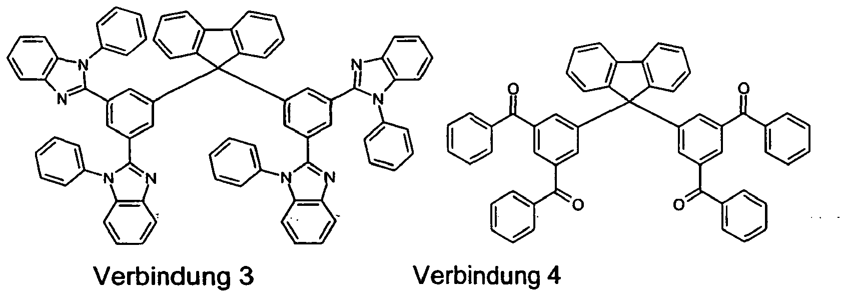

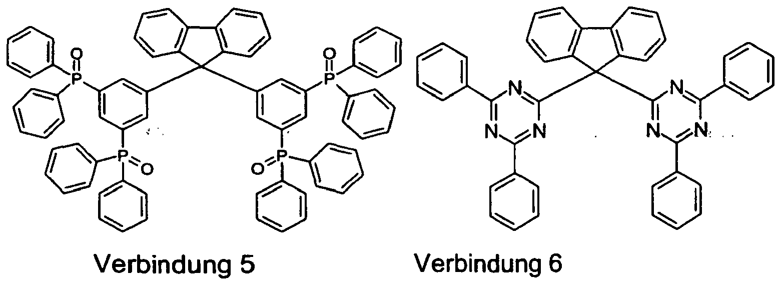

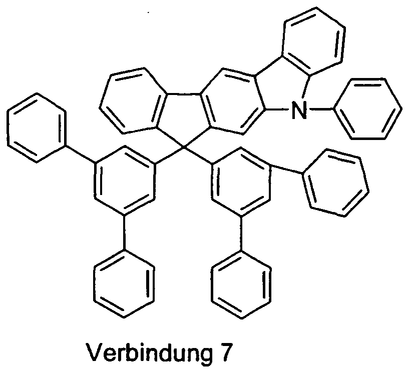

Diese noch nicht optimierten OLEDs werden standardmäßig charakterisiert; hierfür werden die Elektrolumineszenzspektren, die Effizienz (gemessen in cd/A) in Abhängigkeit von der Helligkeit, berechnet aus Strom-Spannungs-Helligkeit-Kennlinien (IUL-Kennlinien), und die Lebensdauer bestimmt.

Mit OLEDs hergestellt nach dem oben beschriebenen Aufbau und Materialien als Vergleichsexperiment mit NPB als Lochtransportmaterial erhält man unter den oben beschriebenen Bedingungen typischerweise eine maximale Effizienz von etwa 30 cd/A bei Farbkoordinaten von CIE: x = 0.38, y = 0.57. Für die Referenzleuchtdichte von 1000 cd/m² werden Spannungen von 4.4 V benötigt. Die Lebensdauer beträgt etwa 7700 h bei einer Anfangsleuchtdichte von 1000 cd/m² (s. Tabelle 1, Beispiel 26). Mit Amin-1 als Lochtransportmaterial in einem ansonsten gleichen Deviceaufbau (s. Tabelle 1, Beispiel 27) erhält man zwar eine bessere maximale Effizienz im Bereich von 41 cd/A, jedoch werden für die Referenzleuchtdichte von 1000 cd/m² Spannungen von 5.3 V benötigt und die Lebensdauer beträgt nur etwa 5600 h.

Im Gegensatz dazu zeigen erfindungsgemäße OLEDs hergestellt mit dem erfindungsgemäßen Elektronenblockiermaterial (Verbindung 1) eine deutlich erhöhte maximale Effizienz von 47 cd/A bei Farbkoordinaten von CIE: x = 0.38, y = 0.58, wobei die benötigte Spannung für die Referenzleuchtdichte von 1000 cd/m² bei 4.4 V liegt (s. Tabelle 1, Beispiel 28). Die Lebensdauer bei einer Anfangsleuchtdichte von 1000 cd/m² ist mit 7400 h vergleichbar mit dem Vergleichsbeispiel 26 (s. Tabelle 1, Beispiel 28).

Im Gegensatz zum Vergleichsexperiment zeigen erfindungsgemäße OLEDs hergestellt mit dem Wirtsmaterial (Verbindung 2) anstelle des Spiro-Ketons bei ansonsten gleichem Aufbau eine maximale Effizienz von 35 cd/A bei verbesserten Farbkoordinaten von CIE: x = 0.31, y = 0.62, wobei die benötigte Spannung für die Referenzleuchtdichte von 1000 cd/m² bei 5.2 V liegt (s. Tabelle 1, Beispiel 29). Die Lebensdauer bei einer Anfangsleuchtdichte von 1000 cd/m² ist mit 6900 h vergleichbar wie bei Verwendung des Spiro-Ketons (s. Tabelle 1, Beispiel 29). Setzt man Verbindung 1 als Elektronenblockiermaterial und Verbindung 3 als Elektronentransportmaterial anstelle von Alq ein, so erhält man maximale Effizienzen von 54 cd/A bei Farbkoordinaten von CIE: x = 0.37, y = 0.59, wobei die benötigte Spannung für die Referenzleuchtdichte von 1000 cd/m² bei 4.1 V liegt (s. Tabelle 1, Beispiel 30). Die Lebensdauer bei einer Anfangsleuchtdichte von 1000 cd/m² ist mit 7200 h vergleichbar und die Spannung mit 4.1 V niedriger als mit dem Referenzmaterial Alq (s. Tabelle 1, Beispiel 22).

Setzt man die erfindungsgemäßen Verbindungen 4, 5 bzw. 7 als Matrixmaterial ein, erhält man sehr gute Effizienzen in Kombination mit guten Lebensdauern. Verbindung 6 eignet sich unter anderem als Elektronenleiter, der bei niedrigen Spannungen gute Effizienzen und Lebensdauern liefert.

**Tabelle 1: Device-Ergebnisse mit erfindungsgemäßen Verbindungen mit Ir(ppy)₃ als Dotand**

| Bsp. | HTUEBL 20 nm | EML 40 nm | ETL 20 nm | Max. Eff. [cd/A] | Spannung [V] bei 1000 cd/m² | CIE (x, y) | Lebensdauer [h], Anfangs-helligkeit 1000 cd/m² |
|---|---|---|---|---|---|---|---|
| 26 Vergl. | NPB | SK: Ir(ppy)₃ | Alq | 30 | 4.4 | 0.38/ 0.57 | 7700 |
| 27 Vergl. | Amin-1 | SK: Ir(ppy)₃ | Alq | 41 | 5.3 | 0.38/ 0.58 | 5600 |
| 28 | Verb. 1 | SK: Ir(ppy)₃ | Alq | 47 | 4.4 | 0.38/ 0.58 | 7400 |
| 29 | NPB | Verb. 2 : Ir(ppy)₃ | Alq | 35 | 5.2 | 0.31/ 0.62 | 6900 |
| 30 | Verb. 1 | SK: Ir(ppy)₃ | Verb. 3 | 54 | 4.1 | 0.37/ 0.59 | 7200 |
| 31 | NPB | Verb. 4 : Ir(ppy)₃ | Alq | 41 | 5.2 | 0.36/ 0.59 | 7000 |
| 32 | Verb. 1 | Verb. 4 : Ir(PPY)₃ | Alq | 57 | 4.3 | 0.36/ 0.59 | 7800 |
| 33 | Verb. 1 | Verb. 5 : 'r(PPY)₃ | Alq | 61 | 4.5 | 0.36/ 0.59 | 7200 |
| 34 | Verb. 1 | SK: Ir(ppy)₃ | Verb. 6 | 52 | 4.0 | 0.37/ 0.60 | 7300 |
| 35 | Verb. 1 | Verb. 7 Ir(ppy)₃ | Alq | 53 | 3.9 | 0.38/ 0.59 | 8100 |

## Patentansprüche

1. Verbindungen gemäß Formel (1), 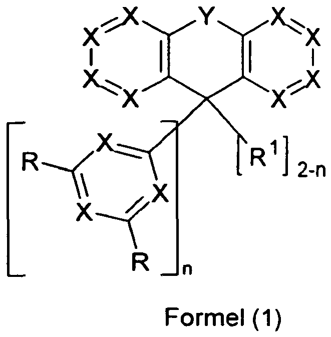 wobei für die verwendeten Symbole und Indizes gilt:
X ist bei jedem Auftreten gleich oder verschieden CR¹ oder N, wobei in jedem Cyclus maximal 3 Gruppen X für N stehen; oder zwei direkt benachbarte Gruppen X stehen für eine Einheit der folgenden Formel (7), 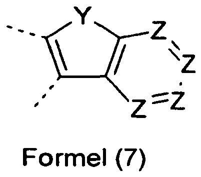 wobei die gestrichelten Bindungen die Verknüpfung der Einheit mit den benachbarten C- bzw. N-Atomen andeutet;
Y ist bei jedem Auftreten gleich oder verschieden eine Einfachbindung oder eine Gruppe ausgewählt aus BR¹, C(R¹)₂, C(=O), NR¹, P(=O)R¹ oder O;
Z ist bei bei jedem Auftreten gleich oder verschieden CR¹ oder N, wobei in jedem Cyclus maximal zwei Symbole Z für N stehen;
R ist bei jedem Auftreten gleich oder verschieden Br, I, Triflat, B(OR²)₂, B(R²)₂, B(N(R²)₂)₂, NAr₂, N(R²)₂, SiAr₃, Si(R²)₃, C(=O)Ar, C(=O)R², OAr, OR², SAr, SR², S(=O)Ar, S(=O)R², S(=O)₂Ar, S(=O)₂R², PAr₂, P(R²)₂, P(=O)Ar₂, P(=O)(R²)₂ oder ein aromatisches oder heteroaromatisches Ringsystem mit 5 bis 60 aromatischen Ringatomen, welches durch einen oder mehrere Reste R¹ substituiert sein kann;
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
R¹ ist bei jedem Auftreten gleich oder verschieden H, D, F, Cl, Br, I, CHO, N(R²)₂, N(Ar)₂. C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², eine geradkettige Alkyl-, Alkenyl-, Alkinyl-, Alkoxy- oder Thioalkoxygruppe mit 1 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches oder aromatisches Ringsystem bilden;
R² ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches, aliphatisches oder aromatisches Ringsystem bilden;
n ist 1 oder 2;
dabei sind die folgenden Verbindungen von der Erfindung ausgenommen:

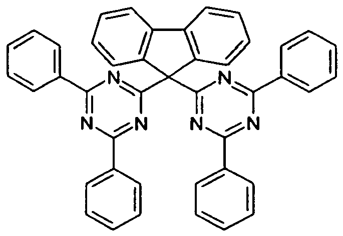

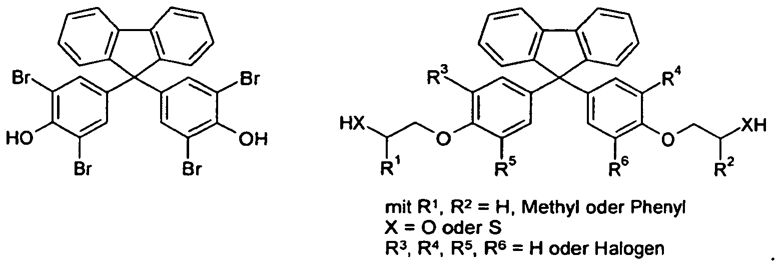

2. Verbindungen nach Anspruch 1 gemäß Formel (2), (3), (8), (9), (10) oder (11), 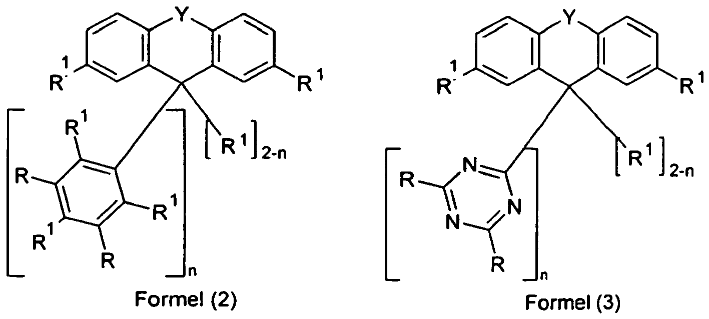 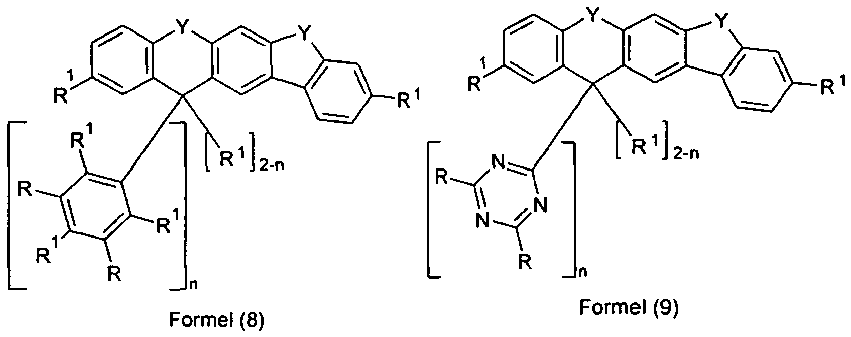 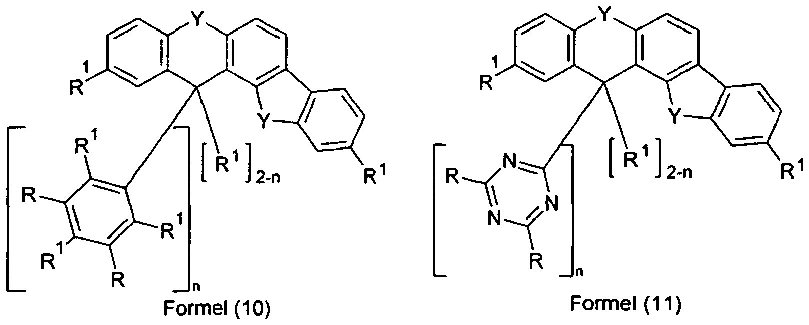 wobei die verwendeten Symbole und Indizes die in Anspruch 1 aufgeführten Bedeutungen haben.

3. Verbindungen nach Anspruch 1 oder 2, **dadurch gekennzeichnet, dass** das Symbol Y für eine Einfachbindung oder für eine Gruppe ausgewählt aus C(R¹)₂, O oder NR¹ steht.

4. Verbindungen nach einem oder mehreren der Ansprüche 1 bis 3 gemäß Formel (2a), (3a), (8a), (8b), (9a), (9b), (10a), (10b), (11a) oder (11b), 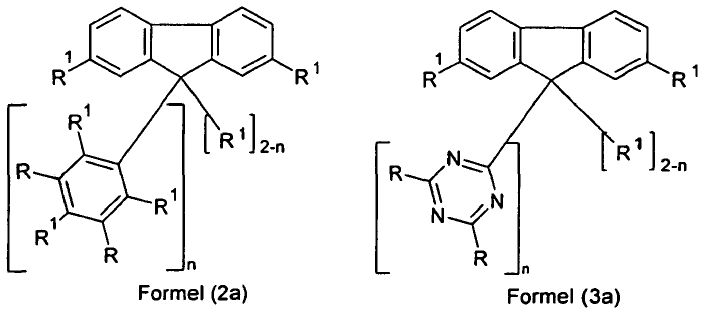 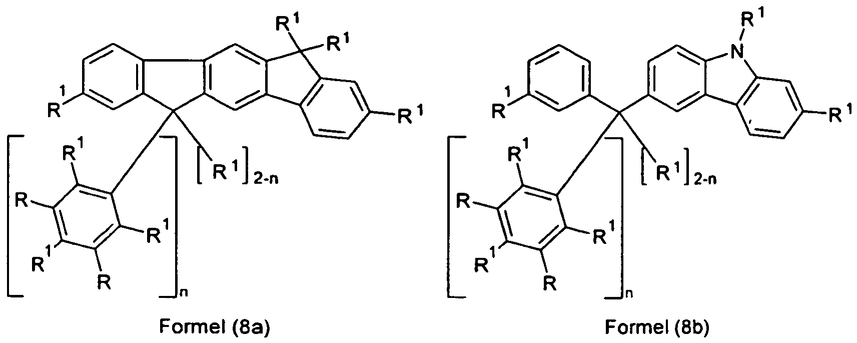 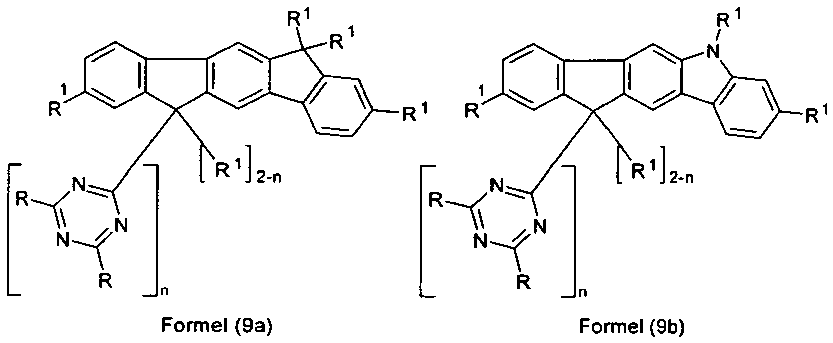 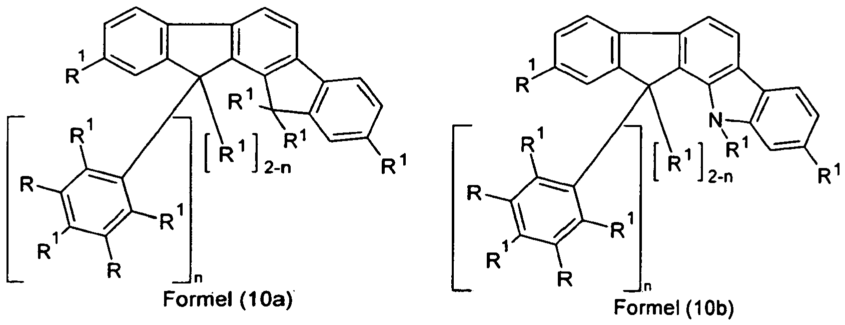 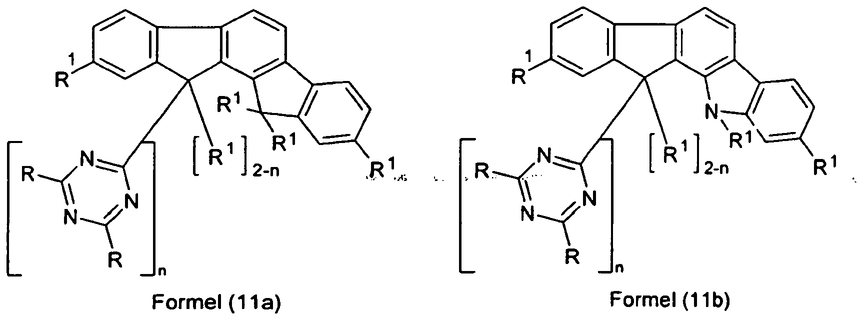 wobei die verwendeten Symbole die in Anspruch 1 aufgeführten Bedeutungen haben.

5. Verbindungen nach einem oder mehreren der Ansprüche 1 bis 3 gemäß Formel (4a) oder (4b), 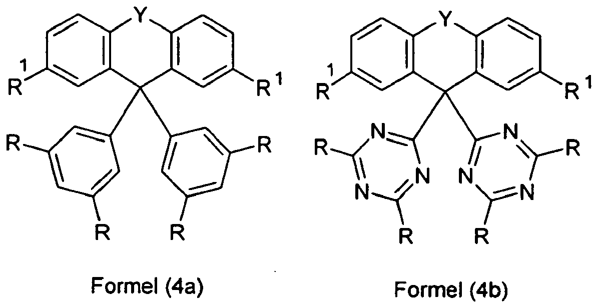 wobei die verwendeten Symbole die in Anspruch 1 aufgeführten Bedeutungen haben.

6. Verbindungen nach einem oder mehreren der Ansprüche 1 bis 5 gemäß Formel (4c), (4d), (8c), (8d), (9c), (9d), (10c), (10d), (11c) und (11d), 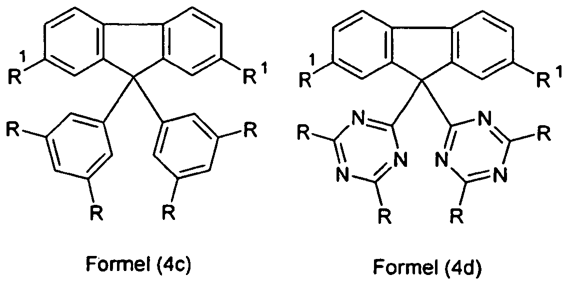 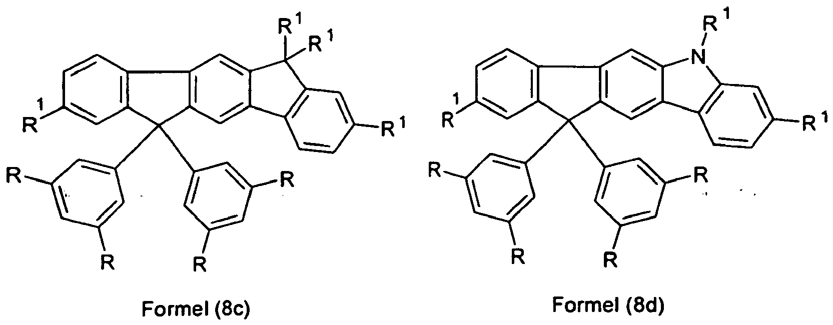 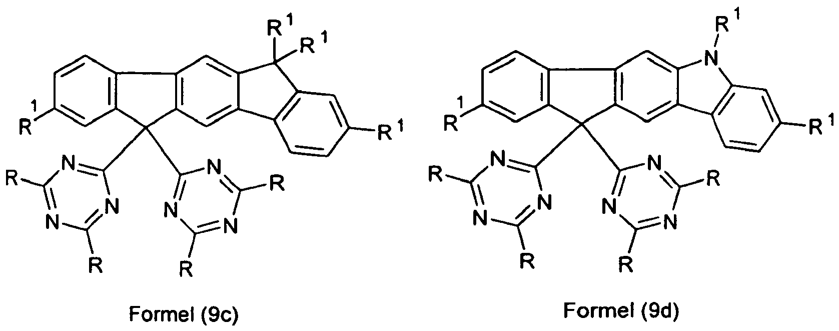 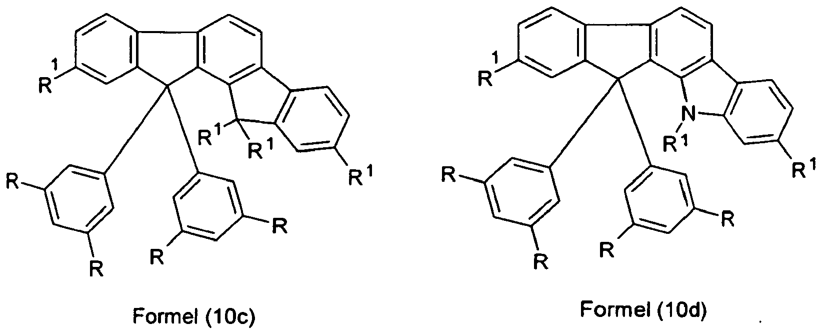 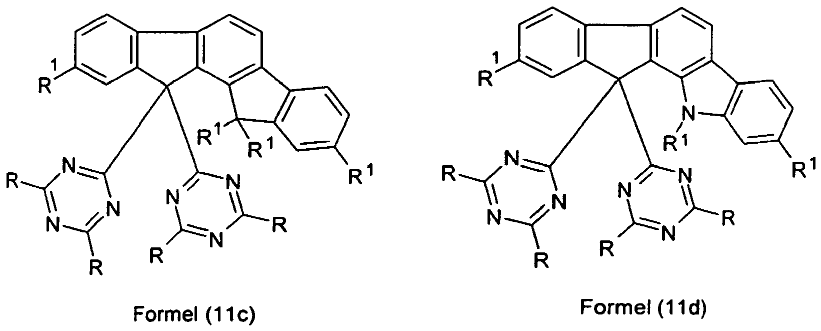 wobei die verwendeten Symbole und Indizes die in Anspruch 1 genannten Bedeutungen haben.

7. Verbindungen nach einem oder mehreren der Ansprüche 1 bis 6, **dadurch gekennzeichnet, dass** das Symbol R gleich oder verschieden bei jedem Auftreten für NAr₂, C(=O)Ar, P(=O)Ar₂ oder für ein aromatisches oder heteroaromatisches Ringsystem mit 5 bis 30 aromatischen Ringatomen, welches durch einen oder mehrere nicht-aromatische Reste R¹ substituiert sein kann, steht.

8. Verbindungen nach einem oder mehreren der Ansprüche 1 bis 7, **dadurch gekennzeichnet, dass** der Rest R bzw. R¹, wenn er für eine Gruppe N(Ar)₂ steht, ausgewählt ist aus den Gruppen der Formel (5) oder der Formel (6), 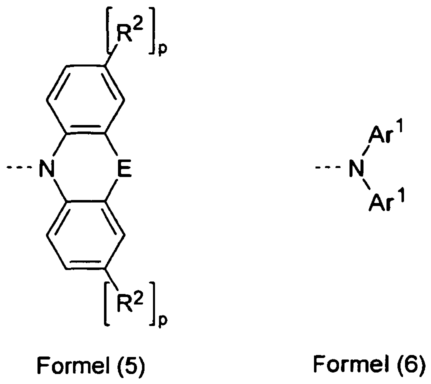 wobei R² die oben aufgeführte Bedeutung hat und weiterhin gilt:
E steht für eine Einfachbindung, O, S, N(R²) oder C(R²)₂;
Ar¹ ist gleich oder verschieden bei jedem Auftreten ein aromatisches oder heteroaromatisches Ringsystem mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, bevorzugt mit 18 bis 22 aromatischen Ringatomen, welche jeweils mit einem oder mehreren Resten R² substituiert sein kann;
p ist bei jedem Auftreten gleich oder verschieden 0 oder 1;
und/oder dass der Rest R, wenn er ein aromatisches bzw. hetoroaromatisches Ringsystem darstellt, ausgewählt ist aus Phenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl.

9. Verfahren zur Herstellung von Verbindungen nach einem oder mehreren der Ansprüche 1 bis 8, umfassend die Reaktion von Bis(3,5-dibrom)-benzophenon mit einem substituierten oder unsubstituierten 2-Lithiobiphenyl, 2-Lithiodiphenylether, 2-Lithiodiphenylthioether, 2-(2-Lithiophenyl)-2-phenyl-1,3-dioxolan, 2-Lithiophenyldiphenylamin oder einer entsprechenden Grignardverbindung zu den Triarylmethanolen, gefolgt von Cyclisierung unter sauren Bedingungen und gegebenenfalls gefolgt von weiterer Umsetzung der Bromgruppen.

10. Dimere, Trimere, Tetramere, Pentamere, Oligomere, Polymere oder Dendrimere enthaltend eine oder mehrere Verbindungen nach einem oder mehreren der Ansprüche 1 bis 8, wobei ein oder mehrere Reste R¹ oder R² Bindungen zwischen den Verbindungen gemäß Formel (1) im Dimer, Trimer, Tetramer bzw. Pentamer bzw. Bindungen der Verbindung gemäß Formel (1) zum Polymer, Oligomer oder Dendrimer darstellen oder wobei diese Bindung über Substituenten an den Gruppen R erfolgt.

11. Mischungen enthaltend mindestens eine Verbindung nach einem oder mehreren der Ansprüche 1 bis 8 oder 10 und mindestens eine weitere Verbindung.

12. Lösungen enthaltend mindestens eine Verbindung nach einem oder mehreren der Ansprüche 1 bis 8 oder 10 und mindestens ein organisches Lösemittel.

13. Verwendung von Verbindungen nach einem oder mehreren der Ansprüche 1 bis 8 oder 10 in elektronischen Vorrichtungen.

14. Elektronische Vorrichtung, enthaltend mindestens eine Verbindung nach einem oder mehreren der Ansprüche 1 bis 8 oder 10, insbesondere ausgewählt aus organischen Elektrolumineszenzvorrichtungen (OLED, PLED), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

15. Organische Elektrolumineszenzvorrichtung nach Anspruch 14, **dadurch gekennzeichnet, dass** die Verbindung nach einem oder mehreren der Ansprüche 1 bis 8 als Matrixmaterial für fluoreszierende oder phosphoreszierende Verbindungen eingesetzt wird, insbesondere wenn eine oder mehrere Gruppen R und/oder R¹ für C(=O)Ar, S(=O)Ar, S(=O)₂Ar oder P(=O)Ar₂ stehen, und/oder dass die Verbindung nach einem oder mehreren der Ansprüche 1 bis 8 als Lochtransportmaterial bzw. als Lochinjektionsmaterial bzw. als Elektronenblockiermaterial bzw. als Excitonenblockiermaterial eingesetzt wird, insbesondere wenn eine oder mehrere Gruppen R und/oder R¹ für N(Ar)₂ stehen, und/oder dass die Verbindung nach einem oder mehreren der Ansprüche 1 bis 8 als Elektronentransportmaterial bzw. als Lochblockiermaterial eingesetzt wird, insbesondere wenn die Gruppe Y für C=O, P(=O), SO oder SO₂ steht und/oder mindestens einer der Substituenten R und/oder R¹ für eine Heteroarylgruppe, welche einen elektronenarmen Heterocyclus darstellt, oder für C(=O)Ar, P(=O)Ar₂, S(=O)Ar oder S(O)₂Ar steht.

## Claims

1. Compounds of the formula (1), 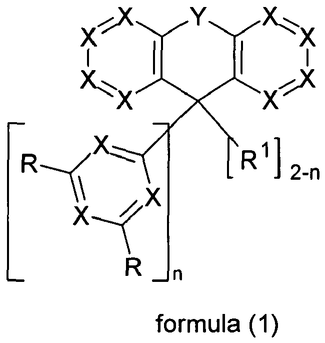 where the following applies to the symbols and indices used:
X is on each occurrence, identically or differently, CR¹ or N, where a maximum of 3 groups X in each ring stand for N; or two directly adjacent groups X stand for a unit of the following formula (7), 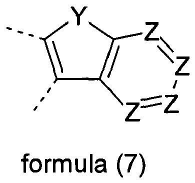 where the dashed bonds indicate the link from the unit to the adjacent C or N atoms;
Y is on each occurrence, identically or differently, a single bond or a group selected from BR¹, C(R¹)₂, C(=O), NR¹, P(=O)R¹ or O;
Z is on each occurrence, identically or differently, CR¹ or N, where a maximum of two symbols Z in each ring stand for N;
R is on each occurrence, identically or differently Br, I, triflate, B(OR²)₂, B(R²)₂, B(N(R²)₂)₂, NAr₂, N(R²)₂, SiAr₃, Si(R²)₃, C(=O)Ar, C(=O)R², OAr, OR², SAr, SR², S(=O)Ar, S(=O)R², S(=O)₂Ar, S(=O)₂R², PAr₂, P(R²)₂, P(=O)Ar₂, P(=O)(R²)₂ or an aromatic or heteroaromatic ring system having 5 to 60 aromatic ring atoms, which may be substituted by one or more radicals R¹;
Ar is on each occurrence, identically or differently, an aromatic or heteroaromatic ring system having 5 to 30 aromatic ring atoms, which may be substituted by one or more non-aromatic radicals R¹; two radicals Ar here which are bonded to the same nitrogen or phosphorus atom may also be linked to one another by a single bond or a bridge selected from B(R²), C(R²)₂, Si(R²)₂, C=O, C=NR², C=C(R²)₂, O, S, S=O, SO₂, N(R²), P(R²) and P(=O)R²;
R¹ is on each occurrence, identically or differently, H, D, F, Cl, Br, I, CHO, N(R²)₂, N(Ar)₂, C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², a straight-chain alkyl, alkenyl, alkynyl, alkoxy or thioalkoxy group having 1 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C≡C, Si(R²)₂, Ge(R²)₂, Sn(R²)_{2,} C=O, C=S, C=Se, C=NR², P(=O)(R²),SO, SO₂, NR², O, S or CONR² and where one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems; two or more adjacent substituents R¹ here may also form a mono- or polycyclic, aliphatic or aromatic ring system with one another;
R² is on each occurrence, identically or differently, H, D or an aliphatic, aromatic and/or heteroaromatic hydrocarbon radical having 1 to 20 C atoms, in which, in addition, H atoms may be replaced by F; two or more adjacent substituents R² here may also form a mono- or polycyclic, aliphatic or aromatic ring system with one another;
n is 1 or 2;
the following compounds are excluded from the invention:

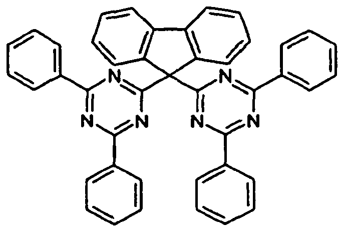

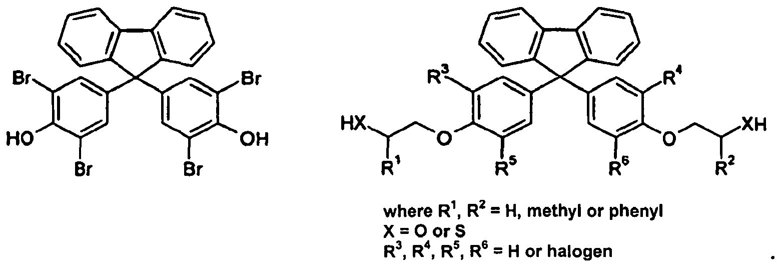

2. Compounds according to Claim 1 of the formula (2), (3), (8), (9), (10) or (11), 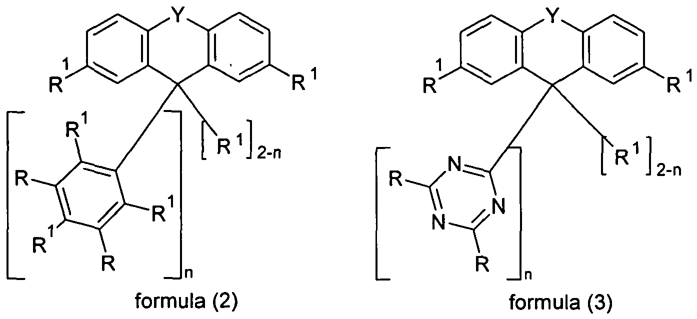 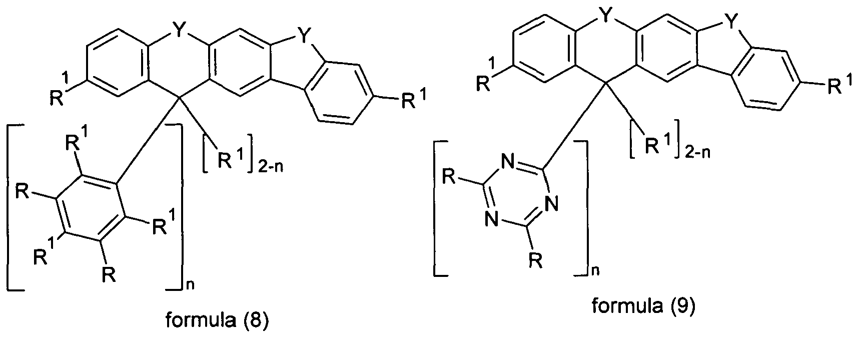 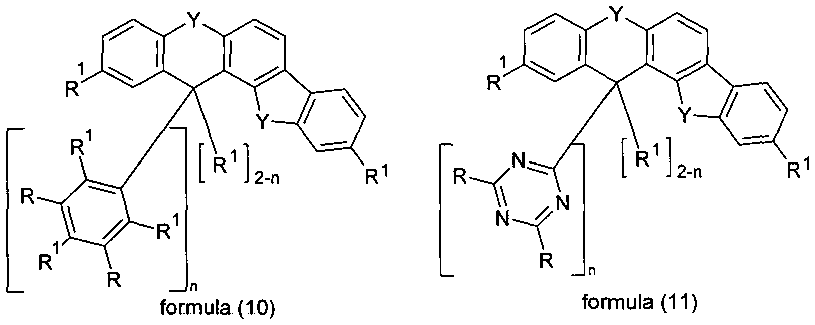 where the symbols and indices used have the meanings indicated in Claim 1.

3. Compounds according to Claim 1 or 2, **characterised in that** the symbol Y stands for a single bond or for a group selected from C(R¹)₂, O or NR¹.

4. Compounds according to one or more of Claims 1 to 3 of the formula (2a), (3a), (8a), (8b), (9a), (9b), (10a), (10b), (11a) or (11b), 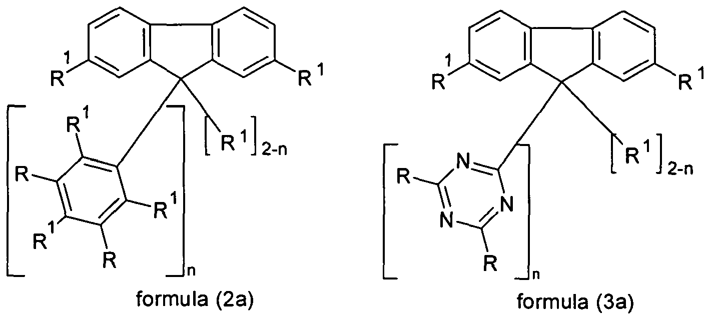 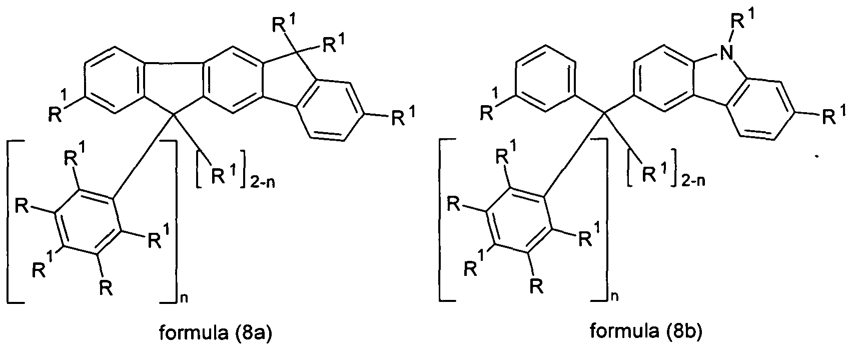 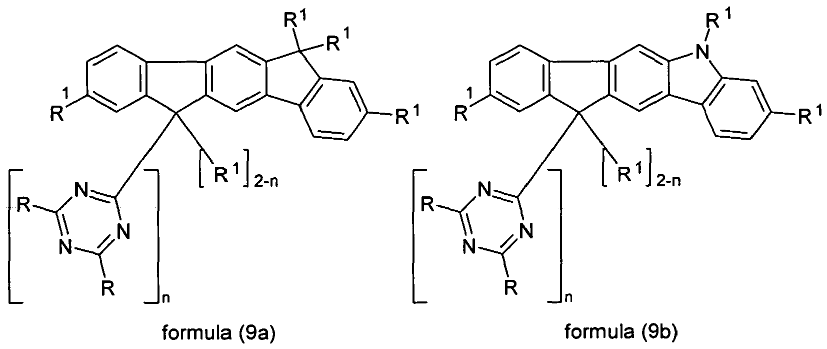 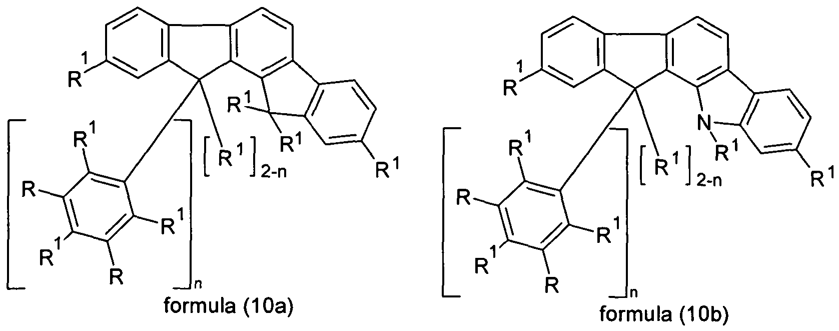 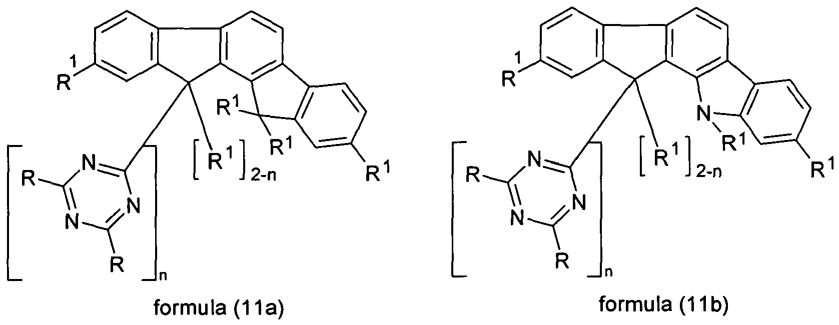 where the symbols used have the meanings indicated in Claim 1.

5. Compounds according to one or more of Claims 1 to 3 of the formula (4a) or (4b), 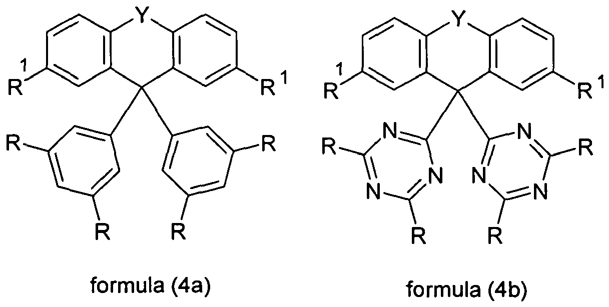 where the symbols used have the meanings indicated in Claim 1.

6. Compounds according to one or more of Claims 1 to 5 of the formula (4c), (4d), (8c), (8d), (9c), (9d), (10c), (10d), (11c) or (11 d), 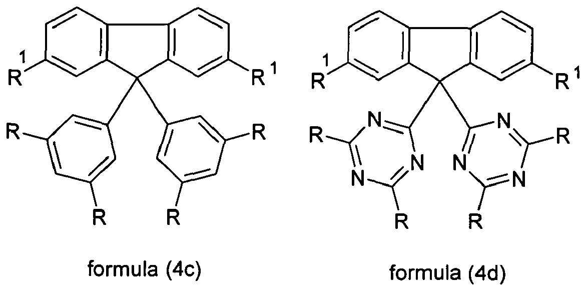 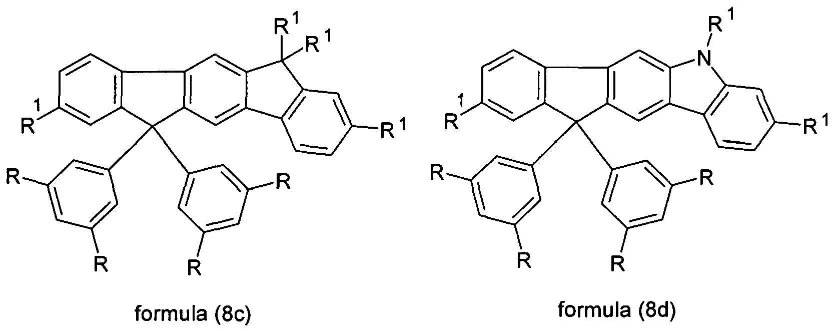 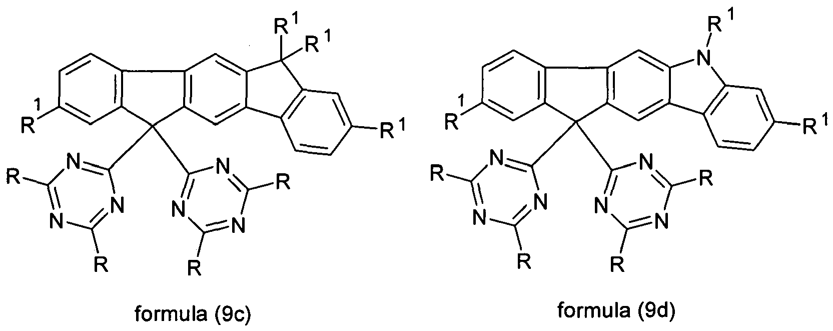 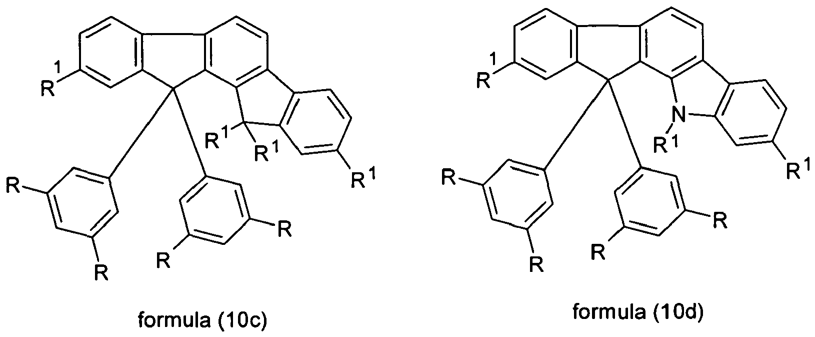 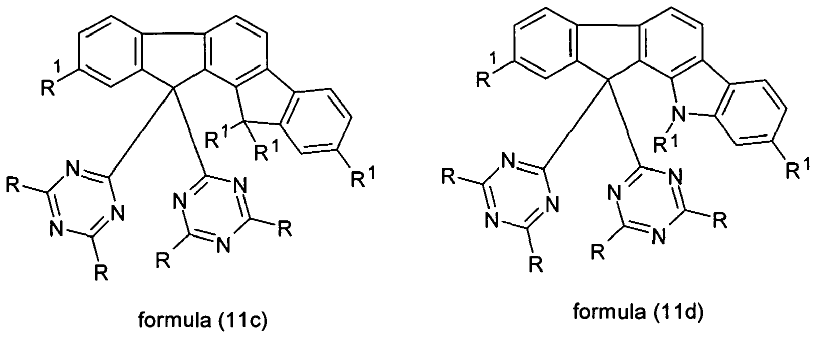 where the symbols and indices used have the meanings mentioned in Claim 1.

7. Compounds according to one or more of Claims 1 to 6, **characterised in that** the symbol R, identically or differently on each occurrence, stands for NAr₂, C(=O)Ar, P(=O)Ar₂ or for an aromatic or heteroaromatic ring system having 5 to 30 aromatic ring atoms, which may be substituted by one or more non-aromatic radicals R¹.

8. Compounds according to one or more of Claims 1 to 7, **characterised in that** the radical R or R¹, if it stands for a group N(Ar)₂, is selected from the groups of the formula (5) or of the formula (6), 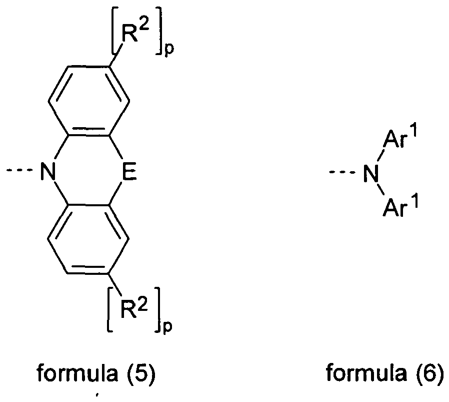 where R² has the meaning indicated above, and furthermore:
E stands for a single bond, O, S, N(R²) or C(R²)₂;
Ar¹ is, identically or differently on each occurrence, an aromatic or heteroaromatic ring system having 5 to 20 aromatic ring atoms or a triarylamine group having 15 to 30 aromatic ring atoms, each of which may be substituted by one or more radicals R², preferably an aryl or heteroaryl group having 6 to 14 aromatic ring atoms or a triarylamine group having 18 to 30 aromatic ring atoms, preferably having 18 to 22 aromatic ring atoms, each of which may be substituted by one or more radicals R²;
p is on each occurrence, identically or differently, 0 or 1;
and/or **in that** the radical R, if it represents an aromatic or heteroaromatic ring system, is selected from phenyl, 1-naphthyl, 2-naphthyl, anthracenyl, phenylanthracenyl, 1- or 2-naphthylanthracenyl, binaphthyl, pyrenyl, fluoranthenyl, 2-, 3-, 4-, 5-, 6- or 7-benzanthracenyl, N-benzimidazolyl, phenyl-N-benzimidazolyl, N-phenylbenzimidazolyl or phenyl-N-phenylbenzimidazolyl.

9. Process for the preparation of compounds according to one or more of Claims 1 to 8, comprising the reaction of bis(3,5-dibromo)benzophenone with a substituted or unsubstituted 2-lithiobiphenyl, 2-lithiodiphenyl ether, 2-lithiodiphenyl thioether, 2-(2-lithiophenyl)-2-phenyl-1,3-dioxolane, 2-lithiophenyldiphenylamine or a corresponding Grignard compound to give the triarylmethanols, followed by cyclisation under acidic conditions and optionally followed by further reaction of the bromine groups.

10. Dimers, trimers, tetramers, pentamers, oligomers, polymers or dendrimers containing one or more compounds according to one or more of Claims 1 to 8, where one or more radicals R¹ or R² represent bonds between the compounds of the formula (1) in the dimer, trimer, tetramer or pentamer or bonds from the compound of the formula (1) to the polymer, oligomer or dendrimer or where this bonding takes place via substituents on the groups R.

11. Mixtures comprising at least one compound according to one or more of Claims 1 to 8 or 10 and at least one further compound.

12. Solutions comprising at least one compound according to one or more of Claims 1 to 8 or 10 and at least one organic solvent.

13. Use of compounds according to one or more of Claims 1 to 8 or 10 in electronic devices.

14. Electronic device comprising at least one compound according to one or more of Claims 1 to 8 or 10, in particular selected from organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) or organic photoreceptors.

15. Organic electroluminescent device according to Claim 14, **characterised in that** the compound according to one or more of Claims 1 to 8 is employed as matrix material for fluorescent or phosphorescent compounds, in particular if one or more groups R and/or R¹ stand for C(=O)Ar, S(=O)Ar, S(=O)₂Ar or P(=O)Ar₂, and/or **in that** the compound according to one or more of Claims 1 to 8 is employed as hole-transport material or as hole-injection material or as electron-blocking material or as exciton-blocking material, in particular if one or more groups R and/or R¹ stand for N(Ar)₂, and/or **in that** the compound according to one or more of Claims 1 to 8 is employed as electron-transport material or as hole-blocking material, in particular if the group Y stands for C=O, P(=O), SO or SO₂ and/or at least one of the substituents R and/or R¹ stands for a heteroaryl group which represents an electron-deficient heterocycle or for C(=O)Ar, P(=O)Ar₂, S(=O)Ar or S(O)₂Ar.

## Revendications

1. Composés de la formule (1), 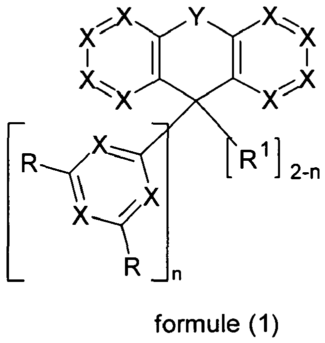 dans laquelle ce qui suit s'applique aux symboles et indices utilisés :
X est, pour chaque occurrence, de manière identique ou différente, CR¹ ou N, où un maximum de 3 groupes X dans chaque cycle représentent N ; ou deux groupes directement adjacents X représentent une unité de la formule qui suit (7), 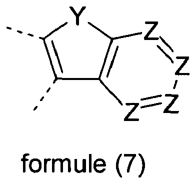 où les liaisons en pointillés représentent le lien depuis l'unité jusqu'aux atomes de C ou de N adjacents ;
Y est, pour chaque occurrence, de manière identique ou différente, une liaison simple ou un groupe choisi parmi BR¹, C(R¹)₂, C(=O), NR¹, P(=O)R¹ ou O ;
Z est, pour chaque occurrence, de manière identique ou différente, CR¹ ou N, où un maximum de deux symboles Z dans chaque cycle représentent N ;
R est, pour chaque occurrence, de manière identique ou différente, Br, I, triflate, B(OR²)₂, B(R²)₂, B(N(R²)₂)₂, NAr₂, N(R²)₂, SiAr₃, Si(R²)₃, C(=O)Ar, C(=O)R², OAr, OR², SAr, SR², S(=O)Ar, S(=O)R², S(=O)₂Ar, S(=O)₂R², PAr₂, P(R²)₂, P(=O)Ar₂, P(=O)(R²)₂ ou un système de cycle aromatique ou hétéroaromatique comportant de 5 à 60 atomes de cycle aromatique, lesquels peuvent être substitués par un ou plusieurs radicaux R¹ ;
Ar est, pour chaque occurrence, de manière identique ou différente, un système de cycle aromatique ou hétéroaromatique comportant de 5 à 30 atomes de cycle aromatique, lesquels peuvent être substitués par un ou plusieurs radicaux non aromatiques R¹ ; deux radicaux Ar qui, ici, sont liés à l'atome d'azote ou de phosphore peuvent également être liés l'un à l'autre par une liaison simple ou un pont choisi parmi B(R²), C(R²)₂, Si(R²)₂, C=O, C=NR², C=C(R²)₂, O, S, S=O, SO₂, N(R²), P(R²) et P(=O)R²
R¹ est, pour chaque occurrence, de manière identique ou différente, H, D, F, CI, Br, I, CHO, N(R²)_{2,} N(Ar)₂, C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², un groupe alkyle, alkényle, alkynyle, alcoxy ou thioalcoxy en chaîne droite comportant de 1 à 40 atomes de C ou un groupe alkyle, alkényle, alkynyle, alcoxy ou thioalcoxy ramifié ou cyclique comportant de 3 à 40 atomes de C, dont chacun peut être substitué par un ou plusieurs radicaux R², où un ou plusieurs groupes CH₂ non adjacents peuvent être remplacés par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et où un ou plusieurs atomes de H peuvent être remplacés par F, Cl, Br, I, CN ou NO₂, ou un système de cycle aromatique ou hétéroaromatique comportant de 5 à 60 atomes de cycle aromatique, lesquels peuvent dans chaque cas être substitués par un ou plu- sieurs radicaux R², ou un groupe aryloxy ou hétéroaryloxy comportant de 5 à 60 atomes de cycle aromatique, lesquels peuvent être substitués par un ou plusieurs radicaux R², ou une combinaison de ces systèmes ; deux ou plusieurs substituents adjacents R¹ ici peuvent également former un système mono- ou polycyclique, aliphatique ou aromatique les uns avec les autres ;
R² est, pour chaque occurrence, de manière identique ou différente, H, D ou un radical hydrocarbone aliphatique, aromatique et/ou hétéroaromatique comportant de 1 à 20 atomes C, où, en outre, des atomes de H peuvent être remplacés par F ; deux ou plusieurs substituents adjacents R² peuvent ici également former un système de cycle mono- ou polycyclique, aliphatique ou aromatique les uns avec les autres ;
n est 1 ou 2 ;
les composés qui suivent sont exclus de l'invention :

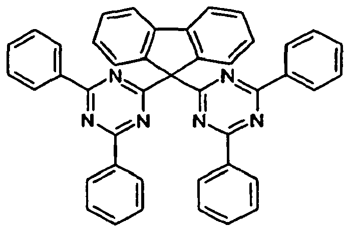

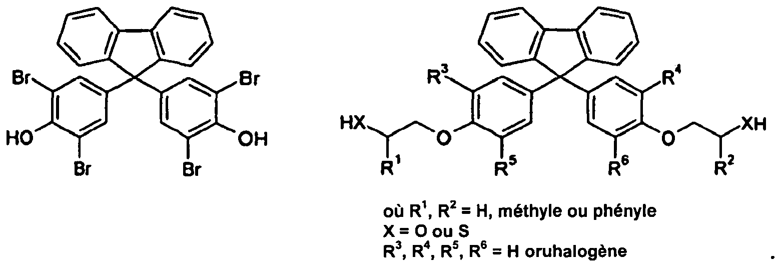

2. Composés selon la revendication 1 de la formule (2), (3), (8), (9), (10) ou (11), 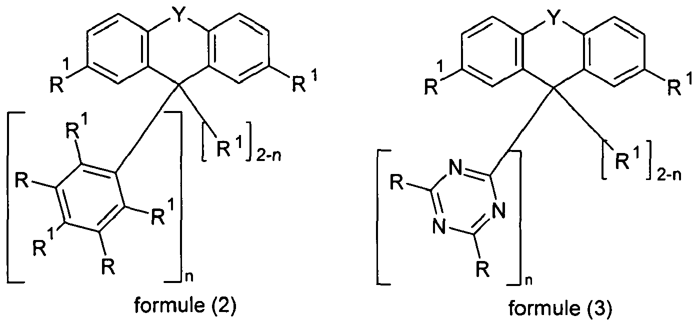 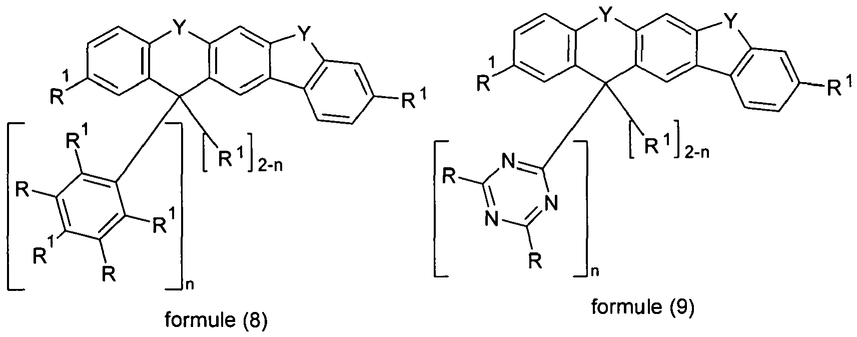 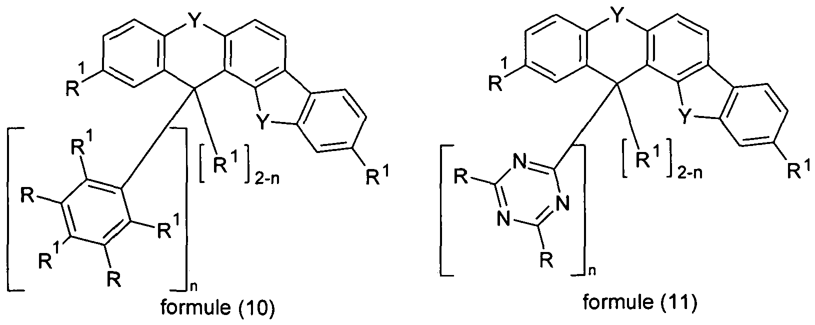 où les symboles et indices utilisés présentent les significations indiquées selon la revendication 1.

3. Composés selon la revendication 1 ou 2, **caractérisés en ce que** le symbole Y représente une liaison simple ou un groupe choisi parmi C(R¹)₂, O ou NR¹.

4. Composés selon une plusieurs revendications 1 à 3 de la formule (2a), (3a), (8a), (8b), (9a), (9b), (10a), (10b), (11a) ou (11b), 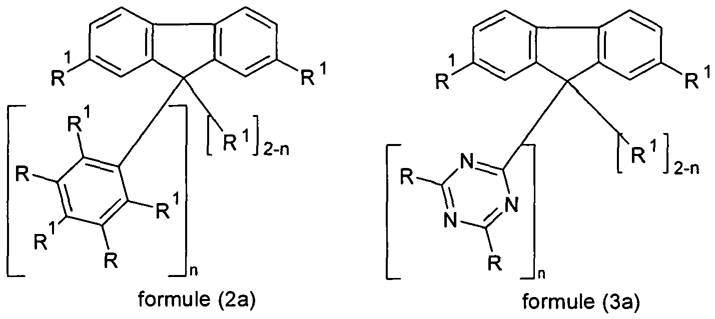 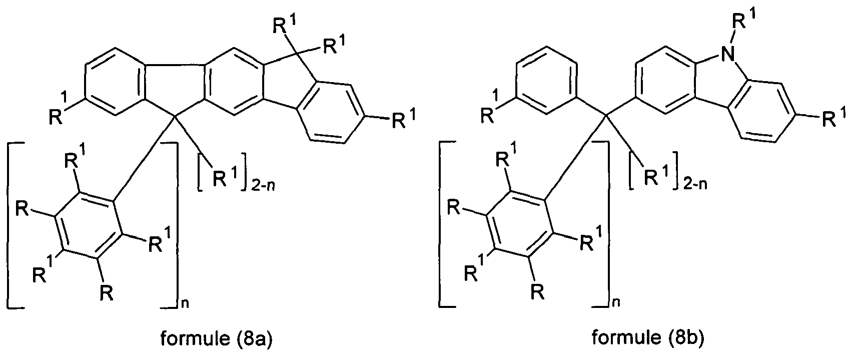 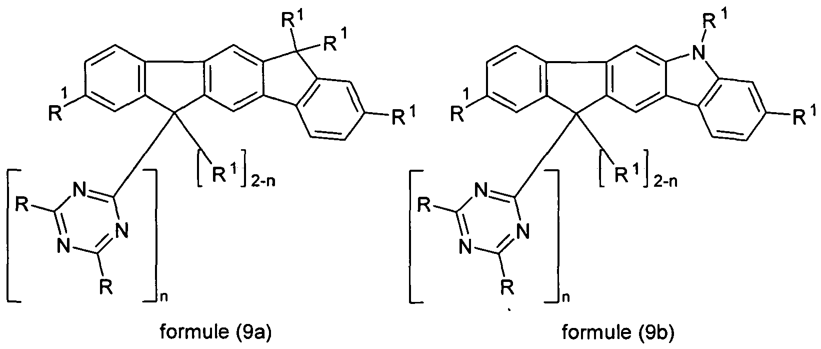 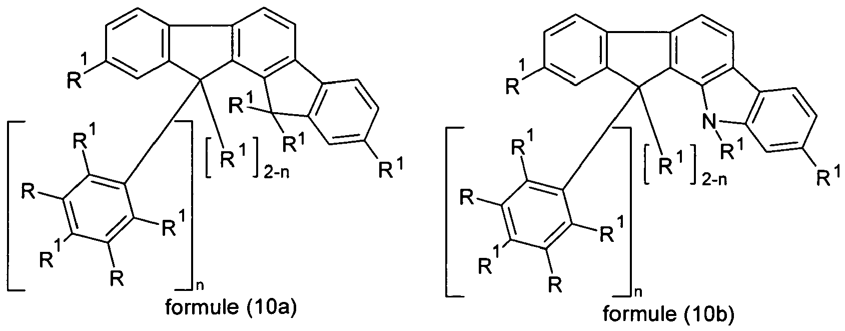 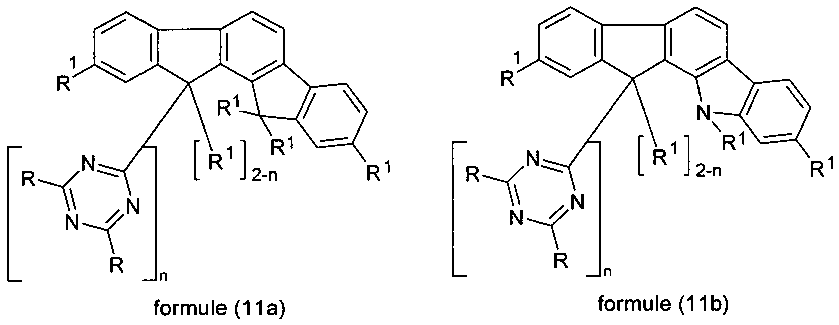 où les symboles utilisés présentent les significations indiquées selon la revendication 1.

5. Composés selon une ou plusieurs des revendications 1 à 3 de la formule (4a) ou (4b), 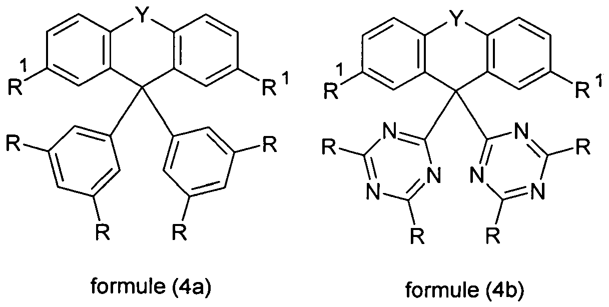 où les symboles utilisés présentent les significations indiquées selon la revendication 1.

6. Composés selon une ou plusieurs des revendications 1 à 5 de la formule (4c), (4d), (8c), (8d), (9c), (9d), (10c), (10d), (11c) ou (11d), 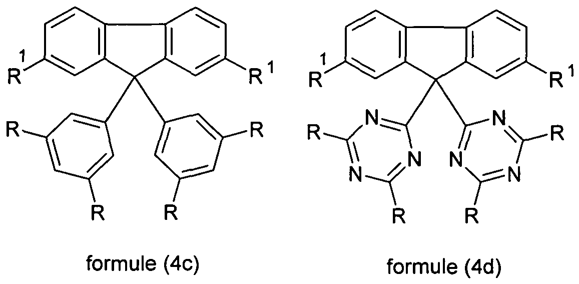 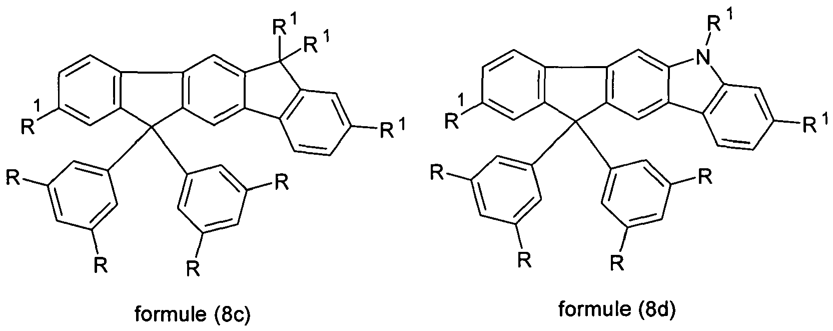 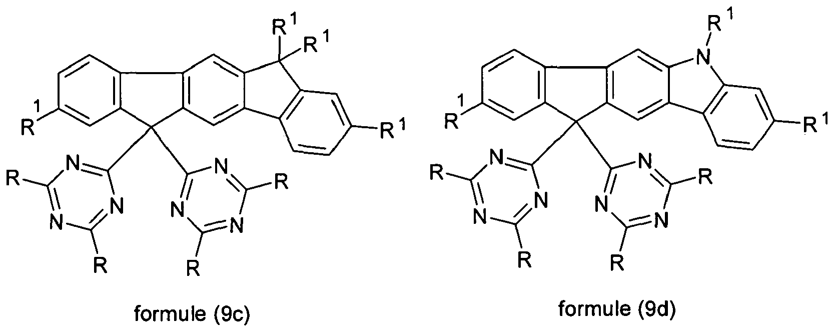 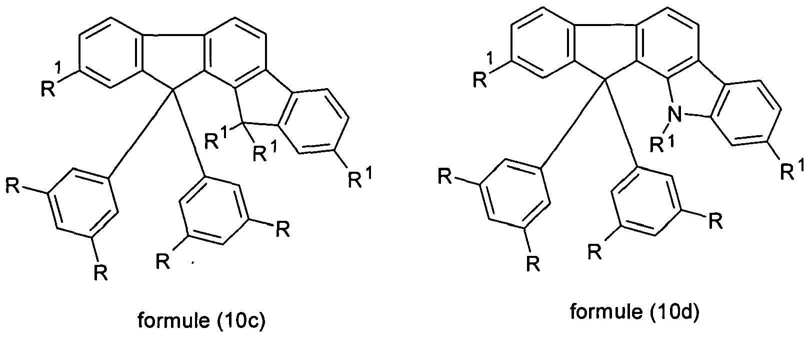 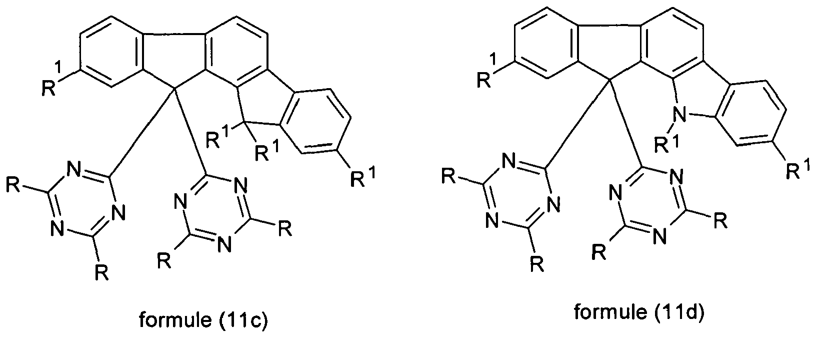 où les symboles utilisés présentent les significations indiquées selon la revendication 1.

7. Composés selon une ou plusieurs des revendications 1 à 6, **caractérisés en ce que** le symbole R, de manière identique ou différente pour chaque occurrence, représente NAr₂, C(=O)Ar, P(=O)Ar₂ ou un système de cycle aromatique ou hétéroaromatique comportant de 5 à 30 atomes de cycle aromatique, lesquels peuvent être substitués par un ou plusieurs radicaux non aromatiques R¹.

8. Composés selon une ou plusieurs des revendications 1 à 7, **caractérisés en ce que** le radical R ou R¹, s'il représente un groupe N(Ar)₂, est choisi parmi les groupes de la formule (5) ou de la formule (6), 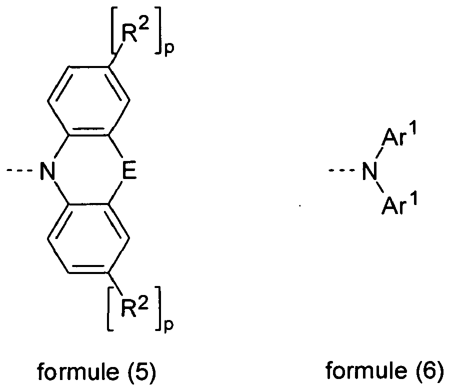 dans lesquelles R² présente la signification indiquée ci avant, et en outre :
E représente une liaison simple, O, S, N(R²) ou C(R²)₂ ;
Ar¹ est, de manière identique ou différente pour chaque occurrence, un système de cycle aromatique ou hétéroaromatique comportant de 5 à 20 atomes de cycle aromatique ou un groupe triarylamine comportant de 15 à 30 atomes de cycle aromatique, dont chacun peut être substitué par un ou plusieurs radicaux R², de préférence un groupe aryle ou hétéroaryle comportant de 6 à 14 atomes de cycle aromatique ou un groupe triarylamine comportant de 18 à 30 atomes de cycle aromatique, de préférence comportant de 18 à 22 atomes de cycle aromatique, dont chacun peut être substitué par un ou plusieurs radicaux R² ;
p est, pour chaque occurrence, de manière identique ou différente, 0 ou 1 ;
et/ou **en ce que** le radical R, s'il représente un système de cycle aromatique ou hétéroaromatique, est choisi parmi phényle, 1-naphtyle, 2-naphtyle, anthracényle, phénylanthracényle, 1- ou 2-naphtylanthracényle, binaphtyle, pyrényle, fluoranthényle, 2-, 3-, 4-, 5-, 6- ou 7-benzanthracényle, N-benzimidazolyle, phényl-N-benzimidazolyle, N-phénylbenzimidazolyle ou phényl-N-phénylbenzimidazolyle.

9. Procédé pour la préparation de composés selon une ou plusieurs des revendications 1 à 8, comprenant la réaction de bis(3,5-dibromo)-benzophénone avec un 2-lithiobiphényle, 2-lithiodiphényléther, 2-lithiodiphénylthioéther, 2-(2-lithiophényl)-2-phényl-1,3-dioxolane, 2-lithiophényldiphénylamine substitué ou non substitué ou un composé de Grignard correspondant pour obtenir les triarylméthanols, suivie par une cyclisation sous des conditions acides et en option, suivie par une réaction supplémentaire des groupes brome.

10. Dimères, trimères, tétramères, pentamères, oligomères, polymères ou dendrimères contenant un ou plusieurs composés selon une ou plusieurs des revendications 1 à 8, où un ou plusieurs radicaux R¹ ou R² représentent des liaisons entre les composés de la formule (1) dans le dimère, trimère, tétramère ou pentamère ou des liaisons depuis le composé de la formule (1) sur le polymère, l'oligomère ou le dendrimère ou où cette liaison est réalisée via des substituents sur les groupes R.

11. Mélanges comprenant au moins un composé selon une ou plusieurs des revendications 1 à 8 ou 10 et au moins un autre composé.

12. Solutions comprenant au moins un composé selon une ou plusieurs des revendications 1 à 8 ou 10 et au moins un solvant organique.

13. Utilisation de composés selon une ou plusieurs des revendications 1 à 8 ou 10 dans des dispositifs électroniques.

14. Dispositif électronique comprenant au moins un composé selon une ou plusieurs des revendications 1 à 8 ou 10, en particulier choisi parmi des dispositifs électroluminescents organiques (OLED, PLED), des transistors à effet de champ (O-FET), des transistors à film mince organiques (O-TFT), des transistors émetteurs de lumière organiques (O-LET), des circuits intégrés organiques (O-IC), des cellules solaires organiques (O-SC), des dispositifs à extinction de champ organiques (O-FQD), des cellules électrochimiques émettrices de lumière (LEC), des diodes laser organiques (O-laser) ou des photorécepteurs organiques.

15. Dispositif électroluminescent organique selon la revendication 14, **caractérisé en ce que** le composé selon une ou plusieurs des revendications 1 à 8 est utilisé en tant que matériau matriciel pour des composés fluorescents ou phosphorescents, en particulier si un ou plusieurs groupes R et/ou R¹ représentent C(=O)Ar, S(=O)Ar, S(=O)₂Ar ou P(=O)Ar₂, et/ou **en ce que** le composé selon une ou plusieurs des revendications 1 à 8 est utilisé en tant que matériau de transport de trous ou en tant que matériau d'injection de trous ou en tant que matériau de blocage d'électrons ou en tant que matériau de blocage d'excitons, en particulier si un ou plusieurs groupes R et/ou R¹ représentent N(Ar)₂, et/ou **en ce que** le composé selon une ou plusieurs des revendications 1 à 8 est utilisé en tant que matériau de transport d'électrons ou en tant que matériau de blocage de trous, en particulier si le groupe Y représente C=O, P(=O), SO ou SO₂ et/ou au moins l'un des substituents R et/ou R¹ représente un groupe hétéroaryle, lequel représente un hétérocycle déficient en électrons ou C(=O)Ar, P(=O)Ar₂, S(=O)Ar ou S(O)₂Ar.
